# EUROPEAN PATENT APPLICATION

(11) **EP 1 854 429 A1**
(43) Date of publication of application: **14.11.2007**
(21) Application number: 07108088.1
(22) Date of filing: 11.05.2007
(51) Int. Cl.: A61F 2/24

(54) **Intraoperative and post-operative adjustment of an annuloplasty ring**

(30) Priority: 12.05.2006 US 799909 P
(71) Applicant: Micardia Corporation, Irvine CA 92618 (US)
(72) Inventor: Le, le, San Jose, CA 95131 (US); Flores, Jesus, Perris, CA 92570 (US); Tran, Jason, Santa Ana, CA 92704 (US); Shaolian, Samuel, Newport Beach, CA 92660 (US); Marmureanu, Alexander, Los Angeles, CA 90067 (US)
(74) Representative: Hill, Justin John

(57) **Abstract**

An intraoperative adjustment device (2900) is disclosed. In some embodiments, the device includes an elongate body (2930) comprising a proximal end and a distal end, the distal end configured to penetrate an outer surface of an adjustable cardiac implant implanted in a patient's heart, and the proximal end and the distal end connected by at least one energy-transfer member. In some embodiments, the distal end includes at least one electrode (2910) coupled to the energy-transfer member and configured to deliver an activation energy to the adjustable cardiac implant. In some embodiments, the proximal end is configured to attach to an external energy source that provides the activation energy, and the proximal end is configured to be located outside the patient's body while the distal end is coupled to the adjustable cardiac implant (3110) that is implanted in the patient's heart.

## Description

### BACKGROUND OF THE INVENTION

### Field of the Invention

The present disclosure relates generally to devices and methods for reinforcing body structures, for example, heart valves, and more particularly, for adjusting an annuloplasty ring intraoperatively.

### Description of the Related Art

The circulatory system of mammals includes the heart and the interconnecting vessels throughout the body that include both veins and arteries. The human heart includes four chambers, which are the left and right atrium and the left and right ventricles. The mitral valve, which allows blood flow in one direction, is positioned between the left ventricle and left atrium. The tricuspid valve is positioned between the right ventricle and the right atrium. The aortic valve is positioned between the left ventricle and the aorta, and the pulmonary valve is positioned between the right ventricle and pulmonary artery. The heart valves function in concert to move blood throughout the circulatory system. The right ventricle pumps oxygen-poor blood from the body to the lungs and then into the left atrium. From the left atrium, the blood is pumped into the left ventricle and then out the aortic valve into the aorta. The blood is then recirculated throughout the tissues and organs of the body and returns once again to the right atrium.

If the valves of the heart do not function properly, due either to disease or congenital defects, the circulation of the blood may be compromised. Diseased heart valves may be stenotic, wherein the valve does not open sufficiently to allow adequate forward flow of blood through the valve, and/or incompetent, wherein the valve does not close completely. Incompetent heart valves cause regurgitation or excessive backward flow of blood through the valve when the valve is closed. For example, certain diseases of the heart valves can result in dilation of the heart and one or more heart valves. When a heart valve annulus dilates, the valve leaflet geometry deforms and causes ineffective closure of the valve leaflets. The ineffective closure of the valve can cause regurgitation of the blood, accumulation of blood in the heart, and other problems.

Diseased or damaged heart valves can be treated by valve replacement surgery, in which damaged leaflets are excised and the annulus is sculpted to receive a replacement valve. Another repair technique that has been shown to be effective in treating incompetence is annuloplasty, in which the effective size of the valve annulus is contracted by attaching a prosthetic annuloplasty repair segment or ring to an interior wall of the heart around the valve annulus. The annuloplasty ring reinforces the functional changes that occur during the cardiac cycle to improve coaptation and valve integrity. Thus, annuloplasty rings help reduce reverse flow or regurgitation while permitting good hemodynamics during forward flow.

Generally, annuloplasty rings comprise an inner substrate of a metal such as stainless steel or titanium, or a flexible material such as silicone rubber or Dacron@. The inner substrate is generally covered with a biocompatible fabric or cloth to allow the ring to be sutured to the heart tissue. Annuloplasty rings may be stiff or flexible, may be open or closed, and may have a variety of shapes including circular, D-shaped, or C-shaped. The configuration of the ring is generally based on the shape of the heart valve being repaired or on the particular application. For example, the tricuspid valve is generally circular and the mitral valve is generally D-shaped. Further, C-shaped rings may be used for tricuspid valve repairs, for example, because it allows a surgeon to position the break in the ring adjacent the atrioventricular node, thus avoiding the need for suturing at that location.

Annuloplasty rings support the heart valve annulus and restore the valve geometry and function. Although the implantation of an annuloplasty ring can be effective, the heart of a patient may change geometry over time after implantation. For example, the heart of a child will grow as the child ages. As another example, after implantation of an annuloplasty ring, dilation of the heart caused by accumulation of blood may cease and the heart may begin returning to its normal size. Whether the size of the heart grows or reduces after implantation of an annuloplasty ring, the ring may no longer be the appropriate size for the changed size of the valve annulus.

### SUMMARY OF THE INVENTION

An intraoperative adjustment device useful for adjusting a size, dimension, or shape of an implanted annuloplasty ring substantially contemporaneously with the implantation of the annuloplasty ring or other adjustable device by applying energy to the annuloplasty ring appropriate for the adjustment thereof is disclosed. Also disclosed are methods for using the intraoperative adjustment device in the adjustment of an annuloplasty ring and an annuloplasty ring system. In certain embodiments, the annuloplasty ring is adjustable using an activation energy source, for example, radio frequency (RF) energy, microwave energy, ultrasonic energy, magnetic energy, electric energy, thermal energy, combinations thereof, and the like.

The capability to alter the shape and dimensions of an implanted annuloplasty ring permits titration of valve dimensions once loading forces are applied to the mitral valve so that dynamic remodeling can be achieved. This method is particularly applicable to the ischemic and dilated cardiomyopathy subset of mitral regurgitation because dynamic forces are more involved in valve competency in these diseased states. Valves conducive to treatment with this type of device include the mitral and tricuspid valves.

In certain embodiments, an intraoperative adjustment device is disclosed. The device comprises an elongate body comprising a proximal end and a distal end, the distal end configured to penetrate an outer surface of an adjustable cardiac implant implanted in a patient's heart, and the proximal end and the distal end connected by at least one energy-transfer member. The distal end comprises at least one electrode coupled to the energy-transfer member and configured to deliver an activation energy to the adjustable cardiac implant. The proximal end is configured to attach to an external energy source that provides the activation energy, and the proximal end is configured to be located outside the patient's body while the distal end is coupled to the adjustable cardiac implant that is implanted in the patient's heart.

In certain embodiments of the device, the distal end further comprises at least one thermocouple. In certain embodiments, the at least one thermocouple is proximal to the at least one electrode. In certain embodiments, the distal end further comprises a flexible member extending distally from the at least one electrode and a needle coupled to a distal end of the flexible member. In certain embodiments, the needle is curved. In certain embodiments, the flexible member comprises a suture. In certain embodiments, the at least one electrode is configured to penetrate the outer surface of the adjustable cardiac implant. In certain embodiments, a portion of the distal end that penetrates the adjustable cardiac implant is sharp. In certain embodiments, a length of the electrode is substantially equal to or less than a cross-sectional thickness of the adjustable cardiac implant. In certain embodiments, the distal end further comprises a coiled housing proximal to the at least one electrode. In certain embodiments, the distal end further comprises a plurality of electrodes. In certain embodiments, the at least one energy-transfer member comprises a plurality of energy-transfer members, each of which is coupled to at least one of the plurality of electrodes. In certain embodiments, the electrode comprises a biocompatible, thermally conductive material. In certain embodiments, the electrode comprises a biocompatible, electrically conductive material. In certain embodiments, the external energy source comprises a radio frequency generator, and the device further comprises the radio frequency generator. In certain embodiments, the device further comprises a malleable element that permits the elongate body to flex from a first shape to a second shape and substantially retain the second shape.

In certain embodiments, an intraoperative adjustment device is disclosed. The device comprises an elongate body comprising a proximal end and a distal end, the distal end configured to enter a chamber of a heart. The device further comprises an extendable probe comprising a biocompatible, thermally and/or electrically conductive material disposed on the first end of the body. The device further comprises a handle coupled to the proximal end of the body. A distal end of the extendable probe is configured to move from a retracted position that is substantially within the distal end, to an extended position that is substantially protruding distally from the distal end. The extendable probe, when in the extended position, is configured to transfer energy from the proximal end to an adjustable cardiac implant that is implanted in the heart. The device further comprises a stop mechanism configured to prevent a distal end of the extendable probe from proximally retracting out of the elongate body.

In certain embodiments of the device, the extendable probe is configured to penetrate an outer surface of an adjustable device. In certain embodiments, the distal end of the elongate body further comprises a thermocouple. In certain embodiments, the extendable probe comprises a sharp end. In certain embodiments, the extendable probe comprises a substantially helical shape. In certain embodiments, the extendable probe is configured to screw into the adjustable cardiac implant. In certain embodiments, the extendable probe comprises a substantially hook shape.

In certain embodiments, an intraoperative adjustment device is disclosed. The device comprises an elongate body having a distal portion, a proximal portion, and an electrode assembly disposed between the proximal portion and the distal portion. The electrode assembly comprises an electrode configured to deliver energy to an adjustable cardiac implant. The distal portion of the elongate body comprises a suture coupled to a distal end of the electrode assembly. The proximal portion of the elongate body comprises a conducting element that transfers energy from an energy source to the electrode. The device further comprises a needle coupled to a distal end of the suture, the needle configured to penetrate a surface of the adjustable cardiac implant.

In certain embodiments of the device, the device further comprises a sheath disposed over at least a portion of the conducting element. In certain embodiments, the device further comprises a thermocouple positioned to detect a temperature at the distal electrode.

In certain embodiments, an adjustable cardiac implant system is disclosed. The system comprises an adjustable cardiac implant configured to be implanted at or near a base of a patient's heart valve. The adjustable cardiac implant comprises a shape-memory element configured to undergo a transformation in shape and/or size in response to an application of energy, thereby resulting in a change in a dimension of the annulus of the patient's heart valve. The adjustable cardiac implant system further comprises a first conducting member coupled to the adjustable cardiac implant, the first conducting member configured to transfer energy from an energy source to the adjustable cardiac implant resulting in the transformation in shape and/or size of the shape-memory element, wherein the first conducting member extends through a point located at least one centimeter away from an outer surface of the adjustable cardiac implant.

In certain embodiments of the system, the system further comprises a second conducting member coupled to the adjustable cardiac implant, the second conducting member configured to transfer energy from the energy source to the adjustable cardiac implant. In certain embodiments, the first conducting member comprises a wire. In certain embodiments, the first conducting member is reversibly coupled to the adjustable cardiac implant. In certain embodiments, the first conducting member extends outside the patient's heart when the adjustable cardiac implant is implanted at or near the base of the patient's heart valve. In certain embodiments, the first conducting member conducts at least one of heat and electromagnetic energy to the adjustable cardiac implant. In certain embodiments, the adjustable cardiac implant further comprises a flexible material disposed over the shape-memory element and the first conducting member.

For purposes of summarizing the invention, certain aspects, advantages and novel features of the invention have been described herein. It is to be understood that not necessarily all such advantages may be achieved in accordance with any particular embodiment of the invention. Thus, the invention may be embodied or carried out in a manner that achieves or optimizes one advantage or group of advantages as taught herein without necessarily achieving other advantages as may be taught or suggested herein.

### BRIEF DESCRIPTION OF THE DRAWINGS

Systems and methods which embody the various features of the invention will now be described with reference to the following drawings:

FIG. 1A is a top view of an adjustable annuloplasty ring according to certain embodiments of the invention;

FIG. 1B is a side view of the annuloplasty ring of FIG. 1A;

FIG. 1C is a transverse cross-sectional view of the annuloplasty ring of FIG. 1A;

FIG. 2 is a graphical representation of the diameter of an annuloplasty ring in relation to the temperature of the annuloplasty ring according to certain embodiments of the invention;

FIG. 3A is a top view in partial section of an adjustable annuloplasty ring having a D-shaped configuration according to certain embodiments of the invention;

FIG. 3B is a side view of the annuloplasty ring of FIG. 3A;

FIG. 3C is a transverse cross-sectional view of the annuloplasty ring of FIG. 3A;

FIG. 4A is a top view of an annuloplasty ring having a substantially circular configuration according to certain embodiments of the invention;

FIG. 4B is a side view of the annuloplasty ring of FIG. 4A;

FIG. 4C is a transverse cross-sectional view of the annuloplasty ring of FIG. 4A;

FIG. 5 is a top view of an annuloplasty ring having a substantially D-shaped configuration according to certain embodiments of the invention;

FIG. 6A is a schematic diagram of a top view of a shape memory wire having a substantially D-shaped configuration according to certain embodiments of the invention;

FIGS. 6B-6E are schematic diagrams of side views of the shape memory wire of FIG. 6A according to certain embodiments of the invention;

FIG. 7A is a perspective view in partial section of an annuloplasty ring comprising the shape memory wire of FIG. 6A according to certain embodiments of the invention;

FIG. 7B is a perspective view in partial section of a portion of the annuloplasty ring of FIG. 7A;

FIG. 8 is a schematic diagram of a shape memory wire having a substantially C-shaped configuration according to certain embodiments of the invention;

FIG. 9A is a perspective view in partial section of an annuloplasty ring comprising the shape memory wire of FIG. 8 according to certain embodiments of the invention;

FIG. 9B is a perspective view in partial section of a portion of the annuloplasty ring of FIG. 9A;

FIG. 10A is a perspective view in partial section an annuloplasty ring comprising a first shape memory wire and a second shape memory wire according to certain embodiments of the invention;

FIG. 10B is a top cross-sectional view of the annuloplasty ring of FIG. 10A;

FIG. 11A is a perspective view in partial section of an annuloplasty ring comprising a first shape memory wire and a second shape memory wire according to certain embodiments of the invention;

FIG. 11B is a top cross-sectional view of the annuloplasty ring of FIG. 11A;

FIG. 12 is a perspective view of a shape memory wire wrapped in a coil according to certain embodiments of the invention;

FIGS. 13A and 13B are schematic diagrams illustrating an annuloplasty ring according to certain embodiments of the invention;

FIG. 14 is a schematic diagram illustrating an annuloplasty ring according to certain embodiments of the invention;

FIG. 15 is a schematic diagram illustrating an annuloplasty ring according to certain embodiments of the invention;

FIGS. 16A and 16B are schematic diagrams illustrating an annuloplasty ring having a plurality of temperature response zones or sections according to certain embodiments of the invention;

FIGS. 17A and 17B are schematic diagrams illustrating an annuloplasty ring having a plurality of temperature response zones or sections according to certain embodiments of the invention;

FIG. 18 is a sectional view of a mitral valve with respect to an exemplary annuloplasty ring according to certain embodiments of the invention;

FIG. 19 is a schematic diagram of a substantially C-shaped wire comprising a shape memory material configured to contract in a first direction and expand in a second direction according to certain embodiments of the invention;

FIGS. 20A and 20B are schematic diagrams of a body member usable by an annuloplasty ring according to certain embodiments of the invention;

FIGS. 21A and 21B are schematic diagrams of a body member usable by an annuloplasty ring according to certain embodiments of the invention;

FIGS. 22A and 22B are schematic diagrams of a body member usable by an annuloplasty ring according to certain embodiments of the invention;

FIG. 23 is a transverse cross-sectional view of the body member of FIGS. 21A and 21B;

FIG. 24 is a perspective view of a body member usable by an annuloplasty ring according to certain embodiments comprising a first shape memory band and a second shape memory band;

FIG. 25A is a schematic diagram illustrating the body member of FIG. 24 in a first configuration or shape according to certain embodiments of the invention;

FIG. 25B is a schematic diagram illustrating the body member of FIG. 24 in a second configuration or shape according to certain embodiments of the invention;

FIG. 25C is a schematic diagram illustrating the body member of FIG. 24 in a third configuration or shape according to certain embodiments of the invention;

FIG. 26 is a perspective view illustrating an annuloplasty ring comprising one or more thermal conductors according to certain embodiments of the invention;

FIGS. 27A-27C are transverse cross-sectional views of the annuloplasty ring of FIG. 26 schematically illustrating exemplary embodiments of the invention for conducting thermal energy to an internal shape memory wire;

FIG. 28 is a schematic diagram of an annuloplasty ring comprising one or more thermal conductors according to certain embodiments of the invention;

FIG. 29A illustrates a cross-section of an intraoperative adjustment device according to certain embodiments of the invention;

FIG. 29B illustrates a section A-A of FIG. 29A of the body of the intraoperative adjustment device;

FIG. 29C illustrates an end view of the tip electrode of the intraoperative adjustment device illustrated in FIG. 29A;

FIG. 29D illustrates a side view of the tip electrode of the intraoperative adjustment device illustrated in FIG. 29A;

FIG. 30 is a flow chart illustrating a method for adjusting an annuloplasty ring using the intraoperative adjustment device illustrated in FIG. 29A according to certain embodiments of the invention;

FIG. 31 schematically illustrates the use of the intraoperative adjustment device illustrated in FIG. 29A in adjusting an annuloplasty ring implanted on the tricuspid valve according to certain embodiments of the invention;

FIGS. 32A-32C illustrate an intraoperative adjustment device comprising an extendable device with a punctured tip according to certain embodiments of the invention;

FIGS. 32D-32G illustrate cross-sectional and isometric views of embodiments of intraoperative adjustment devices comprising helical and hook-shaped extendable probes according to certain embodiments of the invention;

FIG. 33 illustrates in cross-section an embodiment of an intraoperative adjustment device comprising a means for providing a fluid, for example, saline, during the adjustment procedure according to certain embodiments of the invention;

FIG. 34 illustrates in cross-section an intraoperative adjustment device according to certain embodiments of the invention similar to the device illustrated in FIG. 29A where the thermal couple is offset from a longitudinal axis;

FIGS. 35A-35B illustrate an embodiment of an intraoperative adjustment device according to certain embodiments of the invention;

FIGS. 36A-36E are a side view, partial cross sections, and cross sections illustrating an implantable adjustment electrode useful for intraoperative and/or post-operative adjustment of an adjustable device comprising a shape memory material according to certain embodiments of the invention;

FIG. 37 is a flowchart illustrating a method for adjusting an adjustable device comprising a shape memory material using the device illustrated in FIGS. 36A-36E according to certain embodiments of the invention;

FIGS. 38A-38E are different views illustrating an integrated implantable adjustment electrode and adjustable annuloplasty ring system according to certain embodiments of the invention;

FIG. 39 is a flowchart illustrating a method for adjusting the annuloplasty ring of the integrated implantable adjustment electrode and adjustable annuloplasty ring system illustrated in FIGS. 38A-38E according to certain embodiments of the invention;

FIG. 40 is a side view illustrating an adjustment device for intraoperative adjustment of an adjustable device according to certain embodiments of the invention;

FIG. 41 is a flowchart illustrating a method for adjusting an adjustable device using the device illustrated in FIG. 40 according to certain embodiments of the invention;

FIG. 42 is a side view illustrating an adjustment device for intraoperative adjustment of an adjustable device according to certain embodiments of the invention; and

FIG. 43 is a flowchart illustrating a method for adjusting an adjustable device using the device illustrated in FIG. 42 according to certain embodiments of the invention.

### DETAILED DESCRIPTION OF EXEMPLARY EMBODIMENTS

### Externally Adjustable Annuloplasty Rings

The present invention involves systems and methods for adjusting adjustable rings and other adjustable implants used for reinforcing dysfunctional heart valves and other body structures. In certain embodiments, such an adjustable annuloplasty ring is implanted into the body of a patient such as a human or other animal. The adjustable annuloplasty ring is implanted through an incision or body opening either thoracically (e.g., open-heart surgery) or percutaneously (e.g., via a femoral artery or vein, or other arteries or veins) as is known to someone skilled in the art. The adjustable annuloplasty ring is attached to the annulus of a heart valve to improve leaflet coaptation and to reduce regurgitation. The annuloplasty ring may be selected from one or more shapes comprising a round or circular shape, an oval shape, a C-shape, a D-shape, a U-shape, an open circle shape, an open oval shape, and other curvilinear shapes.

The size of the annuloplasty ring can be adjusted post-operatively to compensate for changes in the size of the heart. As used herein, "post-operatively" refers to a time after implanting the adjustable annuloplasty ring and closing the body opening through which the adjustable annuloplasty ring was introduced into the patient's body. For example, the annuloplasty ring may be implanted in a child whose heart grows as the child gets older. Thus, the size of the annuloplasty ring may need to be increased. As another example, the size of an enlarged heart may start to return to its normal size after an annuloplasty ring is implanted. Thus, the size of the annuloplasty ring may need to be decreased post-operatively to continue to reinforce the heart valve annulus.

The size of the annuloplasty ring can also be adjusted intraoperatively. As used herein, "intraoperatively" refers to a time during implanting the adjustable annuloplasty ring through a body opening through by which the adjustable annuloplasty ring is introduced into the patient's body.

In certain embodiments, the annuloplasty ring comprises a shape memory material that is responsive to changes in temperature and/or exposure to a magnetic field. Shape memory is the ability of a material to regain its shape after deformation. Shape memory materials include polymers, metals, metal alloys and ferromagnetic alloys. The annuloplasty ring is adjusted in vivo by applying an energy source to activate the shape memory material and cause it to change to a memorized shape. The energy source may include, for example, radio frequency (RF) energy, x-ray energy, microwave energy, ultrasonic energy such as focused ultrasound, high intensity focused ultrasound (HIFU) energy, light energy, electric field energy, magnetic field energy, combinations of the foregoing, or the like. For example, one embodiment of electromagnetic radiation that is useful is infrared energy having a wavelength in a range between approximately 750 nanometers and approximately 1600 nanometers. This type of infrared radiation may be produced efficiently by a solid state diode laser. In certain embodiments, the annuloplasty ring implant is selectively heated using short pulses of energy having an on and off period between each cycle. The energy pulses provide segmental heating which allows segmental adjustment of portions of the annuloplasty ring without adjusting the entire implant.

In certain embodiments, the annuloplasty ring includes an energy absorbing material to increase heating efficiency and localize heating in the area of the shape memory material. Thus, damage to the surrounding tissue is reduced or minimized. Energy absorbing materials for light or laser activation energy may include nanoshells, nanospheres and the like, particularly where infrared laser energy is used to energize the material. Such nanoparticles may be made from a dielectric, such as silica, coated with an ultra thin layer of a conductor, such as gold, and be selectively tuned to absorb a particular frequency of electromagnetic radiation. In certain such embodiments, the nanoparticles range in size between about 5 nanometers and about 20 nanometers and can be suspended in a suitable material or solution, such as saline solution. Coatings comprising nanotubes or nanoparticles can also be used to absorb energy from, for example, HIFU, MRI, inductive heating, or the like.

In certain embodiments, thin film deposition or other coating techniques such as sputtering, reactive sputtering, metal ion implantation, physical vapor deposition, and chemical deposition can be used to cover portions or all of the annuloplasty ring. Such coatings can be either solid or microporous. When HIFU energy is used, for example, a microporous structure traps and directs the HIFU energy toward the shape memory material. The coating improves thermal conduction and heat removal. In certain embodiments, the coating also enhances radio-opacity of the annuloplasty ring implant. Coating materials can be selected from various groups of biocompatible organic or non-organic, metallic or non-metallic materials such as Titanium Nitride (TiN), Iridium Oxide (Irox), Carbon, Platinum black, Titanium Carbide (TiC) and other materials used for pacemaker electrodes or implantable pacemaker leads. Other materials discussed herein or known in the art can also be used to absorb energy.

In addition, or in certain embodiments, fine conductive wires such as platinum coated copper, titanium, tantalum, stainless steel, gold, or the like, are wrapped around the shape memory material to allow focused and rapid heating of the shape memory material while reducing undesired heating of surrounding tissues.

In certain embodiments, the energy source is applied surgically either during implantation or at a later time. For example, the shape memory material can be heated during implantation of the annuloplasty ring by touching the annuloplasty ring with warm object. As another example, the energy source can be surgically applied after the annuloplasty ring has been implanted by percutaneously inserting a catheter into the patient's body and applying the energy through the catheter. For example, RF energy, light energy or thermal energy (e.g., from a heating element using resistance heating) can be transferred to the shape memory material through a catheter positioned on or near the shape memory material. Alternatively, thermal energy can be provided to the shape memory material by injecting a heated fluid through a catheter or circulating the heated fluid in a balloon through the catheter placed in close proximity to the shape memory material. As another example, the shape memory material can be coated with a photodynamic absorbing material which is activated to heat the shape memory material when illuminated by light from a laser diode or directed to the coating through fiber optic elements in a catheter. In certain such embodiments, the photodynamic absorbing material includes one or more drugs that are released when illuminated by the laser light.

In certain embodiments, a removable subcutaneous electrode or coil couples energy from a dedicated activation unit. In certain such embodiments, the removable subcutaneous electrode provides telemetry and power transmission between the system and the annuloplasty ring. The subcutaneous removable electrode allows more efficient coupling of energy to the implant with minimum or reduced power loss. In certain embodiments, the subcutaneous energy is delivered via inductive coupling.

In certain embodiments, the energy source is applied in a non-invasive manner from outside the patient's body. In certain such embodiments, the external energy source is focused to provide directional heating to the shape memory material so as to reduce or minimize damage to the surrounding tissue. For example, in certain embodiments, a handheld or portable device comprising an electrically conductive coil generates an electromagnetic field that non-invasively penetrates the patient's body and induces a current in the annuloplasty ring. The current heats the annuloplasty ring and causes the shape memory material to transform to a memorized shape. In certain such embodiments, the annuloplasty ring also comprises an electrically conductive coil wrapped around or embedded in the memory shape material. The externally generated electromagnetic field induces a current in the annuloplasty ring's coil, causing it to heat and transfer thermal energy to the shape memory material.

In certain embodiments, an external HIFU transducer focuses ultrasound energy onto the implanted annuloplasty ring to heat the shape memory material. In certain such embodiments, the external HIFU transducer is a handheld or portable device. The terms "HIFU," "high intensity focused ultrasound" or "focused ultrasound" as used herein are broad terms and are used at least in their ordinary sense and include, without limitation, acoustic energy within a wide range of intensities and/or frequencies. For example, HIFU includes acoustic energy focused in a region, or focal zone, having an intensity and/or frequency that is considerably less than what is currently used for ablation in medical procedures. Thus, in certain such embodiments, the focused ultrasound is not destructive to the patient's cardiac tissue. In certain embodiments, HIFU includes acoustic energy within a frequency range of approximately 0.5 MHz and approximately 30 MHz and a power density within a range of approximately 1 W/cm² and approximately 500 W/cm².

In certain embodiments, the annuloplasty ring comprises an ultrasound absorbing material or hydro-gel material that allows focused and rapid heating when exposed to the ultrasound energy and transfers thermal energy to the shape memory material. In certain embodiments, a HIFU probe is used with an adaptive lens to compensate for heart and respiration movement. The adaptive lens has multiple focal point adjustments. In certain embodiments, a HIFU probe with adaptive capabilities comprises a phased array or linear configuration. In certain embodiments, an external HIFU probe comprises a lens configured to be placed between a patient's ribs to improve acoustic window penetration and reduce or minimize issues and challenges regarding passing through bones. In certain embodiments, HIFU energy is synchronized with an ultrasound imaging device to allow visualization of the annuloplasty ring implant during HIFU activation. In addition, or in certain embodiments, ultrasound imaging is used to non-invasively monitor the temperature of tissue surrounding the annuloplasty ring by using principles of speed of sound shift and changes to tissue thermal expansion.

In certain embodiments, non-invasive energy is applied to the implanted annuloplasty ring using a Magnetic Resonance Imaging (MRI) device. In certain such embodiments, the shape memory material is activated by a constant magnetic field generated by the MRI device. In addition, or in certain embodiments, the MRI device generates RF pulses that induce current in the annuloplasty ring and heat the shape memory material. The annuloplasty ring can include one or more coils and/or MRI energy absorbing material to increase the efficiency and directionality of the heating. Suitable energy absorbing materials for magnetic activation energy include particulates of ferromagnetic material. Suitable energy absorbing materials for RF energy include ferrite materials as well as other materials configured to absorb RF energy at resonant frequencies thereof.

In certain embodiments, the MRI device is used to determine the size of the implanted annuloplasty ring before, during and/or after the shape memory material is activated. In certain such embodiments, the MRI device generates RF pulses at a first frequency to heat the shape memory material and at a second frequency to image the implanted annuloplasty ring. Thus, the size of the annuloplasty ring can be measured without heating the ring. In certain such embodiments, an MRI energy absorbing material heats sufficiently to activate the shape memory material when exposed to the first frequency and does not substantially heat when exposed to the second frequency. Other imaging techniques known in the art can also be used to determine the size of the implanted ring including, for example, ultrasound imaging, computed tomography (CT) scanning, X-ray imaging, or the like. In certain embodiments, such imaging techniques also provide sufficient energy to activate the shape memory material.

In certain embodiments, imaging and resizing of the annuloplasty ring is performed as a separate procedure at some point after the annuloplasty ring as been surgically implanted into the patient's heart and the patient's heart, pericardium and chest have been surgically closed. However, in certain embodiments, it is advantageous to perform the imaging after the heart and/or pericardium have been closed, but before closing the patient's chest, to check for leakage or the amount of regurgitation. If the amount of regurgitation remains excessive after the annuloplasty ring has been implanted, energy from the imaging device (or from another source as discussed herein) can be applied to the shape memory material so as to at least partially contract the annuloplasty ring and reduce regurgitation to an acceptable level. Thus, the success of the surgery can be checked and corrections can be made, if necessary, before closing the patient's chest.

In certain embodiments, activation of the shape memory material is synchronized with the heart beat during an imaging procedure. For example, an imaging technique can be used to focus HIFU energy onto an annuloplasty ring in a patient's body during a portion of the cardiac cycle. As the heart beats, the annuloplasty ring may move in and out of this area of focused energy. To reduce damage to the surrounding tissue, the patient's body is exposed to the HIFU energy only during portions of the cardiac cycle that focus the HIFU energy onto the cardiac ring. In certain embodiments, the energy is gated with a signal that represents the cardiac cycle such as an electrocardiogram signal. In certain such embodiments, the synchronization and gating is configured to allow delivery of energy to the shape memory materials at specific times during the cardiac cycle to avoid or reduce the likelihood of causing arrhythmia or fibrillation during vulnerable periods. For example, the energy can be gated so as to only expose the patient's heart to the energy during the T wave of the electrocardiogram signal.

As discussed above, shape memory materials include, for example, polymers, metals, and metal alloys including ferromagnetic alloys. Exemplary shape memory polymers that are usable for certain embodiments of the present invention are disclosed by Langer, et al. in U.S. Patent No. 6,720,402, issued April 13, 2004, U.S. Patent No. 6,388,043, issued May 14, 2002, and 6,160,084, issued December 12, 2000, each of which are hereby incorporated by reference herein. Shape memory polymers respond to changes in temperature by changing to one or more permanent or memorized shapes. In certain embodiments, the shape memory polymer is heated to a temperature between approximately 38 degrees Celsius and approximately 60 degrees Celsius. In certain embodiments, the shape memory polymer is heated to a temperature in a range between approximately 40 degrees Celsius and approximately 55 degrees Celsius. In certain embodiments, the shape memory polymer has a two-way shape memory effect wherein the shape memory polymer is heated to change it to a first memorized shape and cooled to change it to a second memorized shape. The shape memory polymer can be cooled, for example, by inserting or circulating a cooled fluid through a catheter.

Shape memory polymers implanted in a patient's body can be heated non-invasively using, for example, external light energy sources such as infrared, near-infrared, ultraviolet, microwave and/or visible light sources. Preferably, the light energy is selected to increase absorption by the shape memory polymer and reduce absorption by the surrounding tissue. Thus, damage to the tissue surrounding the shape memory polymer is reduced when the shape memory polymer is heated to change its shape. In certain embodiments, the shape memory polymer comprises gas bubbles or bubble containing liquids such as fluorocarbons and is heated by inducing a cavitation effect in the gas/liquid when exposed to HIFU energy. In certain embodiments, the shape memory polymer may be heated using electromagnetic fields and may be coated with a material that absorbs electromagnetic fields.

Certain metal alloys have shape memory qualities and respond to changes in temperature and/or exposure to magnetic fields. Exemplary shape memory alloys that respond to changes in temperature include titanium-nickel, copper-zinc-aluminum, copper-aluminum-nickel, iron-manganese-silicon, iron-nickel-aluminum, gold-cadmium, combinations of the foregoing, and the like. In certain embodiments, the shape memory alloy comprises a biocompatible material such as a titanium-nickel alloy.

Shape memory alloys exist in two distinct solid phases called martensite and austenite. The martensite phase is relatively soft and easily deformed, whereas the austenite phase is relatively stronger and less easily deformed. For example, shape memory alloys enter the austenite phase at a relatively high temperature and the martensite phase at a relatively low temperature. Shape memory alloys begin transforming to the martensite phase at a start temperature (Mₛ) and finish transforming to the martensite phase at a finish temperature (M_{f}). Similarly, such shape memory alloys begin transforming to the austenite phase at a start temperature (Aₛ) and finish transforming to the austenite phase at a finish temperature (A_{f}). Both transformations have a hysteresis. Thus, the Mₛ temperature and the A_{f} temperature are not coincident with each other, and the M_{f} temperature and the Aₛ temperature are not coincident with each other.

In certain embodiments, the shape memory alloy is processed to form a memorized shape in the austenite phase in the form of a ring or partial ring. The shape memory alloy is then cooled below the M_{f} temperature to enter the martensite phase and deformed into a larger or smaller ring. For example, in certain embodiments, the shape memory alloy is formed into a ring or partial ring that is larger than the memorized shape but still small enough to improve leaflet coaptation and reduce regurgitation in a heart valve upon being attached to the heart valve annulus. In certain such embodiments, the shape memory alloy is sufficiently malleable in the martensite phase to allow a user such as a physician to adjust the circumference of the ring in the martensite phase by hand to achieve a desired fit for a particular heart valve annulus. After the ring is attached to the heart valve annulus, the circumference of the ring can be adjusted non-invasively by heating the shape memory alloy to an activation temperature (e.g., temperatures ranging from the Aₛ temperature to the A_{f} temperature).

Thereafter, when the shape memory alloy is exposed to a temperature elevation and transformed to the austenite phase, the alloy changes in shape from the deformed shape to the memorized shape. Activation temperatures at which the shape memory alloy causes the shape of the annuloplasty ring to change shape can be selected and built into the annuloplasty ring such that collateral damage is reduced or eliminated in tissue adjacent the annuloplasty ring during the activation process. Exemplary A_{f} temperatures for suitable shape memory alloys range between approximately 45 degrees Celsius and approximately 70 degrees Celsius. Furthermore, exemplary Mₛ temperatures range between approximately 10 degrees Celsius and approximately 20 degrees Celsius, and exemplary M_{f} temperatures range between approximately -1 degrees Celsius and approximately 15 degrees Celsius. The size of the annuloplasty ring can be changed all at once or incrementally in small steps at different times in order to achieve the adjustment necessary to produce the desired clinical result.

Certain shape memory alloys may further include a rhombohedral phase, having a rhombohedral start temperature (Rₛ) and a rhombohedral finish temperature (R_{f}), that exists between the austenite and martensite phases. An example of such a shape memory alloy is a NiTi alloy, which is commercially available from Memry Corporation (Bethel, Connecticut). In certain embodiments, an exemplary Rₛ temperature range is between approximately 30 degrees Celsius and approximately 50 degrees Celsius, and an exemplary R_{f} temperature range is between approximately 20 degrees Celsius and approximately 35 degrees Celsius. One benefit of using a shape memory material having a rhombohedral phase is that in the rhomobohedral phase the shape memory material may experience a partial physical distortion, as compared to the generally rigid structure of the austenite phase and the generally deformable structure of the martensite phase.

Certain shape memory alloys exhibit a ferromagnetic shape memory effect wherein the shape memory alloy transforms from the martensite phase to the austenite phase when exposed to an external magnetic field. The term "ferromagnetic" as used herein is a broad term and is used in its ordinary sense and includes, without limitation, any material that easily magnetizes, such as a material having atoms that orient their electron spins to conform to an external magnetic field. Ferromagnetic materials include permanent magnets, which can be magnetized through a variety of modes, and materials, such as metals, that are attracted to permanent magnets. Ferromagnetic materials also include electromagnetic materials that are capable of being activated by an electromagnetic transmitter, such as one located outside the heart 100. Furthermore, ferromagnetic materials may include one or more polymer-bonded magnets, wherein magnetic particles are bound within a polymer matrix, such as a biocompatible polymer. The magnetic materials can comprise isotropic and/or anisotropic materials, such as for example NdFeB (Neodynium Iron Boron), SmCo (Samarium Cobalt), ferrite and/or AlNiCo (Aluminum Nickel Cobalt) particles.

Thus, an annuloplasty ring comprising a ferromagnetic shape memory alloy can be implanted in a first configuration having a first shape and later changed to a second configuration having a second (e.g., memorized) shape without heating the shape memory material above the Aₛ temperature. Advantageously, nearby healthy tissue is not exposed to high temperatures that could damage the tissue. Further, since the ferromagnetic shape memory alloy does not need to be heated, the size of the annuloplasty ring can be adjusted more quickly and more uniformly than by heat activation.

Exemplary ferromagnetic shape memory alloys include Fe-C, Fe-Pd, Fe-Mn-Si, Co-Mn, Fe-Co-Ni-Ti, Ni-Mn-Ga, Ni₂MnGa, Co-Ni-Al, and the like. Certain of these shape memory materials may also change shape in response to changes in temperature. Thus, the shape of such materials can be adjusted by exposure to a magnetic field, by changing the temperature of the material, or both.

In certain embodiments, combinations of different shape memory materials are used. For example, annuloplasty rings according to certain embodiments comprise a combination of shape memory polymer and shape memory alloy (e.g., NiTi). In certain such embodiments, an annuloplasty ring comprises a shape memory polymer tube and a shape memory alloy (e.g., NiTi) disposed within the tube. Such embodiments are flexible and allow the size and shape of the shape memory to be further reduced without impacting fatigue properties. In addition, or in certain embodiments, shape memory polymers are used with shape memory alloys to create a bi-directional (e.g., capable of expanding and contracting) annuloplasty ring. Bi-directional annuloplasty rings can be created with a wide variety of shape memory material combinations having different characteristics.

In the following description, reference is made to the accompanying drawings, which form a part hereof, and which show, by way of illustration, specific embodiments or processes in which the invention may be practiced. Where possible, the same reference numbers are used throughout the drawings to refer to the same or like components. In some instances, numerous specific details are set forth in order to provide a thorough understanding of the present disclosure. The present disclosure, however, may be practiced without the specific details or with certain alternative equivalent components and methods to those described herein. In other instances, well-known components and methods have not been described in detail so as not to unnecessarily obscure aspects of the present disclosure.

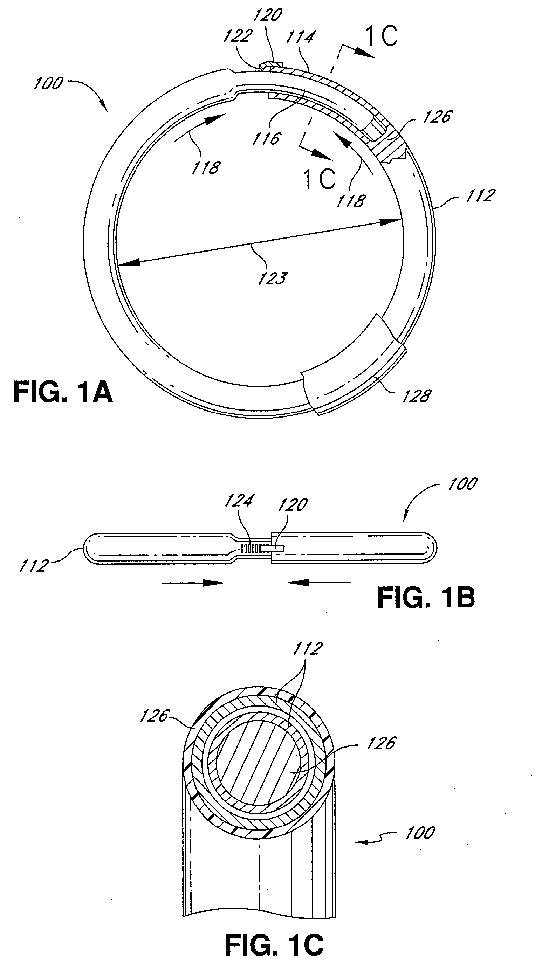

FIGS. 1A-1C illustrate an adjustable annuloplasty ring 100 according to certain embodiments that can be adjusted in vivo after implantation into a patient's body. The annuloplasty ring 100 has a substantially annular configuration and comprises a tubular body member 112 that folds back upon itself in a substantial circle having a nominal diameter as indicated by arrow 123. The tubular body member 112 comprises a receptacle end 114 and an insert end 116. The insert end 116 of the tubular member 112 is reduced in outer diameter or transverse dimension as compared to the receptacle end 114. As used herein, "dimension" is a broad term having its ordinary and customary meaning and includes a size or distance from a first point to a second point along a line or arc. For example, a dimension may be a circumference, diameter, radius, arc length, or the like. As another example, a dimension may be a distance between an anterior portion and a posterior portion of an annulus.

The receptacle end accepts the insert end 116 of the tubular member 112 to complete the ring-like structure of the annuloplasty ring 100. The insert end 116 slides freely within the receptacle end 114 of the annuloplasty ring 100 which allows contraction of the overall circumference of the ring 100 as the insert end 116 enters the receptacle end 114 as shown by arrows 118 in FIG. 1A. In certain embodiments, the nominal diameter or transverse dimension 123 of the annuloplasty ring 100 can be adjusted from approximately 25 mm to approximately 38 mm. However, an artisan will recognize from the disclosure herein that the diameter or transverse dimension 123 of the annuloplasty ring 100 can be adjusted to other sizes depending on the particular application. Indeed, the diameter or transverse dimension 123 of the annuloplasty ring 100 can be configured to reinforce body structures substantially smaller than 25 mm and substantially larger than 38 mm.

An artisan will recognize from the disclosure herein that in certain embodiments the insert end 116 can couple with the receptacle end 114 without being inserted in the receptacle end 114. For example, the insert end 116 can overlap the receptacle end 114 such that it slides adjacent thereto. In certain embodiments, for example, the ends 114, 116 may grooved to guide the movement of the adjacent ends 114, 116 relative to one another. Certain embodiments within the scope of the invention will occur to those skilled in the art.

The annuloplasty ring 100 also comprises a suturable material 128, shown partially cut away in FIG. 1A, and not shown in FIGS. 1B and 1C for clarity. The suturable material 128 is disposed about the tubular member 112 to facilitate surgical implantation of the annuloplasty ring 100 in a body structure, such as about a heart valve annulus. In certain embodiments, the suturable material 128 comprises a suitable biocompatible material such as Dacron@, woven velour, polyurethane, polytetrafluoroethylene (PTFE), heparin-coated fabric, or the like. In certain embodiments, the suturable material 128 comprises a biological material such as bovine or equine pericardium, homograft, patient graft, or cell-seeded tissue. The suturable material 128 may be disposed about the entire circumference of the tubular member 112, or selected portions thereof. For example, in certain embodiments, the suturable material 128 is disposed so as to enclose substantially the entire tubular member 112 except at the narrowed insert end 116 that slides into the receptacle end 118 of the tubular member 112.

As shown in FIGS. 1A and 1B, in certain embodiments, the annuloplasty ring 100 also comprises a ratchet member 120 secured to the receptacle end 114 of the tubular member 112. The ratchet member 120 comprises a pawl 122 configured to engage transverse slots 124 (shown in FIG. 1B) on the insert end 116 of the tubular member 112. The pawl 122 of the ratchet member 120 engages the slots 124 in such a way as to allow contraction of the circumference of the annuloplasty ring 100 and prevent or reduce circumferential expansion of the annuloplasty ring 100. Thus, the ratchet reduces unwanted circumferential expansion of the annuloplasty ring 100 after implantation due, for example, to dynamic forces on the annuloplasty ring 100 from the heart tissue during systolic contraction of the heart.

In certain embodiments, the tubular member 112 comprises a rigid material such as stainless steel, titanium, or the like, or a flexible material such as silicone rubber, Dacron@, or the like. In certain such embodiments, after implantation into a patient's body, the circumference of the annuloplasty ring 100 is adjusted in vivo by inserting a catheter (not shown) into the body and pulling a wire (not shown) attached to the tubular member 112 through the catheter to manually slide the insert end 116 of the tubular member 112 into the receptacle end 114 of the tubular member 112. As the insert end 116 slides into the receptacle end 114, the pawl 122 of the ratchet member 120 engages the slots 124 on the insert end 116 to hold the insert end 116 in the receptacle end 114. Thus, for example, as the size of a heart valve annulus reduces after implantation of the annuloplasty ring 100, the size of the annuloplasty ring 100 can also be reduced to provide an appropriate amount of reinforcement to the heart valve.

In certain embodiments, the tubular member 112 comprises a shape memory material that is responsive to changes in temperature and/or exposure to a magnetic field. As discussed above, the shape memory material may include shape memory polymers (e.g., polylactic acid (PLA), polyglycolic acid (PGA)) and/or shape memory alloys (e.g., nickel-titanium) including ferromagnetic shape memory alloys (e.g., Fe-C, Fe-Pd, Fe-Mn-Si, Co-Mn, Fe-Co-Ni-Ti, Ni-Mn-Ga, Ni₂MnGa, Co-Ni-Al). In certain such embodiments, the annuloplasty ring 100 is adjusted in vivo by applying an energy source such as radio frequency energy, X-ray energy, microwave energy, ultrasonic energy such as high intensity focused ultrasound (HIFU) energy, light energy, electric field energy, magnetic field energy, combinations of the foregoing, or the like. In certain embodiments, the energy source is applied in a non-invasive manner from outside the body. For example, as discussed above, a magnetic field and/or RF pulses can be applied to the annuloplasty ring 100 within a patient's body with an apparatus external to the patient's body such as is commonly used for magnetic resonance imaging (MRI). However, in certain embodiments, the energy source may be applied surgically such as by inserting a catheter into the body and applying the energy through the catheter.

In certain embodiments, the tubular body member 112 comprises a shape memory material that responds to the application of temperature that differs from a nominal ambient temperature, such as the nominal body temperature of 37 degrees Celsius for humans. The tubular member 112 is configured to respond by starting to contract upon heating the tubular member 112 above the As temperature of the shape memory material. In certain such embodiments, the annuloplasty ring 100 has an initial diameter or transverse dimension 123 of approximately 30 mm, and contracts or shrinks to a transverse dimension 123 of approximately 23 mm to approximately 28 mm, or any increment between those values. This produces a contraction percentage in a range between approximately 6 percent and approximately 23 percent, where the percentage of contraction is defined as a ratio of the difference between the starting diameter and finish diameter divided by the starting diameter.

The activation temperatures (e.g., temperatures ranging from the Aₛ temperature to the A_{f} temperature) at which the tubular member 112 contracts to a reduced circumference may be selected and built into the annuloplasty ring 100 such that collateral damage is reduced or eliminated in tissue adjacent the annuloplasty ring 100 during the activation process. Exemplary A_{f} temperatures for the shape memory material of the tubular member 112 at which substantially maximum contraction occurs are in a range between approximately 38 degrees Celsius and approximately 1310 degrees Celsius. In certain embodiments, the A_{f} temperature is in a range between approximately 39 degrees Celsius and approximately 75 degrees Celsius. For certain embodiments that include shape memory polymers for the tubular member 112, activation temperatures at which the glass transition of the material or substantially maximum contraction occur range between approximately 38 degrees Celsius and approximately 60 degrees Celsius. In other such embodiments, the activation temperature is in a range between approximately 40 degrees Celsius and approximately 59 degrees Celsius.

In certain embodiments, the tubular member 112 is shape set in the austenite phase to a remembered configuration during the manufacturing of the tubular member 112 such that the remembered configuration is that of a relatively small circumferential value with the insert end 116 fully inserted into the receptacle end 114. After cooling the tubular member 112 below the M_{f} temperature, the tubular member 112 is manually deformed to a larger circumferential value with the insert end 116 only partially inserted into the receptacle end 114 to achieve a desired starting nominal circumference for the annuloplasty ring 100. In certain such embodiments, the tubular member 112 is sufficiently malleable in the martensite phase to allow a user such as a physician to adjust the circumferential value by hand to achieve a desired fit with the heart valve annulus. In certain embodiments, the starting nominal circumference for the annuloplasty ring 100 is configured to improve leaflet coaptation and reduce regurgitation in a heart valve.

After implantation, the annuloplasty ring 100 is activated non-invasively by the application of energy to the patient's body to heat the tubular member 112. In certain embodiments, an MRI device is used as discussed above to heat the tubular member 112, which then causes the shape memory material of the tubular member 112 to transform to the austenite phase and remember its contracted configuration. Thus, the circumference of the annuloplasty ring 100 is reduced in vivo without the need for surgical intervention. Standard techniques for focusing the magnetic field from the MRI device onto the annuloplasty ring 100 may be used. For example, a conductive coil can be wrapped around the patient in an area corresponding to the annuloplasty ring 100. In certain embodiments, the shape memory material is activated by exposing it other sources of energy, as discussed above.

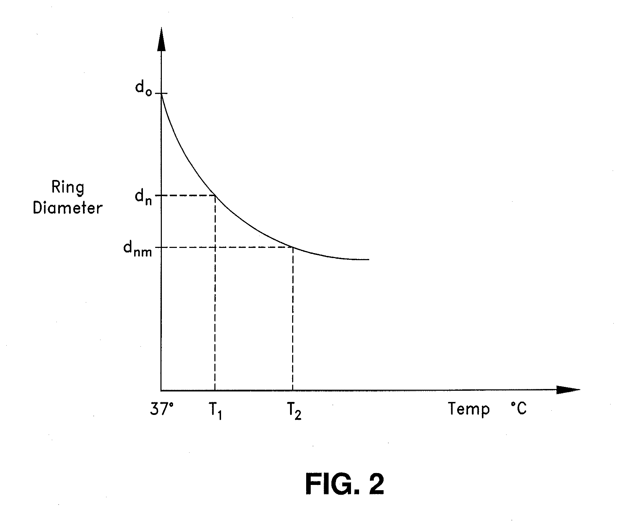

The circumference reduction process, either non-invasively or through a catheter, can be carried out all at once or incrementally in small steps at different times in order to achieve the adjustment necessary to produce the desired clinical result. If heating energy is applied such that the temperature of the tubular member 112 does not reach the A_{f} temperature for substantially maximum transition contraction, partial shape memory transformation and contraction may occur. FIG. 2 graphically illustrates the relationship between the temperature of the tubular member 112 and the diameter or transverse dimension 123 of the annuloplasty ring 100 according to certain embodiments. At body temperature of approximately 37 degrees Celsius, the diameter of the tubular member 112 has a first diameter do. The shape memory material is then increased to a first raised temperature T₁. In response, the diameter of the tubular member 112 reduces to a second diameter dₙ. The diameter of the tubular member 112 can then be reduced to a third diameter dₙₘ by raising the temperature to a second temperature T₂.

As graphically illustrated in FIG. 2, in certain embodiments, the change in diameter from do to dₙₘ is substantially continuous as the temperature is increased from body temperature to T₂. For example, in certain embodiments a magnetic field of about 2.5 Tesla to about 3.0 Tesla is used to raise the temperature of the tubular member 112 above the A_{f} temperature to complete the austenite phase and return the tubular member 112 to the remembered configuration with the insert end 116 fully inserted into the receptacle end 114. However, a lower magnetic field (e.g., 0.5 Tesla) can initially be applied and increased (e.g., in 0.5 Tesla increments) until the desired level of heating and desired contraction of the annuloplasty ring 100 is achieved. In certain embodiments, the tubular member 112 comprises a plurality of shape memory materials with different activation temperatures and the diameter of the tubular member 112 is reduced in steps as the temperature increases.

Whether the shape change is continuous or stepped, the diameter or transverse dimension 123 of the ring 100 can be assessed or monitored during the contraction process to determine the amount of contraction by use of MRI imaging, ultrasound imaging, computed tomography (CT), X-ray or the like. If magnetic energy is being used to activate contraction of the ring 100, for example, MRI imaging techniques can be used that produce a field strength that is lower than that required for activation of the annuloplasty ring 100.

In certain embodiments, the tubular member 112 comprises an energy absorption enhancement material 126. As shown in FIGS. 1A and 1C, the energy absorption enhancement material 126 may be disposed within an inner chamber of the tubular member 112. As shown in FIG. 1C (and not shown in FIG. 1A for clarity), the energy absorption enhancement material 126 may also be coated on the outside of the tubular member 112 to enhance energy absorption by the tubular member 112. For embodiments that use energy absorption enhancement material 126 for enhanced absorption, it may be desirable for the energy absorption enhancement material 126, a carrier material (not shown) surrounding the energy absorption enhancement material 126, if there is one, or both to be thermally conductive. Thus, thermal energy from the energy absorption enhancement material 126 is efficiently transferred to the shape memory material of the annuloplasty ring 100, such as the tubular member 112.

As discussed above, the energy absorption enhancement material 126 may include a material or compound that selectively absorbs a desired heating energy and efficiently converts the non-invasive heating energy to heat which is then transferred by thermal conduction to the tubular member 112. The energy absorption enhancement material 126 allows the tubular member 112 to be actuated and adjusted by the non-invasive application of lower levels of energy and also allows for the use of non-conducting materials, such as shape memory polymers, for the tubular member 112. For certain embodiments, magnetic flux ranging between about 2.5 Tesla and about 3.0 Tesla may be used for activation. By allowing the use of lower energy levels, the energy absorption enhancement material 126 also reduces thermal damage to nearby tissue. Suitable energy absorption enhancement materials 126 are discussed above.

In certain embodiments, a circumferential contraction cycle can be reversed to induce an expansion of the annuloplasty ring 100. Some shape memory alloys, such as NiTi or the like, respond to the application of a temperature below the nominal ambient temperature. After a circumferential contraction cycle has been performed, the tubular member 112 is cooled below the Mₛ temperature to start expanding the annuloplasty ring 100. The tubular member 112 can also be cooled below the M_{f} temperature to finish the transformation to the martensite phase and reverse the contraction cycle. As discussed above, certain polymers also exhibit a two-way shape memory effect and can be used to both expand and contract the annuloplasty ring 100 through heating and cooling processes. Cooling can be achieved, for example, by inserting a cool liquid onto or into the annuloplasty ring 100 through a catheter, or by cycling a cool liquid or gas through a catheter placed near the annuloplasty ring 100. Exemplary temperatures for a NiTi embodiment for cooling and reversing a contraction cycle range between approximately 20 degrees Celsius and approximately 30 degrees Celsius.

In certain embodiments, external stresses are applied to the tubular member 112 during cooling to expand the annuloplasty ring 100. In certain such embodiments, one or more biasing elements (not shown) are operatively coupled to the tubular member 112 so as to exert a circumferentially expanding force thereon. For example, in certain embodiments a biasing element such as a spring (not shown) is disposed in the receptacle end 114 of the tubular member 112 so as to push the insert end 16 at least partially out of the receptacle end 114 during cooling. In such embodiments, the tubular member 112 does not include the ratchet member 120 such that the insert end 116 can slide freely into or out of the receptacle end 114.

In certain embodiments, the tubular member comprises ferromagnetic shape memory material, as discussed above. In such embodiments, the diameter of the tubular member 112 can be changed by exposing the tubular member 112 to a magnetic field. Advantageously, nearby healthy tissue is not exposed to high temperatures that could damage the tissue. Further, since the shape memory material does not need to be heated, the size of the tubular member 112 can be adjusted more quickly and more uniformly than by heat activation.

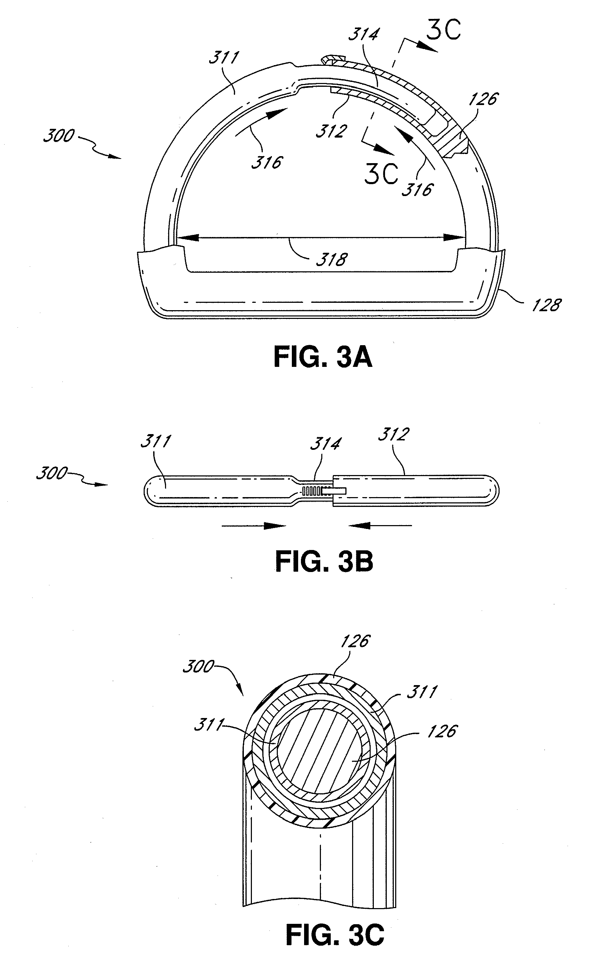

FIGS. 3A-3C illustrate an embodiment of an adjustable annuloplasty ring 300 that is similar to the annuloplasty ring 100 discussed above, but having a D-shaped configuration instead of a circular configuration. The annuloplasty ring 300 comprises a tubular body member 311 having a receptacle end 312 and an insert end 314 sized and configured to slide freely in the hollow receptacle end 312 in an axial direction which allows the annuloplasty ring 300 to constrict upon activation to a lesser circumference or transverse dimension as indicated by arrows 316. The annuloplasty ring 300 has a major transverse dimension indicated by arrow 318 that is reduced upon activation of the annuloplasty ring 300. The major transverse dimension indicated by arrow 318 can be the same as or similar to the transverse dimension indicated by arrow 123 discussed above. In certain embodiments, the features, dimensions and materials of the annuloplasty ring 300 are the same as or similar to the features, dimensions and materials of annuloplasty ring 100 discussed above. The D-shaped configuration of ring 32 allows a proper fit of the ring 32 with the morphology of some particular heart valves.

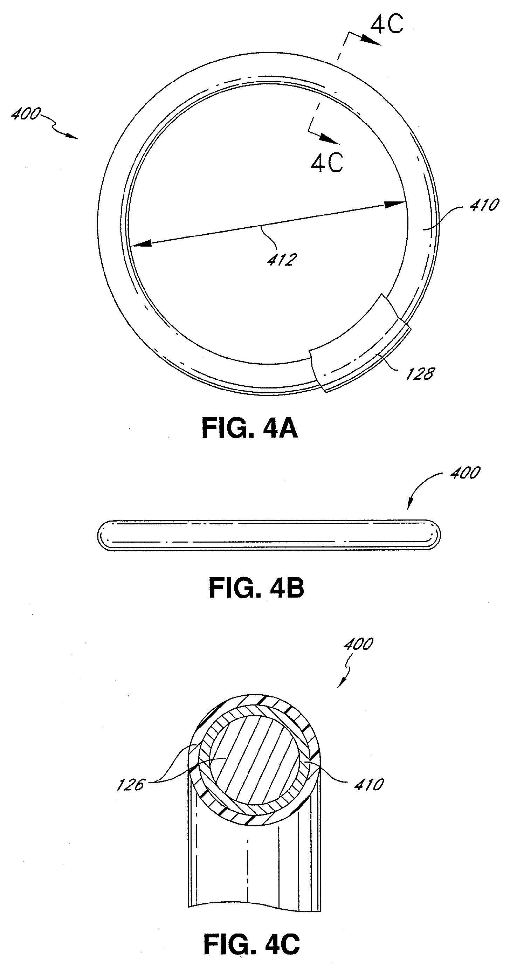

FIGS. 4A-4C show an embodiment of an annuloplasty ring 400 that includes a continuous tubular member 410 surrounded by a suturable material 128. The tubular member 410 has a substantially circular transverse cross section, as shown in FIG. 4C, and has an absorption enhancing material 126 disposed within an inner chamber of the tubular member 410. In certain embodiments, the absorption enhancing material 126 is also disposed on the outer surface of the tubular member 410. The tubular member 410 may be made from a shape memory material such as a shape memory polymer or a shape memory alloy including a ferromagnetic shape memory alloy, as discussed above.

For embodiments of the annuloplasty ring 400 with a tubular member 410 made from a continuous piece of shape memory alloy (e.g., NiTi alloy) or shape memory polymer, the annuloplasty ring 400 can be activated by the surgical and/or non-invasive application of heating energy by the methods discussed above with regard to certain embodiments. For embodiments of the annuloplasty ring 400 with a tubular member 410 made from a continuous piece of ferromagnetic shape memory alloy, the annuloplasty ring 400 can be activated by the non-invasive application of a suitable magnetic field. The annuloplasty ring 400 has a nominal inner diameter or transverse dimension indicated by arrow 412 in FIG. 4A that is set during manufacture of the ring 400. In certain embodiments, the annuloplasty ring 400 is sufficiently malleable when it is implanted into a patient's body that it can be adjusted by hand to be fitted to a particular heart valve annulus.

In certain embodiments, upon activating the tubular member 410 by the application of energy, the tubular member 410 remembers and assumes a configuration wherein the transverse dimension is less than the nominal transverse dimension 412. A contraction in a range between approximately 6 percent to approximately 23 percent may be desirable in certain embodiments which have continuous hoops of shape memory tubular members 410. In certain embodiments, the tubular member 410 comprises a shape memory NiTi alloy having an inner transverse dimension in a range between approximately 25 mm and approximately 38 mm. In certain such embodiments, the tubular member 410 can contract or shrink in a range between approximately 6 percent and approximately 23 percent, where the percentage of contraction is defined as a ratio of the difference between the starting diameter and finish diameter divided by the starting diameter. In certain embodiments, the annuloplasty ring 400 has a nominal inner transverse dimension 412 of approximately 30 mm and an inner transverse dimension in a range between approximately 23 mm and approximately 128 mm in a fully contracted state.

As discussed above in relation to FIG. 2, in certain embodiments, the inner transverse dimension 412 of certain embodiments can be altered as a function of the temperature of the tubular member 410. As also discussed above, in certain such embodiments, the progress of the size change can be measured or monitored in real-time conventional imaging techniques. Energy from conventional imaging devices can also be used to activate the shape memory material and change the inner transverse dimension 412 of the tubular member 410. In certain embodiments, the features, dimensions and materials of the annuloplasty ring 400 are the same as or similar to the features, dimensions and materials of the annuloplasty ring 100 discussed above. For example, in certain embodiments, the tubular member 410 comprises a shape memory material that exhibits a two-way shape memory effect when heated and cooled. Thus, the annuloplasty ring 400, in certain such embodiments, can be contracted and expanded.

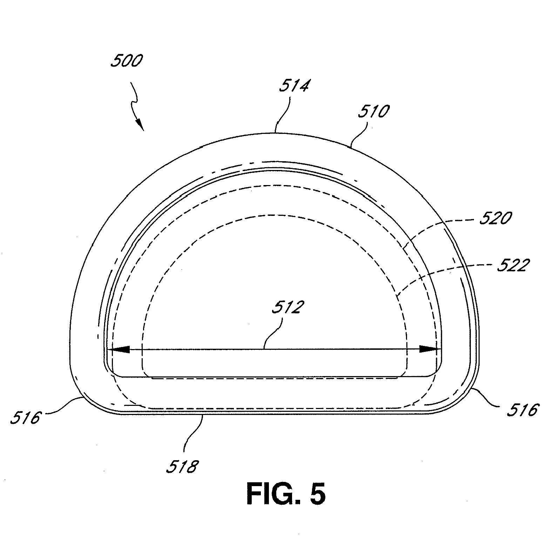

FIG. 5 illustrates a top view of an annuloplasty ring 500 having a D-shaped configuration according to certain embodiments. The annuloplasty ring 500 includes a continuous tubular member 510 comprising a shape memory material that has a nominal inner transverse dimension indicated by arrow 512 that may contract or shrink upon the activation of the shape memory material by surgically or non-invasive applying energy thereto, as discussed above. The tubular member 510 may comprise a homogeneous shape memory material, such as a shape memory polymer or a shape memory alloy including, for example, a ferromagnetic shape memory alloy.

Alternatively, the tubular member 510 may comprise two or more sections or zones of shape memory material having different temperature response curves. The shape memory response zones may be configured in order to achieve a desired configuration of the annuloplasty ring 500 as a whole when in a contracted state, either fully contracted or partially contracted. For example, the tubular member 510 may have a first zone or section 514 that includes the arched portion of the tubular member that terminates at or near the corners 516 and a second zone or section 518 that includes the substantially straight portion of the tubular member 510 disposed directly between the corners 516.

The annuloplasty ring 500 is shown in a contracted state in FIG. 5 as indicated by the dashed lines 520, 522, which represent contracted states of certain embodiments wherein both the first section 514 and second section 518 of the tubular member 510 have contracted axially. A suturable material (not shown), such as the suturable material 128 shown in FIG. 1, may be disposed about the tubular member 510 and the tubular member 510 may comprise or be coated with an energy absorption enhancement material 126, as discussed above. In certain embodiments, the features, dimensions and materials of the annuloplasty ring 500 are the same as or similar to the features, dimensions and materials of the annuloplasty ring 100 discussed above.

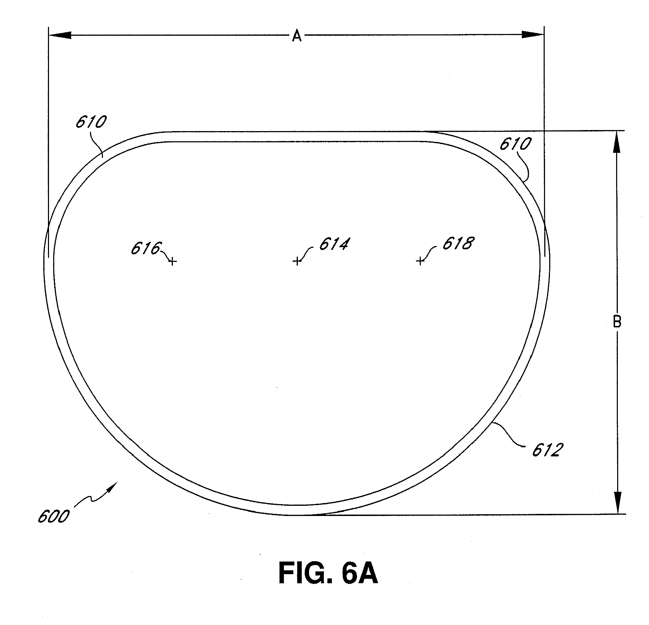

FIG. 6A is a schematic diagram of a top view of a substantially D-shaped wire 600 comprising a shape memory material according to certain embodiments of the invention. The term "wire" is a broad term having its normal and customary meaning and includes, for example, mesh, flat, round, rod-shaped, or band-shaped members. Suitable shape memory materials include shape memory polymers or shape memory alloys including, for example, ferromagnetic shape memory alloys, as discussed above. The wire 600 comprises a substantially linear portion 608, two corner portions 610, and a substantially semi-circular portion 612.

For purposes of discussion, the wire 600 is shown relative to a first reference point 614, a second reference point 616 and a third reference point 618. The radius of the substantially semi-circular portion 612 is defined with respect to the first reference point 614 and the corner portions 610 are respectively defined with respect to the second reference point 616 and the third reference point 618. Also for purposes of discussion, FIG. 6A shows a first transverse dimension A, a second transverse dimension B.

In certain embodiments, the first transverse dimension A is in a range between approximately 20.0 mm and approximately 40.0 mm, the second transverse dimension B is in a range between approximately 10.0 mm and approximately 25.0 mm. In certain such embodiments, the wire 600 comprises a rod having a diameter in a range between approximately 0.45 mm and approximately 0.55 mm, the radius of each corner portion 610 is in a range between approximately 5.8 mm and 7.2 mm, and the radius of the substantially semi-circular portion 612 is in a range between approximately 11.5 mm and approximately 14.0 mm. In certain other such embodiments, the wire 600 comprises a rod having a diameter in a range between approximately 0.90 mm and approximately 1.10 mm, the radius of each corner portion 610 is in a range between approximately 6.1 mm and 7.4 mm, and the radius of the substantially semi-circular portion 612 is in a range between approximately 11.7 mm and approximately 14.3 mm.

In certain embodiments, the first transverse dimension A is in a range between approximately 26.1 mm and approximately 31.9 mm, the second transverse dimension B is in a range between approximately 20.3 mm and approximately 24.9 mm. In certain such embodiments, the wire 600 comprises a rod having a diameter in a range between approximately 0.4 mm and approximately 0.6 mm, the radius of each corner portion 610 is in a range between approximately 6.7 mm and 8.3 mm, and the radius of the substantially semi-circular portion 612 is in a range between approximately 13.3 mm and approximately 16.2 mm. In certain other such embodiments, the wire 600 comprises a rod having a diameter in a range between approximately 0.90 mm and approximately 1.10 mm, the radius of each corner portion 610 is in a range between approximately 6.9 mm and 8.5 mm, and the radius of the substantially semi-circular portion 612 is in a range between approximately 13.5 mm and approximately 16.5 mm.

In certain embodiments, the wire 600 comprises a NiTi alloy configured to transition to its austenite phase when heated so as to transform to a memorized shape, as discussed above. In certain such embodiments, the first transverse dimension A of the wire 600 is configured to be reduced by approximately 10% to 25% when transitioning to the austenite phase. In certain such embodiments, the austenite start temperature Aₛ is in a range between approximately 33 degrees Celsius and approximately 43 degrees Celsius, the austenite finish temperature A_{f} is in a range between approximately 45 degrees Celsius and approximately 55 degrees Celsius, the martensite start temperature Mₛ is less than approximately 30 degrees Celsius, and the martensite finish temperature M_{f} is greater than approximately 20 degrees Celsius. In certain embodiments, the austenite finish temperature A_{f} is in a range between approximately 48.75 degrees Celsius and approximately 51.25 degrees Celsius. Certain embodiments can include other start and finish temperatures for martensite, rhombohedral and austenite phases as described herein.

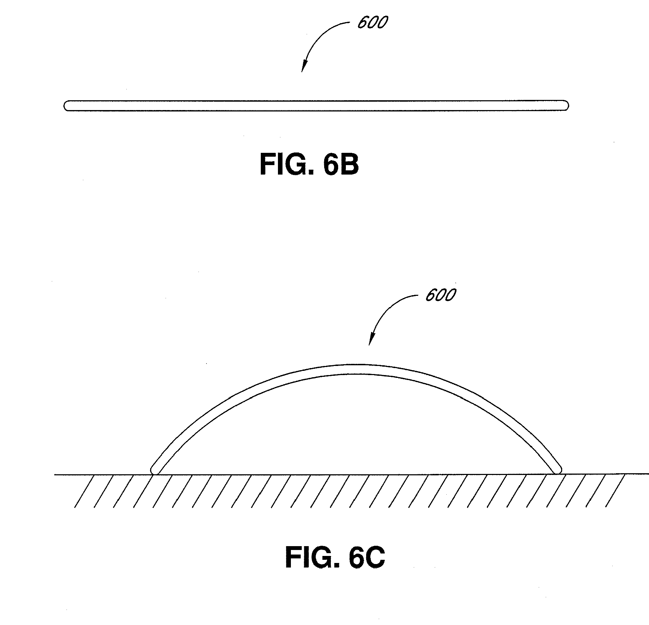

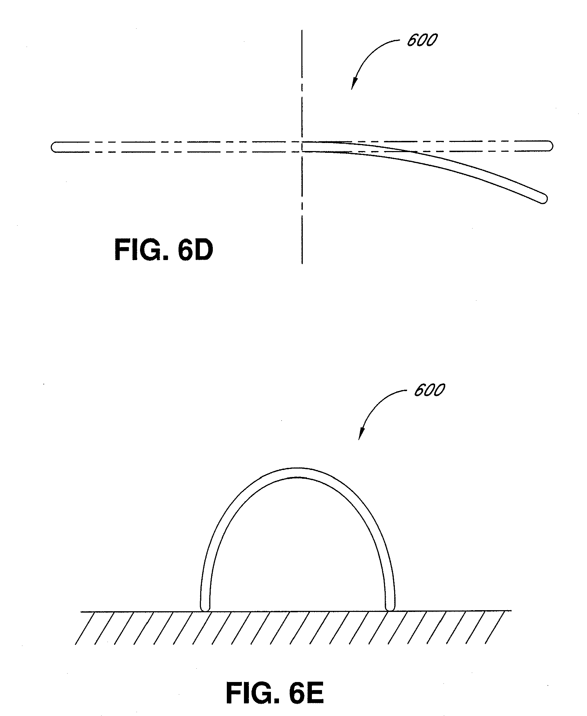

FIGS. 6B-6E are schematic diagrams of side views of the shape memory wire 600 of FIG. 6A according to certain embodiments. In addition to expanding and/or contracting the first transverse dimension A and/or the second transverse dimension B when transitioning to the austenite phase, in certain embodiments the shape memory wire 600 is configured to change shape in a third dimension perpendicular to the first transverse dimension A and the second transverse dimension B. For example, in certain embodiments, the shape memory wire 600 is substantially planar or flat in the third dimension, as shown in FIG. 6B, when implanted into a patient's body. Then, after implantation, the shape memory wire 600 is activated such that it expands or contracts in the first transverse dimension A and/or the second transverse dimension B and flexes or bows in the third dimension such that it is no longer planar, as shown in FIG. 6C. Such bowing may be symmetrical as shown in FIG. 6C or asymmetrical as shown in FIG. 6D to accommodate the natural shape of the annulus.

In certain embodiments, the shape memory wire 600 is configured to bow in the third dimension a distance in a range between approximately 2 millimeters and approximately 10 millimeters. In certain embodiments, the shape memory wire 600 is implanted so as to bow towards the atrium when implanted around a cardiac valve annulus to accommodate the natural shape of the annulus. In certain embodiments, the shape memory wire 600 is configured to bow towards the ventricle when implanted around a cardiac valve to accommodate the natural shape of the annulus.

In certain embodiments, the shape memory wire 600 is bowed in the third dimension, as shown in FIG. 6C, when implanted into the patient's body. Then, after implantation, the shape memory wire 600 is activated such that it expands or contracts in the first transverse dimension A and/or the second transverse dimension B and further flexes or bows in the third dimension, as shown in FIG. 6E. In certain embodiments, the shape memory wire 600 is bowed in the third dimension, as shown in FIG. 6C, when implanted into the patient's body. Then, after implantation, the shape memory wire 600 is activated such that it expands or contracts in the first transverse dimension A and/or the second transverse dimension B and changes shape in the third dimension so as to become substantially flat, as shown in FIG. 6B. An artisan will recognize from the disclosure herein that other annuloplasty rings disclosed herein can also be configured to bow or change shape in a third dimension so as to accommodate or further reinforce a valve annulus.

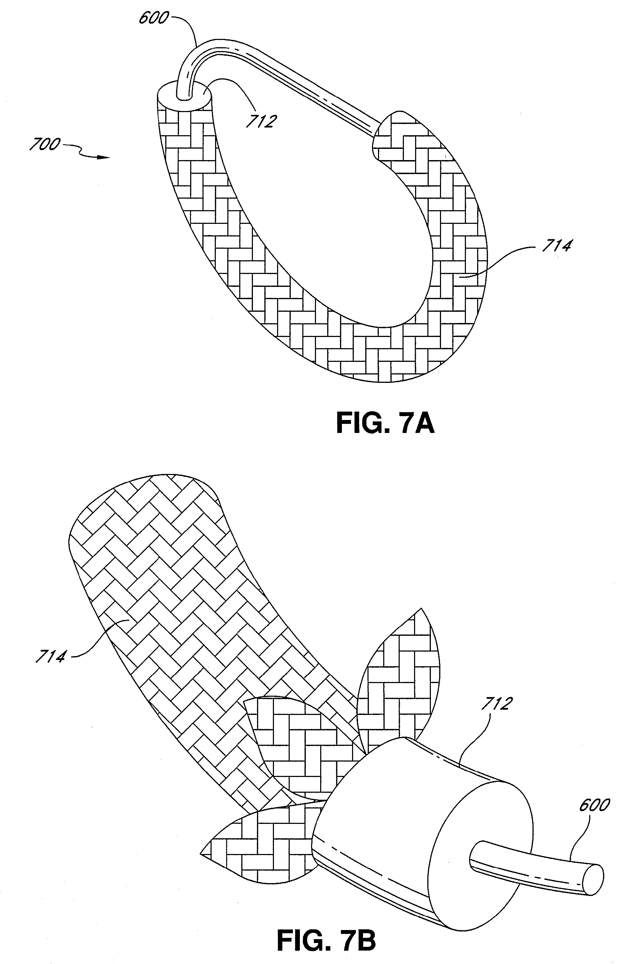

FIG. 7A is a perspective view illustrating portions of an annuloplasty ring 700 comprising the wire 600 shown in FIG. 6A according to certain embodiments of the invention. The wire 600 is covered by a flexible material 712 such as silicone rubber and a suturable material 714 such as woven polyester cloth, Dacron@, woven velour, polyurethane, polytetrafluoroethylene (PTFE), heparin-coated fabric, or other biocompatible material. In certain embodiments, the suturable material 714 comprises a biological material such as bovine or equine pericardium, homograft, patient graft, or cell-seeded tissue. For illustrative purposes, portions of the flexible material 712 and the suturable material 714 are not shown in FIG. 7A to show the wire 600. However, in certain embodiments, the flexible material 712 and the suturable material 714 are continuous and cover substantially the entire wire 600. Although not shown, in certain embodiments, the wire 600 is coated with an energy absorption enhancement material, as discussed above.

FIG. 7B is an enlarged perspective view of a portion of the annuloplasty ring 700 shown in FIG. 7A. For illustrative purposes, portions of the flexible material 712 are not shown to expose the wire 600 and portions of the suturable material 714 are shown peeled back to expose the flexible material 712. In certain embodiments, the diameter of the flexible material 712 is in a range between approximately 0.10 inches and approximately 0.15 inches. FIG. 7B shows the wire 600 substantially centered within the circumference of the flexible material 712. However, in certain embodiments, the wire 600 is offset within the circumference of the flexible material 712 to allow more space for sutures.

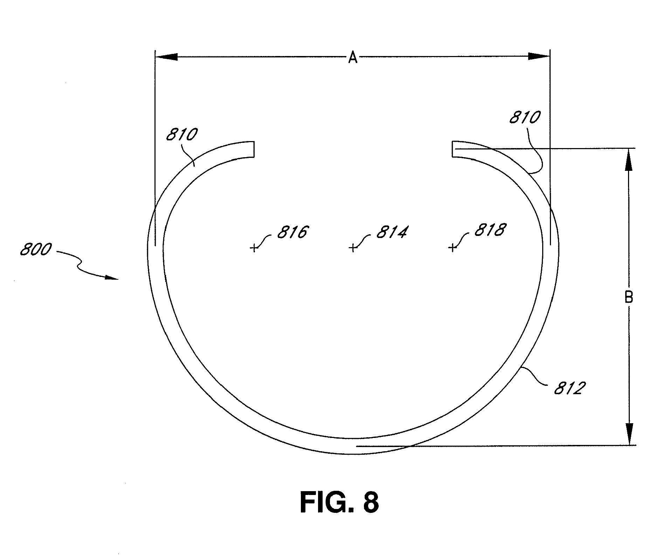

FIG. 8 is a schematic diagram of a substantially C-shaped wire 800 comprising a shape memory material according to certain embodiments of the invention. Suitable shape memory materials include shape memory polymers or shape memory alloys including, for example, ferromagnetic shape memory alloys, as discussed above. The wire 800 comprises two corner portions 810, and a substantially semi-circular portion 812.

For purposes of discussion, the wire 800 is shown relative to a first reference point 814, a second reference point 816 and a third reference point 818. The radius of the substantially semi-circular portion 812 is defined with respect to the first reference point 814 and the corner portions 810 are respectively defined with respect to the second reference point 816 and the third reference point 818. Also for purposes of discussion, FIG. 8 shows a first transverse dimension A and a second transverse dimension B. In certain embodiments, the wire 800 comprises a rod having a diameter and dimensions A and B as discussed above in relation to FIG. 6A.

In certain embodiments, the wire 800 comprises a NiTi alloy configured to transition to its austenite phase when heated so as to transform to a memorized shape, as discussed above. In certain such embodiments, the first transverse dimension A of the wire 800 is configured to be reduced by approximately 10% to 25% when transitioning to the austenite phase. In certain such embodiments, the austenite start temperature Aₛ is in a range between approximately 33 degrees Celsius and approximately 43 degrees Celsius, the austenite finish temperature A_{f} is in a range between approximately 45 degrees Celsius and approximately 55 degrees Celsius, the martensite start temperature Mₛ is less than approximately 30 degrees Celsius, and the martensite finish temperature M_{f} is greater than approximately 20 degrees Celsius. In certain embodiments, the austenite finish temperature A_{f} is in a range between approximately 48.75 degrees Celsius and approximately 51.25 degrees Celsius.

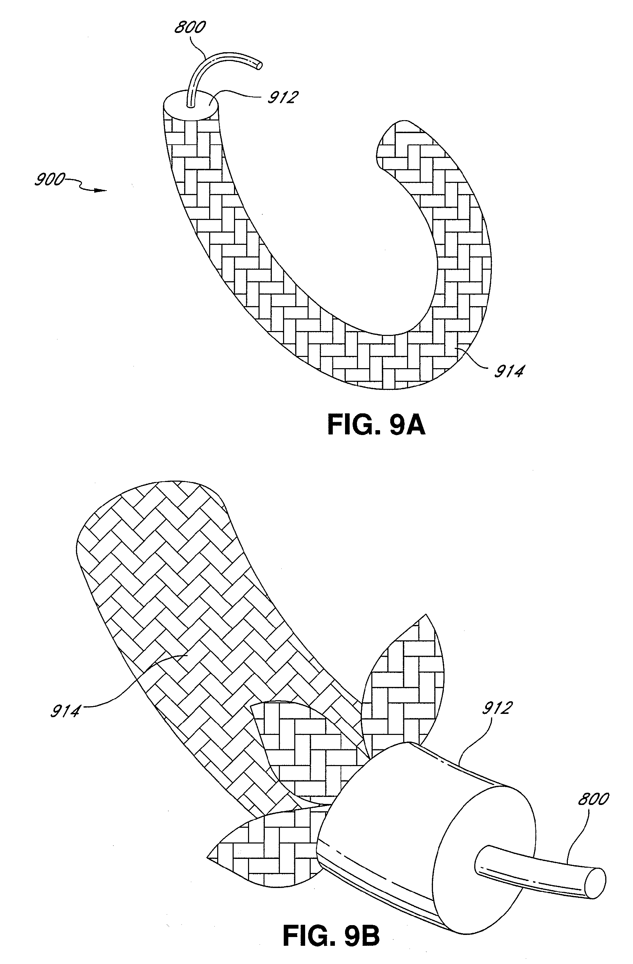

FIG. 9A is a perspective view illustrating portions of an annuloplasty ring 900 comprising the wire 800 shown in FIG. 8 according to certain embodiments of the invention. The wire 800 is covered by a flexible material 912 such as silicone rubber and a suturable material 914 such as woven polyester cloth, Dacron@, woven velour, polyurethane, polytetrafluoroethylene (PTFE), heparin-coated fabric, or other biocompatible material. In certain embodiments, the suturable material 914 comprises a biological material such as bovine or equine pericardium, homograft, patient graft, or cell-seeded tissue. For illustrative purposes, portions of the flexible material 912 and the suturable material 914 are not shown in FIG. 9A to show the wire 800. However, in certain embodiments, the flexible material 912 and the suturable material 914 cover substantially the entire wire 800. Although not shown, in certain embodiments, the wire 800 is coated with an energy absorption enhancement material, as discussed above.

FIG. 9B is an enlarged perspective view of a portion of the annuloplasty ring 900 shown in FIG. 9A. For illustrative purposes, portions of the flexible material 912 are not shown to expose the wire 800 and portions of the suturable material 914 are shown peeled back to expose the flexible material 912. In certain embodiments, the diameter of the flexible material 912 is in a range between approximately 0.10 inches and approximately 0.15 inches. FIG. 9B shows the wire 800 substantially centered within the circumference of the flexible material 912. However, in certain embodiments, the wire 800 is offset within the circumference of the flexible material 912 to allow more space for sutures.

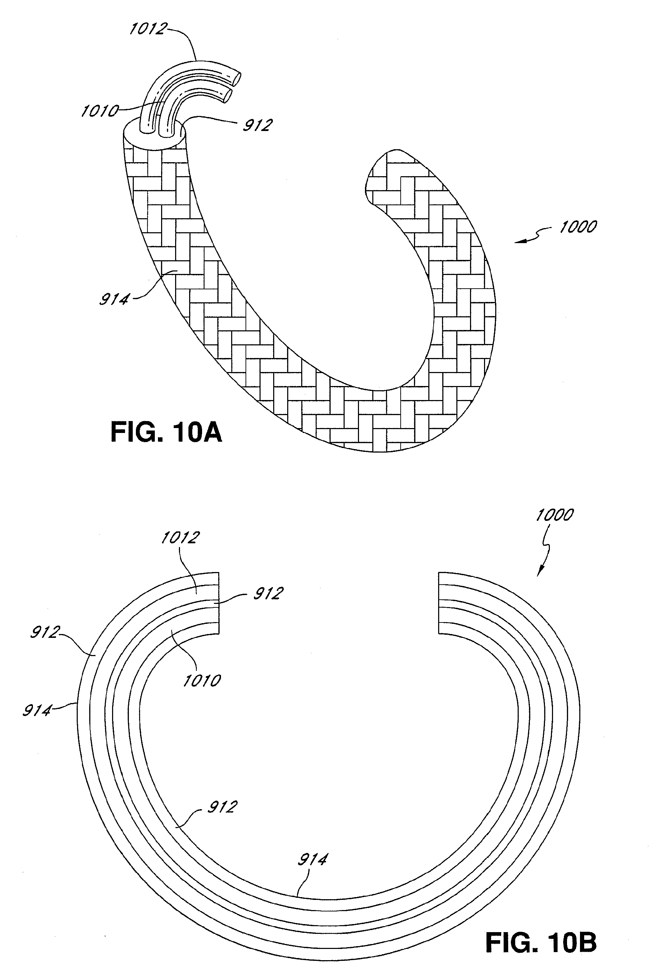

FIG. 10A is a perspective view illustrating portions of an annuloplasty ring 1000 configured to contract and expand according to certain embodiments of the invention. FIG. 10B is a top cross-sectional view of the annuloplasty ring 1000. As discussed above, after the annuloplasty ring 1000 has been contracted, it may become necessary to expand the annuloplasty ring 1000. For example, the annuloplasty ring 1000 may be implanted in a child with an enlarged heart. When the size of the heart begins to recover to its natural size, the annuloplasty ring 1000 can be contracted. Then, as the child gets older and the heart begins to grow, the annuloplasty ring 1000 can be enlarged as needed.

The annuloplasty ring 1000 comprises a first shape memory wire 1010 for contracting the annuloplasty ring 1000 and a second shape memory wire 1012 for expanding the annuloplasty ring 1000. The first and second shape memory wires, 1010, 1012 are covered by the flexible material 912 and the suturable material 914 shown in FIGS. 9A-9B. For illustrative purposes, portions of the flexible material 912 and the suturable material 914 are not shown in FIG. 10A to show the shape memory wires 1010, 1012. However, as schematically illustrated in FIG. 10B, in certain embodiments, the flexible material 912 and the suturable material 914 substantially cover the first and second shape memory wires 1010, 1012. As discussed below, the flexible material 912 operatively couples the first shape memory wire 1010 and the second shape memory wire 1012 such that a shape change in one will mechanically effect the shape of the other. The first and second shape memory wires 1010, 1012 each comprise a shape memory material, such as the shape memory materials discussed above. However, the first and second shape memory wires 1010, 1012 are activated at different temperatures.

In certain embodiments, the annuloplasty ring 1000 is heated to a first temperature that causes the first shape memory wire 1010 to transition to its austenite phase and contract to its memorized shape. At the first temperature, the second shape memory wire 1012 is in its martensite phase and is substantially flexible as compared the contracted first shape memory wire 1010. Thus, when the first shape memory wire 1010 transitions to its austenite phase, it exerts a sufficient force on the second shape memory wire 1012 through the flexible material 912 to deform the second shape memory wire 1012 and cause the annuloplasty ring 1000 to contract.

The annuloplasty ring 1000 can be expanded by heating the annuloplasty ring to a second temperature that causes the second shape memory wire 1012 to transition to its austenite phase and expand to its memorized shape. In certain embodiments, the second temperature is higher than the first temperature. Thus, at the second temperature, both the first and second shape memory wires 1010, 1012 are in their respective austenite phases. In certain such embodiments, the diameter of the second shape memory wire 1012 is sufficiently larger than the diameter of the first shape memory wire 1010 such that the second memory shape wire 1012 exerts a greater force to maintain its memorized shape in the austenite phase than the first shape memory wire 1010. Thus, the first shape memory wire 1010 is mechanically deformed by the force of the second memory shape wire 1012 and the annuloplasty ring 1000 expands.

In certain embodiments, the first memory shape wire 1010 is configured to contract by approximately 10% to 25% when transitioning to its austenite phase. In certain such embodiments, the first memory shape wire 1010 has an austenite start temperature Aₛ in a range between approximately 33 degrees Celsius and approximately 43 degrees Celsius, an austenite finish temperature A_{f} in a range between approximately 45 degrees Celsius and approximately 55 degrees Celsius, a martensite start temperature Mₛ less than approximately 30 degrees Celsius, and a martensite finish temperature M_{f} greater than approximately 20 degrees Celsius. In certain embodiments, the austenite finish temperature A_{f} of the first memory shape wire 1010 is in a range between approximately 48.75 degrees Celsius and approximately 51.25 degrees Celsius.

In certain embodiments, the second memory shape wire 1012 is configured to expand by approximately 10% to 25% when transitioning to its austenite phase. In certain such embodiments, the second memory shape wire 1010 has an austenite start temperature Aₛ in a range between approximately 60 degrees Celsius and approximately 70 degrees Celsius, an austenite finish temperature A_{f} in a range between approximately 65 degrees Celsius and approximately 75 degrees Celsius, a martensite start temperature Mₛ less than approximately 30 degrees Celsius, and a martensite finish temperature M_{f} greater than approximately 20 degrees Celsius. In certain embodiments, the austenite finish temperature A_{f} of the first memory shape wire 1010 is in a range between approximately 68.75 degrees Celsius and approximately 71.25 degrees Celsius.

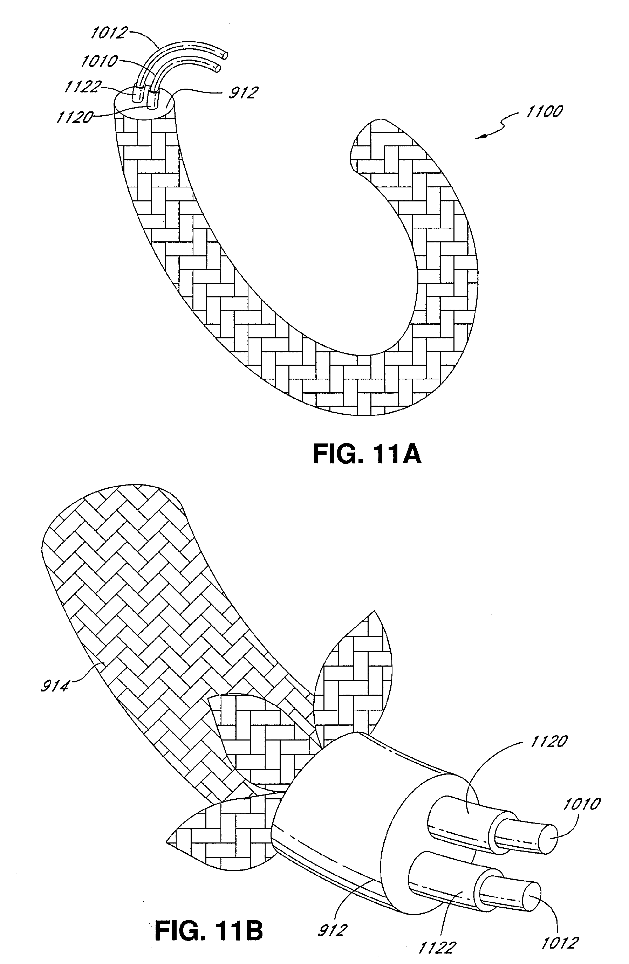

**FIG.** 11A is a perspective view illustrating portions of an annuloplasty ring 1100 according to certain embodiments comprising the first shape memory wire 1010 for contraction, the second shape memory wire 1012 for expansion, the flexible material 912 and the suturable material 914 shown in FIGS. 10A-10B. For illustrative purposes, portions of the flexible material 912 and the suturable material 914 are not shown in FIG. 11A to show the shape memory wires 1010, 1012. However, in certain embodiments, the flexible material 912 and the suturable material 914 substantially cover the first and second shape memory wires 1010, 1012. FIG. 11B is an enlarged perspective view of a portion of the annuloplasty ring 1100 shown in FIG. 11A. For illustrative purposes, portions of the flexible material 912 are not shown to expose the first and second shape memory wires 1010, 1012 and portions of the suturable material 914 are shown peeled back to expose the flexible material 912.

The first shape memory wire 1010 comprises a first coating 1120 and the second shape memory wire 1012 comprises a second coating 1122. In certain embodiments, the first coating 1120 and the second coating 1122 each comprise silicone tubing configured to provide suture attachment to a heart valve annulus. In certain embodiments, the first coating 1120 and the second coating 1122 each comprise an energy absorption material, such as the energy absorption materials discussed above. In certain such embodiments, the first coating 1120 heats when exposed to a first form of energy and the second coating 1122 heats when exposed to a second form of energy. For example, the first coating 1120 may heat when exposed to MRI energy and the second coating 1122 may heat when exposed to HIFU energy. As another example, the first coating 1120 may heat when exposed to RF energy at a first frequency and the second coating 1122 may heat when exposed to RF energy at a second frequency. Thus, the first shape memory wire 1010 and the second shape memory wire 1012 can be activated independently such that one transitions to its austenite phase while the other remains in its martensite phase, resulting in contraction or expansion of the annuloplasty ring 1100.

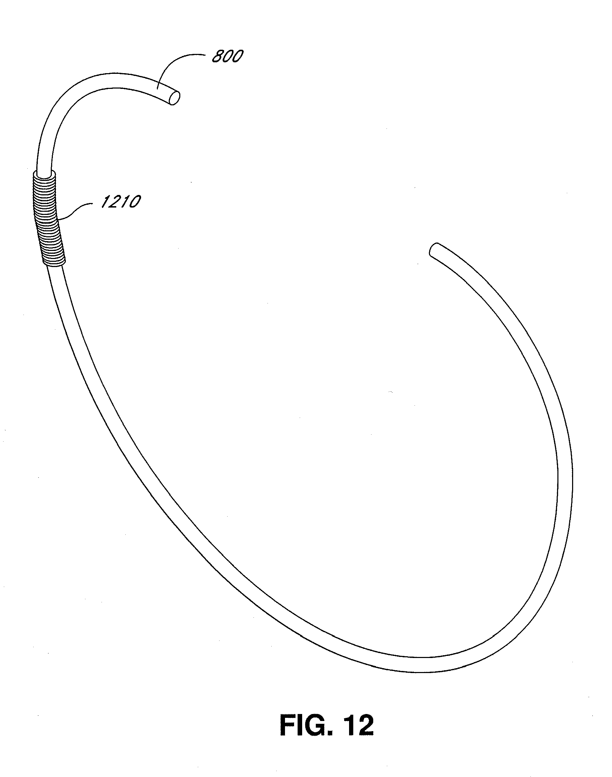

FIG. 12 is a perspective view of a shape memory wire 800, such as the wire 800 shown in FIG. 8, wrapped in an electrically conductive coil 1210 according to certain embodiments of the invention. The coil 1210 is wrapped around a portion of the wire 800 where it is desired to focus energy and heat the wire 800. In certain embodiments, the coil 1210 is wrapped around approximately 5% to approximately 15% of the wire 800. In certain embodiments, the coil 1210 is wrapped around approximately 15% to approximately 70% of the wire 800. In certain embodiments, the coil 1210 is wrapped around substantially the entire wire 800. Although not shown, in certain embodiments, the wire 800 also comprises a coating comprising an energy absorption material, such as the energy absorption materials discussed above. The coating may or may not be covered by the coil 1210.

As discussed above, an electrical current can be non-invasively induced in the coil 1210 using electromagnetic energy. For example, in certain embodiments, a handheld or portable device (not shown) comprising an electrically conductive coil generates an electromagnetic field that non-invasively penetrates the patient's body and induces a current in the coil 1210. The electrical current causes the coil 1210 to heat. The coil 1210, the wire 800 and the coating (if any) are thermally conductive so as to transfer the heat or thermal energy from the coil 1210 to the wire 800. Thus, thermal energy can be directed to the wire 800, or portions thereof, while reducing thermal damage to surrounding tissue.

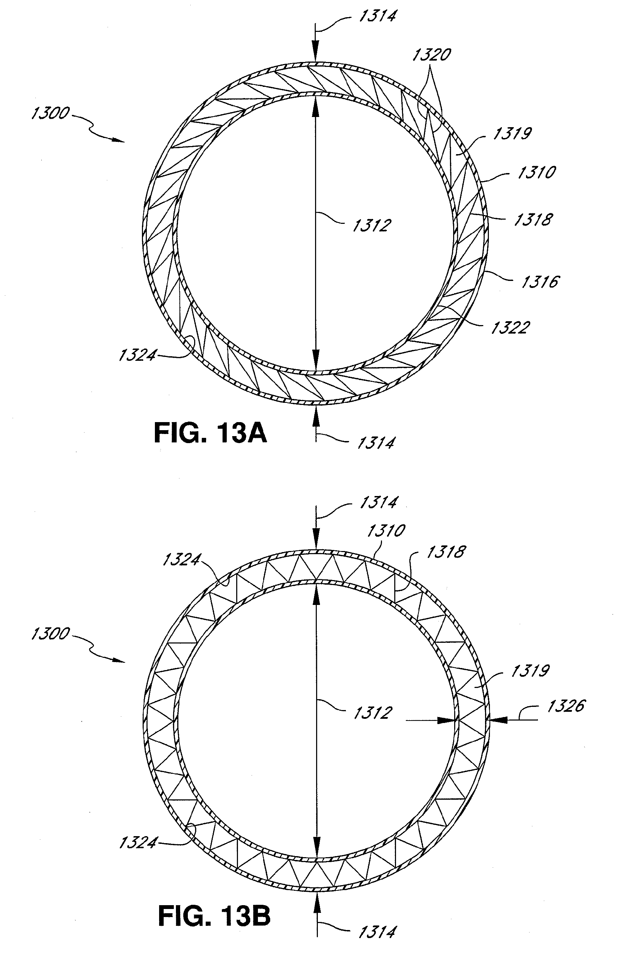

FIGS. 13A and 13B show an embodiment of an annuloplasty ring 1310 having a nominal inner diameter or transverse dimension indicated by arrow 1312 and a nominal outer diameter or transverse dimension indicated by arrows 1314. The ring 1310 includes a tubular member 1316 having a substantially round transverse cross section with an internal shape memory member 1318 disposed within an inner chamber 1319 of the tubular member 1316. The internal shape memory member 1318 is a ribbon or wire bent into a series of interconnected segments 1320. Upon heating of the tubular member 1316 and the internal shape memory member 1318, the inner transverse dimension 1312 becomes smaller due to axial shortening of the tubular member 1316 and an inward radial force applied to an inner chamber surface 1322 of the tubular member 1316 by the internal shape memory member 1318. The internal shape memory member 1318 is expanded upon heating such that the ends of segments 1320 push against the inner chamber surface 1322 and outer chamber surface 1324, as shown by arrow 1326 in FIG. 13B, and facilitate radial contraction of the inner transverse dimension 1312. Thus, activation of the internal shape memory member 1318 changes the relative distance between the against the inner chamber surface 1322 and outer chamber surface 1324.

Although not shown in FIGS. 13A or 13B, The inner shape memory member 1318 may also have a heating energy absorption enhancement material, such as one or more of the energy absorption enhancement materials discussed above, disposed about it within the inner chamber 1319. The energy absorption material may also be coated on an outer surface and/or an inner surface of the tubular member 1316. The inner transverse dimension 1312 of the ring 1310 in FIG. 13B is less than the inner transverse dimension 1312 of the ring 1310 shown in FIG. 13A. However, according to certain embodiments, the outer transverse dimension 1314 is substantially constant in both FIGS. 13A and 13B.

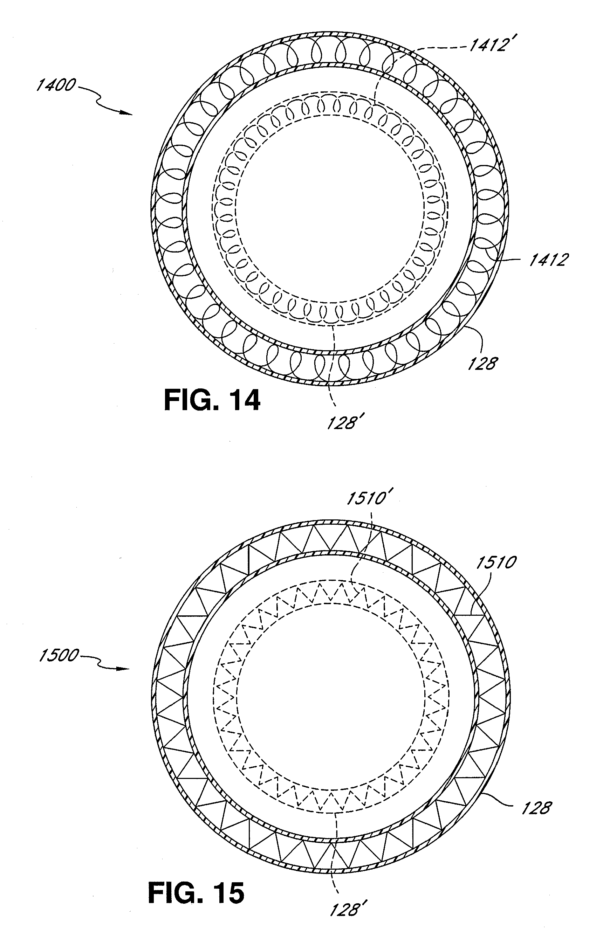

For some indications, it may be desirable for an adjustable annuloplasty ring to have some compliance in order to allow for expansion and contraction of the ring in concert with the expansion and contraction of the heart during the beating cycle or with the hydrodynamics of the pulsatile flow through the valve during the cycle. As such, it may be desirable for an entire annuloplasty ring, or a section or sections thereof, to have some axial flexibility to allow for some limited and controlled expansion and contraction under clinical conditions. FIGS. 14 and 15 illustrate embodiments of adjustable annuloplasty rings that allow some expansion and contraction in a deployed state.

FIG. 14 shows an annuloplasty ring 1400 that is constructed in such a way that it allows mechanical expansion and compression of the ring 1400 under clinical conditions. The ring 1400 includes a coil 1412 made of a shape memory material, such as one or more of the shape memory materials discussed above. The shape memory material or other portion of the ring 1400 may be coated with an energy absorption material, such as the energy absorption materials discussed above. The coil 1412 may have a typical helical structure of a normal spring wire coil, or alternatively, may have another structure such as a ribbon coil. In certain embodiments, the coil 1412 is surrounded by a suturable material 128, such as Dacron@ or the other suturable materials discussed herein. The coiled structure or configuration of the coil 1412 allows the ring 1400 to expand and contract slightly when under physiological pressures and forces from heart dynamics or hydrodynamics of blood flow through a host heart valve.

For embodiments where the coil 1412 is made of NiTi alloy or other shape memory material, the ring 1400 is responsive to temperature changes which may be induced by the application of heating energy on the coil 1412. In certain embodiments, if the temperature is raised, the coil 1412 will contract axially or circumferentially such that an inner transverse dimension of the ring 1400 decreases, as shown by the dashed lines in FIG. 14. In FIG. 14, reference 1412' represents the coil 1412 in its contracted state and reference 128' represents the suturable material 128 in its contracted state around the contracted coil 1412'. In addition, or in certain embodiments, the coil 1412 expands axially or circumferentially such that the inner transverse dimension of the ring 1400 increases. Thus, in certain embodiments, the ring 1400 can be expanded and contracted by applying invasive or non-invasive energy thereto.

FIG. 15 illustrates another embodiment of an adjustable annuloplasty ring 1500 that has dynamic compliance with dimensions, features and materials that may be the same as or similar to those of ring 1400. However, the ring 1500 has a zig-zag ribbon member 1510 in place of the coil 1412 in the embodiment of FIG. 14. In certain embodiments, if the temperature is raised, the ribbon member 1510 will contract axially or circumferentially such that an inner transverse dimension of the ring 1500 decreases, as shown by the dashed lines in FIG. 15. In FIG. 15, reference 1510' represents the ribbon member 1510 in its contracted state and reference 128' represents the suturable material 128 in its contracted state around the contracted ribbon member 1510'. In addition, or in certain embodiments, the ribbon member 1510 expands axially or circumferentially such that the inner transverse dimension of the ring 1500 increases. Thus, in certain embodiments, the ring 1500 can be expanded and contracted by applying invasive or non-invasive energy thereto.

The embodiments of FIGS. 14 and 15 may have a substantially circular configuration as shown in the figures, or may have D-shaped or C-shaped configurations as shown with regard to certain embodiments discussed above. In certain embodiments, the features, dimensions and materials of rings 1400 and 1500 are the same as or similar to the features, dimensions and materials of the annuloplasty ring 400 discussed above.

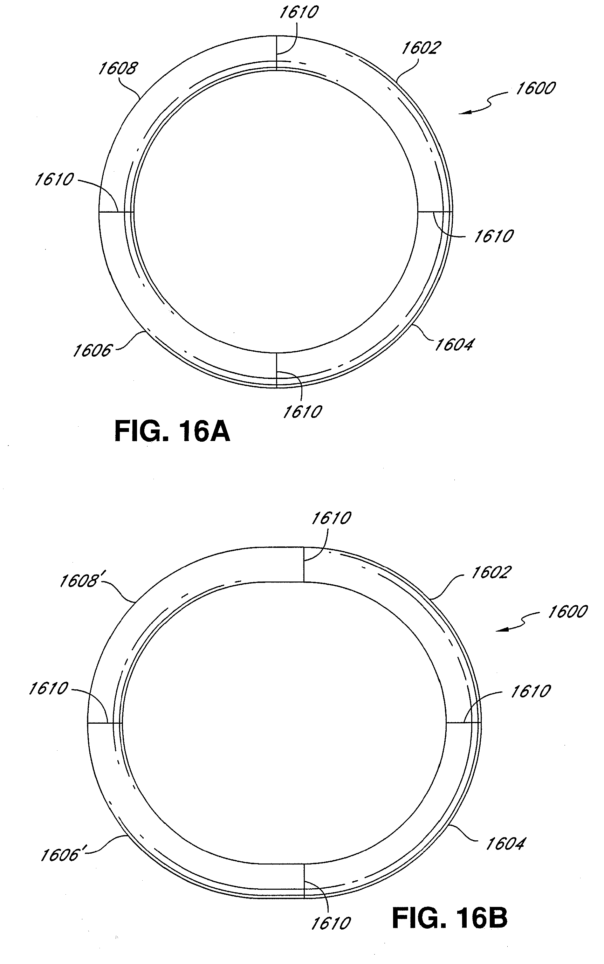

FIGS. 16A and 16B illustrate an annuloplasty ring 1600 according to certain embodiments that has a substantially circular shape or configuration when in the non-activated state shown in FIG. 16A. The ring 1600 comprises shape memory material or materials which are separated into a first temperature response zone 1602, a second temperature response zone 1604, a third temperature response zone 1606 and a fourth temperature response zone 1608. The zones are axially separated by boundaries 1610. Although the ring 1600 is shown with four zones 1602, 1604, 1606, 1608, an artisan will recognize from the disclosure herein that certain embodiments may include two or more zones of the same or differing lengths. For example, one embodiment of an annuloplasty ring 1600 includes approximately three to approximately eight temperature response zones.

In certain embodiments, the shape memory materials of the various temperature response zones 1602, 1604, 1606, 1608 are selected to have temperature responses and reaction characteristics such that a desired shape and configuration can be achieved in vivo by the application of invasive or non-invasive energy, as discussed above. In addition to general contraction and expansion changes, more subtle changes in shape and configuration for improvement or optimization of valve function or hemodynamics may be achieved with such embodiments.

According to certain embodiments, the first zone 1602 and second zone 1604 of the ring 1600 are made from a shape memory material having a first shape memory temperature response. The third zone 1606 and fourth zone 1608 are made from a shape memory material having a second shape memory temperature response. In certain embodiments, the four zones comprise the same shape memory material, such as NiTi alloy or other shape memory material as discussed above, processed to produce the varied temperature response in the respective zones. In certain embodiments, two or more of the zones may comprise different shape memory materials. Certain embodiments include a combination of shape memory alloys and shape memory polymers in order to achieve the desired results.

According to certain embodiments, FIG. 16B shows the ring 1600 after heat activation such that it comprises expanded zones 1606', 1608' corresponding to the zones 1606, 1608 shown in FIG. 16A. As schematically shown in FIG. 16A, activation has expanded the zones 1606', 1608' so as to increase the axial lengths of the segments of the ring 1600 corresponding to those zones. In addition, or in certain embodiments, the zones 1606 and 1608 are configured to contract by a similar percentage instead of expand. In certain embodiments, the zones 1602, 1604, 1606, 1608 are configured to each have a different shape memory temperature response such that each segment corresponding to each zone 1602, 1604, 1606, 1608 could be activated sequentially.

FIG. 16B schematically illustrates that the zones 1606', 1608' have expanded axially (i.e., from their initial configuration as shown by the zones 1606, 1608 in FIG. 16A). In certain embodiments, the zones 1602, 1604 are configured to be thermally activated to remember a shape memory dimension or size upon reaching a temperature in a range between approximately 51 degrees Celsius and approximately 60 degrees Celsius. In certain such embodiments, the zones 1606 and 1608 are configured to respond at temperatures in a range between approximately 41 degrees Celsius and approximately 48 degrees Celsius. Thus, for example, by applying invasive or non-invasive energy, as discussed above, to the ring 1600 until the ring 1600 reaches a temperature of approximately 41 degrees Celsius to approximately 48 degrees Celsius, the zones 1606, 1608 will respond by expanding or contracting by virtue of the shape memory mechanism, and the zones 1602, 1604 will not.

In certain embodiments, the zones 1602, 1604 are configured to expand or contract by virtue of the shape memory mechanism at a temperature in a range between approximately 50 degrees Celsius and approximately 60 degrees Celsius. In certain such embodiments, the zones 1606, 1608 are configured to respond at a temperature in a range between approximately 39 degrees Celsius and approximately 45 degrees Celsius.

In certain embodiments, the materials, dimensions and features of the annuloplasty ring 1600 and the corresponding zones 1602, 1604, 1606, 1608 have the same or similar features, dimensions or materials as those of the other ring embodiments discussed above. In certain embodiments, the features of the annuloplasty ring 1600 are added to the embodiments discussed above.

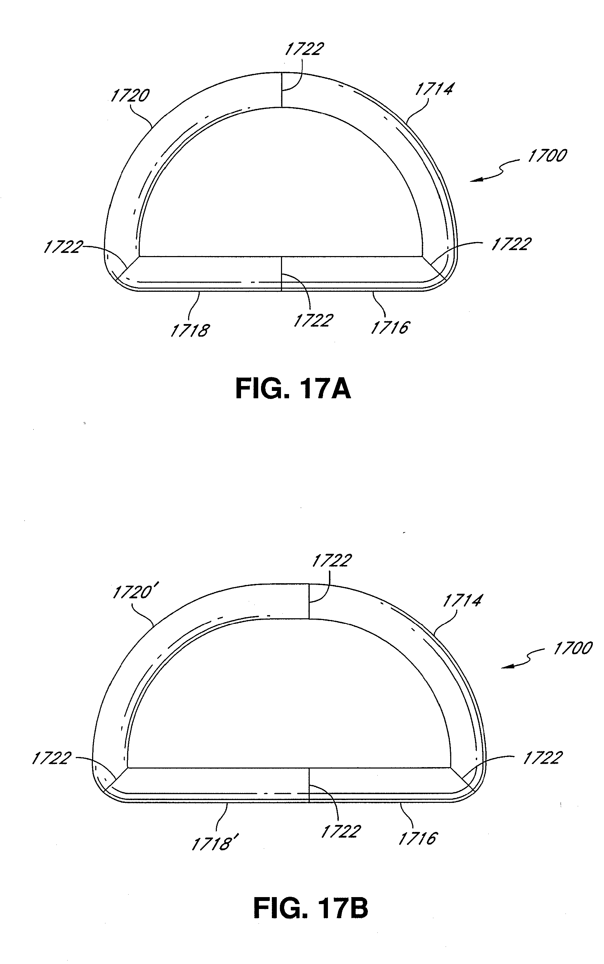

FIGS. 17A and 17B illustrate an annuloplasty ring 1700 according to certain embodiments that is similar to the annuloplasty ring 1600 discussed above, but having a "D-shaped" configuration. The ring 1700 comprises shape memory material or materials which are separated into a first temperature response zone 1714, a second temperature response zone 1716, a third temperature response zone 1718 and a fourth temperature response zone 1720. The segments defined by the zones 1714, 1716, 1718, 1720 are separated by boundaries 1722. Other than the D-shaped configuration, the ring 1700 according to certain embodiments has the same or similar features, dimensions and materials as the features, dimension and materials of the ring 1600 discussed above.

According to certain embodiments, FIG. 17B shows the ring 1700 after heat activation such that it comprises expanded zones 1718', 1720' corresponding to the zones 1718, 1720 shown in FIG. 17A. As schematically shown in FIG. 17B, activation has expanded the zones 1718', 1720' by virtue of the shape memory mechanism. The zones 1718, 1720 could also be selectively shrunk or contracted axially by virtue of the same shape memory mechanism for an embodiment having a remembered shape smaller than the nominal shape shown in FIG. 17A. The transverse cross sections of the rings 1600 and 1700 are substantially round, but can also have any other suitable transverse cross sectional configuration, such as oval, square, rectangular or the like.

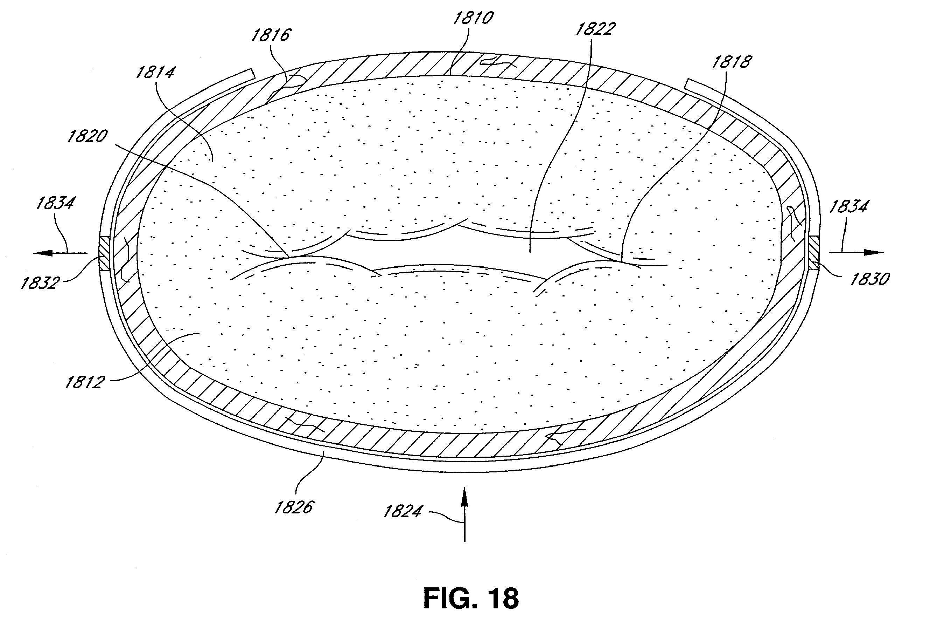

In certain situations, it is advantageous to reshape a heart valve annulus in one dimension while leaving another dimension substantially unchanged or reshaped in a different direction. For example, FIG. 18 is a sectional view of a mitral valve 1810 having an anterior (aortic) leaflet 1812, a posterior leaflet 1814 and an annulus 1816. The anterior leaflet 1812 and the posterior leaflet 1814 meet at a first commissure 1818 and a second commissure 1820. When healthy, the annulus 1816 encircles the leaflets 1812, 1814 and maintains their spacing to provide closure of a gap 1822 during left ventricular contraction. When the heart is not healthy, the leaflets 1812, 1814 do not achieve sufficient coaptation to close the gap 1822, resulting in regurgitation. In certain embodiments, the annulus 1816 is reinforced so as to push the anterior leaflet 1812 and the posterior leaflet 1814 closer together without substantially pushing the first commissure 1818 and the second commissure 1820 toward one another.

FIG. 18 schematically illustrates an exemplary annuloplasty ring 1826 comprising shape memory material configured to reinforce the annulus 1816 according to certain embodiments of the invention. For illustrative purposes, the annuloplasty ring 1826 is shown in an activated state wherein it has transformed to a memorized configuration upon application of invasive or non-invasive energy, as described herein. While the annuloplasty ring 1826 is substantially C-shaped, an artisan will recognize from the disclosure herein that other shapes are possible including, for example, a continuous circular, oval or D-shaped ring.

In certain embodiments, the annuloplasty ring 1826 comprises a first marker 1830 and a second marker 1832 that are aligned with the first commissure 1818 and the second commissure 1820, respectively, when the annuloplasty ring 1826 is implanted around the mitral valve 1810. In certain embodiments, the first marker 1830 and the second marker 1832 comprise materials that can be imaged in-vivo using standard imaging techniques. For example, in certain embodiments, the markers 1830 comprise radiopaque markers or other imaging materials, as is known in the art. Thus, the markers 1830, 1832 can be used for subsequent procedures for alignment with the annuloplasty ring 1826 and/or the commissures 1818, 1820. For example, the markers 1830, 1832 can be used to align a percutaneous energy source, such as a heated balloon inserted through a catheter, with the annuloplasty ring 1826.

When the shape memory material is activated, the annuloplasty ring 1826 contracts in the direction of the arrow 1824 to push the anterior leaflet 1812 toward the posterior leaflet 1814. Such anterior/posterior contraction improves the coaptation of the leaflets 1812, 1814 such that the gap 1824 between the leaflets 1812, 1814 sufficiently closes during left ventricular contraction. In certain embodiments, the annuloplasty ring 1826 also expands in the direction of arrows 1834. Thus, the first commissure 1818 and the second commissure 1820 are pulled away from each other, which draws the leaflets 1812, 1814 closer together and further improves their coaptation. However, in certain embodiments, the annuloplasty ring does not expand in the direction of the arrows 1834. In certain such embodiments, the distance between the lateral portions of the annuloplasty ring 1826 between the anterior portion and the posterior portion (e.g., the lateral portions approximately correspond to the locations of the markers 1830, 1832 in the embodiment shown in FIG. 18) remains substantially the same after the shape memory material is activated.

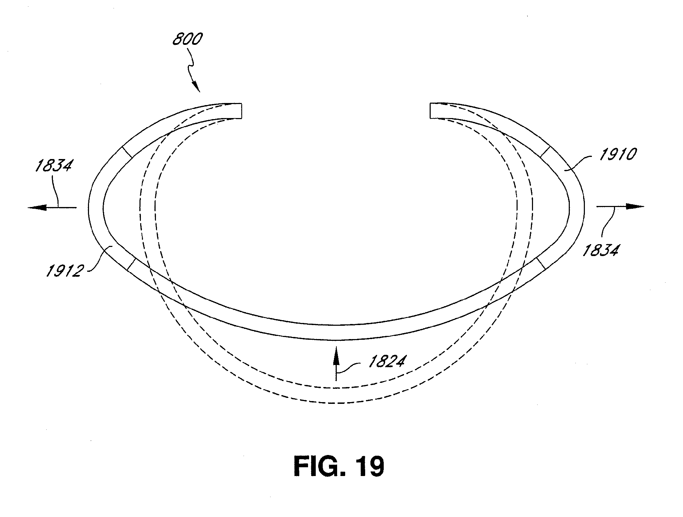

FIG. 19 is a schematic diagram of a substantially C-shaped wire comprising a shape memory material configured to contract in a first direction and expand in a second direction according to certain embodiments of the invention. Suitable shape memory materials include shape memory polymers or shape memory alloys including, for example, ferromagnetic shape memory alloys, as discussed above. FIG. 19 schematically illustrates the wire 800 in its activated configuration or memorized shape. For illustrative purposes, the wire 800 is shown relative to dashed lines representing its deformed shape or configuration when implanted into a body before activation.

When the shape memory material is activated, the wire 800 is configured to respond by contracting in a first direction as indicated by arrow 1824. In certain embodiments, the wire 800 also expands in a second direction as indicated by arrows 1834. Thus, the wire 800 is usable by the annuloplasty ring 1826 shown in FIG. 18 to improve the coaptation of the leaflets 1812, 1814 by contracting the annulus 1816 in the anterior/posterior direction. In certain embodiments, the anterior/posterior contraction is in a range between approximately 10% and approximately 20%. In certain embodiments, only a first portion 1910 and a second portion 1912 of the wire 800 comprise the shape memory material. When the shape memory material is activated, the first portion 1910 and the second portion 1912 of the wire 800 are configured to respond by transforming to their memorized configurations and reshaping the wire 800 as shown.

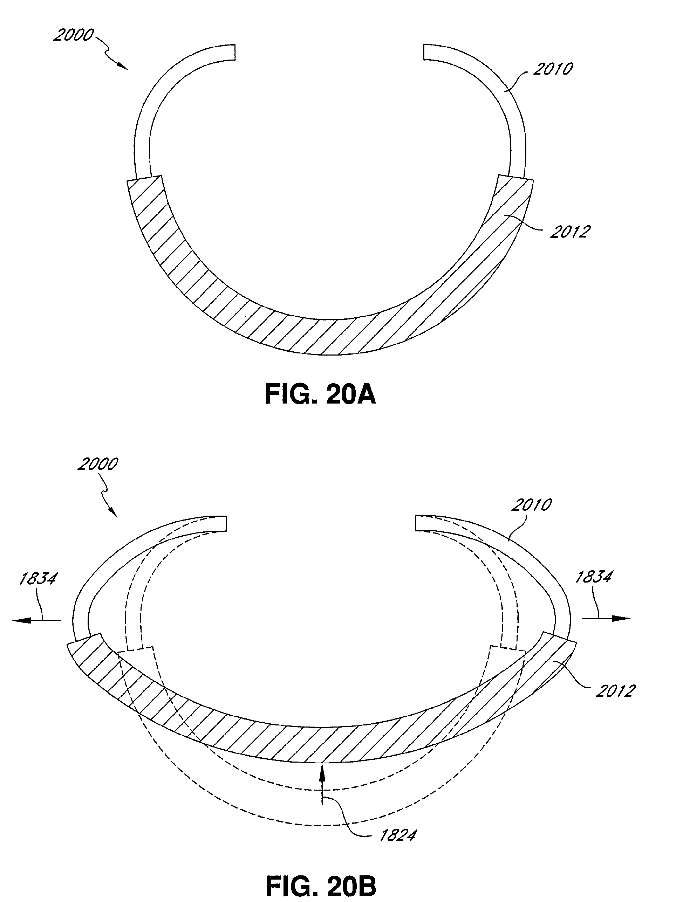

FIGS. 20A and 20B are schematic diagrams of a body member 2000 according to certain embodiments usable by an annuloplasty ring, such as the annuloplasty ring 1826 shown in FIG. 18. Although not shown, in certain embodiments, the body member 2000 is covered by a flexible material such as silicone rubber and a suturable material such as woven polyester cloth, Dacron@, woven velour, polyurethane, polytetrafluoroethylene (PTFE), heparin-coated fabric, or other biocompatible material, as discussed above.

The body member 2000 comprises a wire 2010 and a shape memory tube 2012. As used herein, the terms "tube," "tubular member" and "tubular structure" are broad terms having at least their ordinary and customary meaning and include, for example, hollow elongated structures that may in cross-section be cylindrical, elliptical, polygonal, or any other shape. Further, the hollow portion of the elongated structure may be filled with one or more materials that may be the same as and/or different than the material of the elongated structure. In certain embodiments, the wire 2010 comprises a metal or metal alloy such as stainless steel, titanium, platinum, combinations of the foregoing, or the like. In certain embodiments, the shape memory tube 2012 comprises shape memory materials formed in a tubular structure through which the wire 2010 is inserted. In certain embodiments, the shape memory tube 2012 comprises a shape memory material coated over the wire 2010. Suitable shape memory materials include shape memory polymers or shape memory alloys including, for example, ferromagnetic shape memory alloys, as discussed above. Although not shown, in certain embodiments, the body member 2000 comprises an energy absorption enhancement material, as discussed above.

FIG. 20A schematically illustrates the body member 2000 in a first configuration or shape and FIG. 20B schematically illustrates the body member 2000 in a second configuration or shape after the shape memory tube has been activated. For illustrative purposes, dashed lines in FIG. 20B also show the first configuration of the body member 2000. When the shape memory material is activated, the shape memory tube 2012 is configured to respond by contracting in a first direction as indicated by arrow 1824. In certain embodiments, the shape memory tube 2012 is also configured to expand in a second direction as indicated by arrows 1834. The transformation of the shape memory tube 2012 exerts a force on the wire 2010 so as to change its shape. Thus, the body member 2000 is usable by the annuloplasty ring 1826 shown in FIG. 18 to pull the commissures 1818, 1820 further apart and push the leaflets 1812, 1814 closer together to improve coaptation.

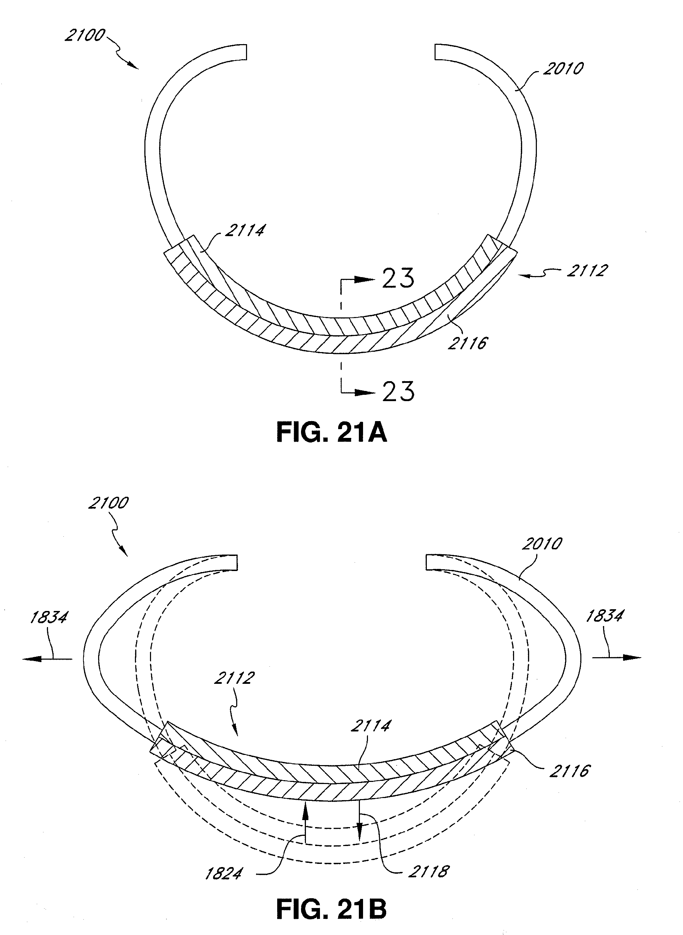

FIGS. 21A and 21B are schematic diagrams of a body member 2100 according to certain embodiments usable by an annuloplasty ring, such as the annuloplasty ring 1826 shown in FIG. 18. Although not shown, in certain embodiments, the body member 2100 is covered by a flexible material such as silicone rubber and a suturable material such as woven polyester cloth, Dacron@, woven velour, polyurethane, polytetrafluoroethylene (PTFE), heparin-coated fabric, or other biocompatible material, as discussed above.

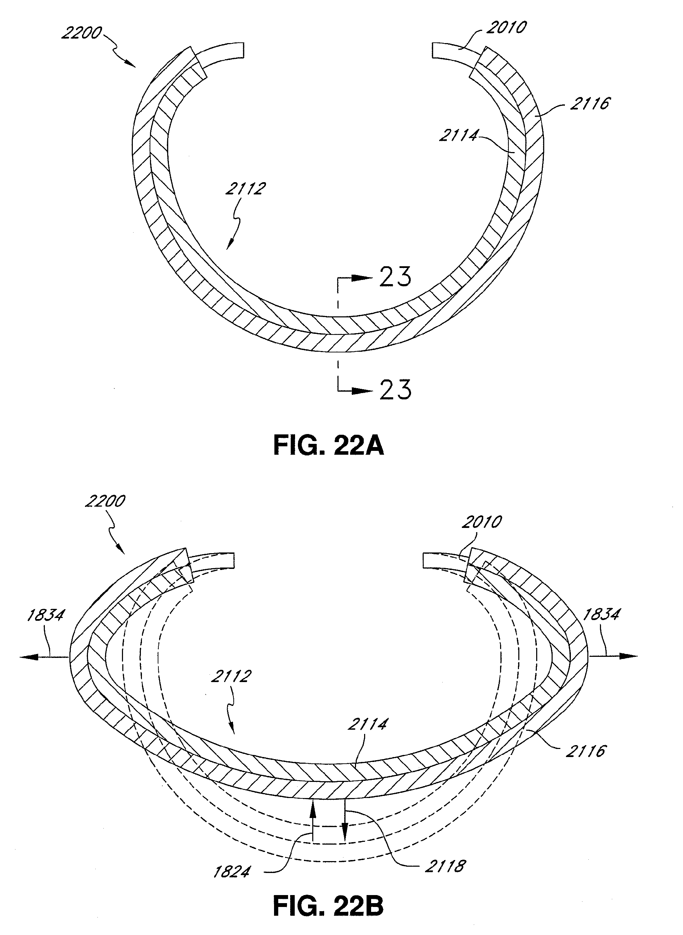

The body member 2100 comprises a wire 2010, such as the wire 2010 shown in FIGS. 20A and 20B and a shape memory tube 2112. As schematically illustrated in FIGS. 21A and 21B, the shape memory tube 2112 is sized and configured to cover a certain percentage of the wire 2010. However, an artisan will recognize from the disclosure herein that in certain embodiments the shape memory tube 2112 may cover other percentages of the wire 2010. Indeed, FIGS. 22A and 22B schematically illustrate another embodiment of a body member 2200 comprising a shape memory tube 2112 covering a substantial portion of a wire 2010. The amount of coverage depends on such factors as the particular application, the desired shape change, the shape memory materials used, the amount of force to be exerted by the shape memory tube 2112 when changing shape, combinations of the foregoing, and the like. For example, in certain embodiments where, as in FIGS. 22A and 22B, the shape memory tube 2112 covers a substantial portion of a wire 2010, portions of the shape memory tube 2112 are selectively heated to reshape the wire 2010 at a particular location. In certain such embodiments, HIFU energy is directed towards, for example, the left side of the shape memory tube 2112, the right side of the shape memory tube 2112, the bottom side of the shape memory tube 2112, or a combination of the foregoing to activate only a portion of the shape memory tube 2112. Thus, the body member 2200 can be reshaped one or more portions at a time to allow selective adjustments.

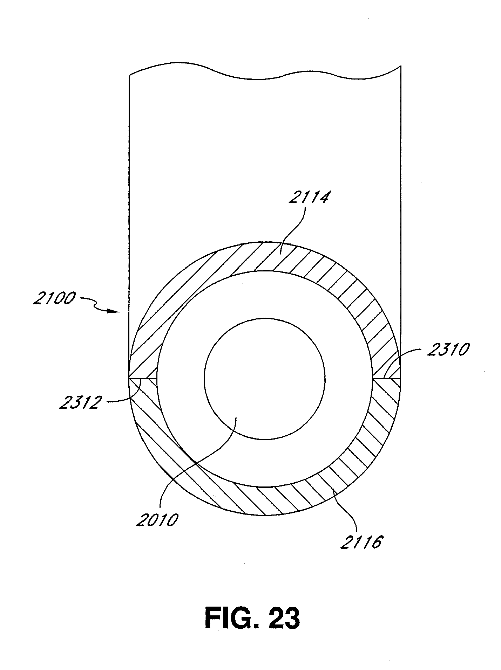

In certain embodiments, the shape memory tube 2112 comprises a first shape memory material 2114 and a second shape memory material 2116 formed in a tubular structure through which the wire 2010 is inserted. In certain such embodiments, the first shape memory material 2114 and the second shape memory material 2116 are each configured as a semi-circular portion of the tubular structure. For example, FIG. 23 is a transverse cross-sectional view of the body member 2100. As schematically illustrated in FIG. 23, the first shape memory material 2114 and the second shape memory material 2116 are joined at a first boundary 2310 and a second boundary 2312. In certain embodiments, silicone tubing (not shown) holds the first shape memory material 2114 and the second shape memory material 2116 together. In certain embodiments, the first shape memory material 2114 and the second shape memory material 2116 each comprise a shape memory coating covering opposite sides of the wire 2010. Suitable shape memory materials include shape memory polymers or shape memory alloys including, for example, ferromagnetic shape memory alloys, as discussed above. Although not shown, in certain embodiments the body member 2100 comprises an energy absorption enhancement material, as discussed above.

FIG. 21A schematically illustrates the body member 2100 in a first configuration or shape before the first shape memory material 2114 and the second shape memory material 2116 are activated. In certain embodiments, the first shape memory material 2114 and the second shape memory material 2116 are configured to be activated or return to their respective memorized shapes at different temperatures. Thus, the first shape memory material 2114 and the second shape memory material 2116 can be activated at different times to selectively expand and/or contract the body member 2100. For example, in certain embodiments, the second shape memory material 2116 is configured to be activated at a lower temperature than the first shape memory material 2114.

FIG. 21B schematically illustrates the body member 2100 in a second configuration or shape after the second shape memory material 2116 has been activated. For illustrative purposes, dashed lines in FIG. 21B also show the first configuration of the body member 2100. When the second shape memory material 2116 is activated, it responds by bending the body member 2100 in a first direction as indicated by arrow 1824. In certain embodiments, activation also expands the body member 2100 in a second direction as indicated by arrows 1834. Thus, the body member 2100 is usable by the annuloplasty ring 1826 shown in FIG. 18 to pull the commissures 1818, 1820 further apart and push the leaflets 1812, 1814 closer together to improve coaptation.

In certain embodiments, the first shape memory material 2114 can then be activated to bend the body member 2100 opposite to the first direction as indicated by arrow 2118. In certain such embodiments, the body member 2100 is reshaped to the first configuration as shown in FIG. 21A (or the dashed lines in FIG. 21B). Thus, for example, if the size of the patient's heart begins to grow again (e.g., due to age or illness), the body member 2100 can be enlarged to accommodate the growth. In certain embodiments, activation of the first shape memory material 2114 further contracts the body member 2100 in the direction of the arrow 1824. In certain embodiments, the first shape memory material 2114 has an austenite start temperature Aₛ in a range between approximately 42 degrees Celsius and approximately 50 degrees Celsius and the second shape memory material 2116 has an austenite start temperature Aₛ in a range between approximately 38 degrees Celsius and 41 degrees Celsius.

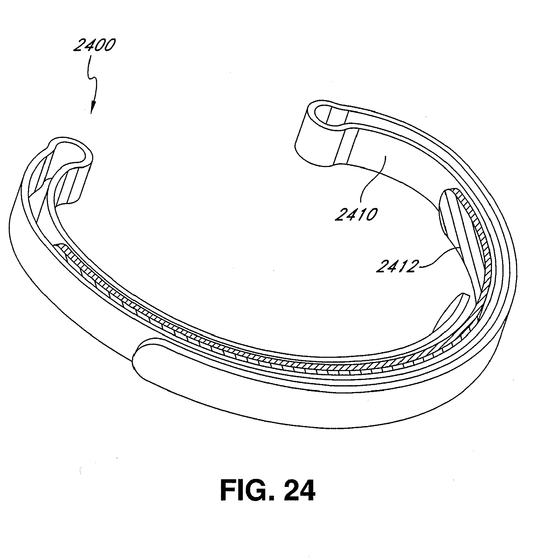

FIG. 24 is a perspective view of a body member 2400 usable by an annuloplasty ring according to certain embodiments comprising a first shape memory band 2410 and a second shape memory band 2412. Suitable shape memory materials for the bands 2410, 2412 include shape memory polymers or shape memory alloys including, for example, ferromagnetic shape memory alloys, as discussed above. Although not shown, in certain embodiments the body member 2100 comprises an energy absorption enhancement material, as discussed above. Although not shown, in certain embodiments, the body member 2100 is covered by a flexible material such as silicone rubber and a suturable material such as woven polyester cloth, Dacron@, woven velour, polyurethane, polytetrafluoroethylene (PTFE), heparin-coated fabric, or other biocompatible material, as discussed above.

The first shape memory band 2410 is configured to loop back on itself to form a substantially C-shaped configuration. However, an artisan will recognize from the disclosure herein that the first shape memory band 2410 can be configured to loop back on itself in other configurations including, for example, circular, D-shaped, or other curvilinear configurations. When activated, the first shape memory band 2410 expands or contracts such that overlapping portions of the band 2410 slide with respect to one another, changing the overall shape of the body member 2400. The second shape memory band 2412 is disposed along a surface of the first shape memory band 2410 such that the second shape memory band 2412 is physically deformed when the first shape memory band 2410 is activated, and the first shape memory band 2410 is physically deformed when the second shape memory band 2412 is activated.

As shown in FIG. 24, in certain embodiments at least a portion of the second shape memory band 2412 is disposed between overlapping portions of the first shape memory band 2410. An artisan will recognize from the disclosure herein, however, that the second shape memory band 2412 may be disposed adjacent to an outer surface or an inner surface of the first shape memory band 2410 rather than between overlapping portions of the first shape memory band 2410. When the second shape memory band 2412 is activated, it expands or contracts so as to slide with respect to the first shape memory band 2410. In certain embodiments, the first shape memory band 2410 and the second shape memory band 2412 are held in relative position to one another by the flexible material and/or suturable material discussed above.

While the first shape memory band 2410 and the second shape memory band 2412 shown in FIG. 24 are substantially flat, an artisan will recognize from the disclosure herein that other shapes are possible including, for example, rod-shaped wire. However, in certain embodiments the first shape memory band 2410 and the second shape memory band 2412 advantageously comprise substantially flat surfaces configured to guide one another during expansion and/or contraction. Thus, the surface area of overlapping portions of the first shape memory band 2410 and/or the second shape memory band 2412 guide the movement of the body member 2400 in a single plane and reduce misalignment (e.g., twisting or moving in a vertical plane) during shape changes. The surface area of overlapping portions also advantageously increases support to a heart valve by reducing misalignment during beating of the heart.

An artisan will recognize from the disclosure herein that certain embodiments of the body member 2400 may not comprise either the first shape memory band 2410 or the second shape memory band 2412. For example, in certain embodiments the body member 2400 does not include the second shape memory band 2412 and is configured to expand and/or contract by only activating the first shape memory band 2410. Further, an artisan will recognize from the disclosure herein that either the first band 2410 or the second band 2412 may not comprise a shape memory material. For example, the first band 2410 may titanium, platinum, stainless steel, combinations of the foregoing, or the like and may be used with or without the second band 2412 to support a coronary valve annulus.

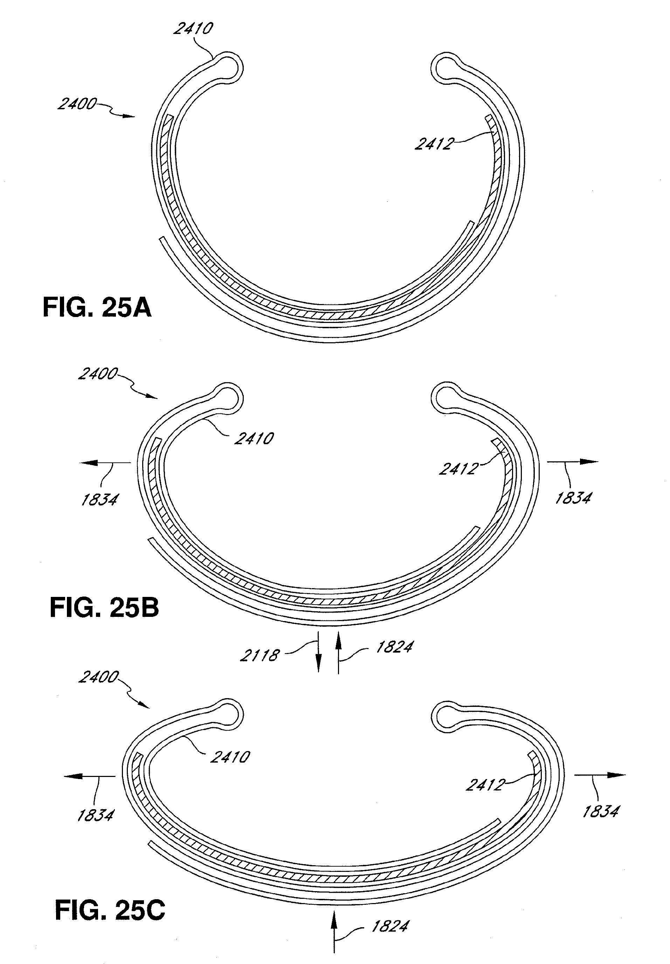

As schematically illustrated in FIGS. 25A-25C, in certain embodiments the body member 2400 is configured to change shape at least twice by activating both the first shape memory band 2410 and the second shape memory band 2412. FIG. 25A schematically illustrates the body member 2400 in a first configuration or shape before the first shape memory band 2410 or the second shape memory band 2412 are activated. In certain embodiments, the first shape memory band 2410 and the second shape memory band 2412 are configured to be activated or return to their respective memorized shapes at different temperatures. Thus, the first shape memory band 2410 and the second shape memory band 2412 can be activated at different times to selectively expand and/or contract the body member 2400. For example (and for purposes of discussing FIGS. 25A-25C), in certain embodiments, the first shape memory band 2410 is configured to be activated at a lower temperature than the second shape memory band 2412. However, an artisan will recognize from the disclosure herein that in certain embodiments the second shape memory band 2412 may be configured to be activated at a lower temperature than the first shape memory band 2410.

FIG. 25B schematically illustrates the body member 2400 in a second configuration or shape after the first shape memory band 2410 has been activated. When the first shape memory band 2410 is activated, it responds by bending the body member 2400 in a first direction as indicated by arrow 1824. In certain embodiments, the activation also expands the body member 2400 in a second direction as indicated by arrows 1834. Thus, the body member 2400 is usable by the annuloplasty ring 1826 shown in FIG. 18 to pull the commissures 1818, 1820 further apart and push the leaflets 1812, 1814 closer together to improve coaptation.

In certain embodiments, the second shape memory band 2412 can then be activated to further contract the body member 2400 in the direction of the arrow 1824 and, in certain embodiments, further expand the body member 2400 in the direction of arrows 1834. In certain such embodiments, activating the second shape memory band 2412 reshapes the body member 2400 to a third configuration as shown in FIG. 25C. Thus, for example, as the patient's heart progressively heals and reduces in size, the body member 2400 can be re-sized to provide continued support and improved leaflet coaptation. In certain embodiments, activation of the second shape memory band 2412 bends the body member 2400 opposite to the first direction as indicated by arrow 2118. In certain such embodiments, activating the second shape memory band 2412 reshapes the body member 2400 to the first configuration as shown in FIG. 25A. Thus, for example, if the size of the patient's heart begins to grow again (e.g., due to age or illness), the body member 2400 can be re-sized to accommodate the growth.

In certain annuloplasty ring embodiments, flexible materials and/or suturable materials used to cover shape memory materials also thermally insulate the shape memory materials so as to increase the time required to activate the shape memory materials through application of thermal energy. Thus, surrounding tissue is exposed to the thermal energy for longer periods of time, which may result in damage to the surrounding tissue. Therefore, in certain embodiments of the invention, thermally conductive materials are configured to penetrate the flexible materials and/or suturable materials so as to deliver thermal energy to the shape memory materials such that the time required to activate the shape memory materials is decreased.

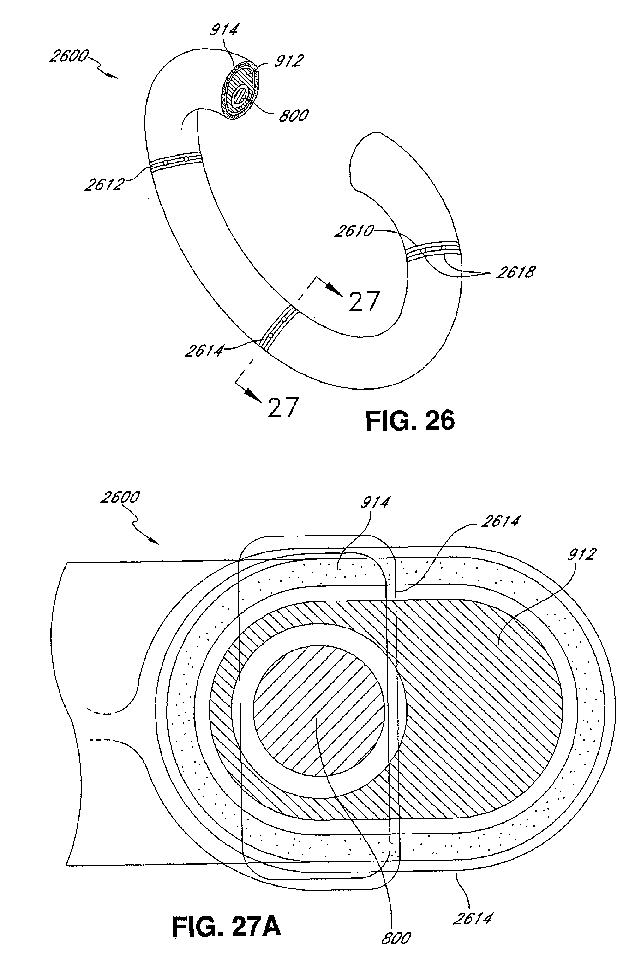

For example, FIG. 26 is a perspective view illustrating an annuloplasty ring 2600 comprising one or more thermal conductors 2610, 2612, 2614 according to certain embodiments of the invention. The annuloplasty ring 2600 further comprises a shape memory wire 800 covered by a flexible material 912 and a suturable material 914, such as the wire 800, the flexible material 912 and the suturable material 914 shown in FIG. 9A. As shown in FIG. 26, in certain embodiments, the shape memory wire 800 is offset from the center of the flexible material 912 to allow more room for sutures to pass through the flexible material 912 and suturable material 914 to attach the annuloplasty ring 2600 to a cardiac valve. In certain embodiments, the flexible material 912 and/or the suturable material 914 are thermally insulative. In certain such embodiments, the flexible material 912 comprises a thermally insulative material. Although the annuloplasty ring 2600 is shown in FIG. 26 as substantially C-shaped, an artisan will recognize from the disclosure herein that the one or more thermal conductors 2610, 2612, 2614 can also be used with other configurations including, for example, circular, D-shaped, or other curvilinear configurations.

In certain embodiments, the thermal conductors 2610, 2612, 2614 comprise a thin (e.g., having a thickness in a range between approximately 0.002 inches and approximately 0.015 inches) wire wrapped around the outside of the suturable material 914 and penetrating the suturable material 914 and the flexible material 912 at one or more locations 2618 so as to transfer externally applied heat energy to the shape memory wire 800. For example, FIGS. 27A-27C are transverse cross-sectional views of the annuloplasty ring 2600 schematically illustrating exemplary embodiments for conducting thermal energy to the shape memory wire 800. In the exemplary embodiment shown in FIG. 27A, the thermal conductor 2614 wraps around the suturable material 914 one or more times, penetrates the suturable material 914 and the flexible material 912, passes around the shape memory wire 800, and exits the flexible material 912 and the suturable material 914. In certain embodiments, the thermal conductor 2614 physically contacts the shape memory wire 800. However, in certain embodiments, the thermal conductor 2614 does not physically contact the shape memory wire 800 but passes sufficiently close to the shape memory wire 800 so as to decrease the time required to activate the shape memory wire 800. Thus, the potential for thermal damage to surrounding tissue is reduced.

In the exemplary embodiment shown in FIG. 27B, the thermal conductor 2614 wraps around the suturable material 914 one or more times, penetrates the suturable material 914 and the flexible material 912, passes around the shape memory wire 800 two or more times, and exits the flexible material 912 and the suturable material 914. By passing around the shape memory wire 800 two or more times, the thermal conductor 2614 concentrates more energy in the area of the shape memory wire 800 as compared to the exemplary embodiment shown in FIG. 27A. Again, the thermal conductor 2614 may or may not physically contact the shape memory wire 800.

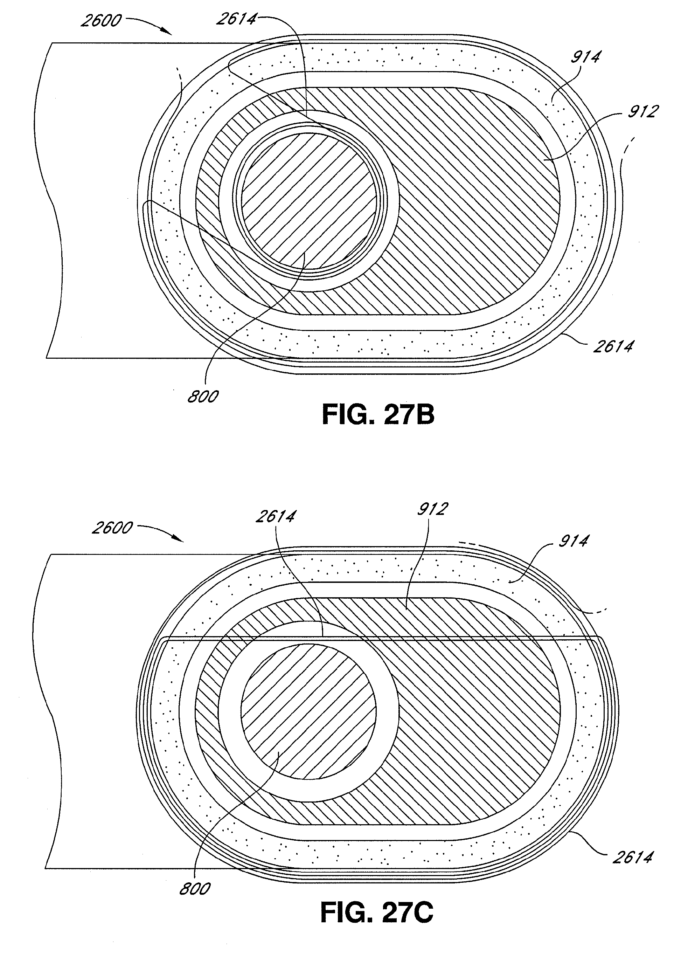

In the exemplary embodiment shown in FIG. 27C, the thermal conductor 2614 wraps around the suturable material 914 one or more times and passes through the suturable material 914 and the flexible material 912 two or more times. Thus, portions of the thermal conductor 2614 are disposed proximate the shape memory wire 800 so as to transfer heat energy thereto. Again, the thermal conductor 2614 may or may not physically contact the shape memory wire 800. An artisan will recognize from the disclosure herein that one or more of the exemplary embodiments shown in FIGS. 27A-27C can be combined and that the thermal conductor 2614 can be configured to penetrate the suturable material 914 and the flexible material 912 in other ways so as to transfer heat to the shape memory wire 800.

Referring again to FIG. 26, in certain embodiments the locations of the thermal conductors 2610, 2612, 2614 are selected based at least in part on areas where energy will be applied to activate the shape memory wire 800. For example, in certain embodiments heat energy is applied percutaneously through a balloon catheter and the thermal conductors 2610, 2612, 2614 are disposed on the surface of the suturable material 914 in locations likely to make contact with the inflated balloon.

In addition, or in certain embodiments, the thermal conductors 2610, 2612, 2614 are located so as to mark desired positions on the annuloplasty ring 2600. For example, the thermal conductors 2610, 2612, 2614 may be disposed at locations on the annuloplasty ring 2600 corresponding to commissures of heart valve leaflets, as discussed above with respect to FIG. 18. As another example, the thermal conductors 2610, 2612, 2614 can be used to align a percutaneous energy source, such as a heated balloon inserted through a catheter, with the annuloplasty ring 2600. In certain such embodiments the thermal conductors 2610, 2612, 2614 comprise radiopaque materials such as gold, copper or other imaging materials, as is known in the art.

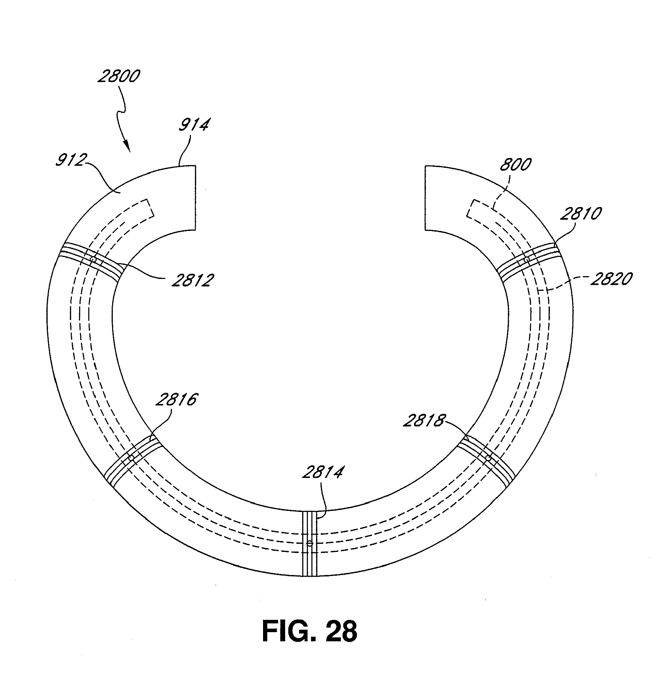

FIG. 28 is a schematic diagram of an annuloplasty ring 2800 according to certain embodiments of the invention comprising one or more thermal conductors 2810, 2812, 2814, 2816, 2818, such as the thermal conductors 2610, 2612, 2614 shown in FIG. 26. As schematically illustrated in FIG. 28, the annuloplasty ring 2800 further comprises a shape memory wire 800 covered by a flexible material 912 and a suturable material 914, such as the wire 800, the flexible material 912 and the suturable material 914 shown in FIG. 9A.

In certain embodiments, the shape memory wire 800 is not sufficiently thermally conductive so as to quickly transfer heat applied in the areas of the thermal conductors 2810, 2812, 2814, 2816, 2818. Thus, in certain such embodiments, the annuloplasty ring 2800 comprises a thermal conductor 2820 that runs along the length of the shape memory wire 800 so as to transfer heat to points of the shape memory wire 800 extending beyond or between the thermal conductors 2810, 2812, 2814, 2816, 2818. In certain embodiments, each of the thermal conductors 2810, 2812, 2814, 2816, 2818, comprise a separate thermally conductive wire configured to transfer heat to the thermal conductive wire 2820. However, in certain embodiments, at least two of the thermal conductors 2810, 2812, 2814, 2816, 2818 and the thermal conductor 2820 comprise one continuous thermally conductive wire.

Thus, thermal energy can be quickly transferred to the annuloplasty ring 2600 or the annuloplasty ring 2800 to reduce the amount of energy required to activate the shape memory wire 800 and to reduce thermal damage to the patient's surrounding tissue.

### Adjustment Device

Mitral valve repair bands or annuloplasty rings are implanted and sized based on the intertrigonal distance at the base of the anterior leaflet of the valve. The posterior circumference of the remodeling ring or band is generally elliptical in shape and proportional to the intertrigonal width. Often, mitral annular dilation resulting from ischemic cardiomyopathy or secondary to valve leakage and left ventricular (LV) volume overload primarily involves increases in the posterior circumference of the mitral annulus. The shape and posterior annular circumference is remodeled by a repair device to permit complete occlusion of the mitral valvular orifice by primarily the anterior leaflet of the mitral valve during systole.

Determining the optimal posterior annular circumference depends on many factors, including: (1) length and shape of the anterior mitral leaflet; (2) height and mobility of the posterior mitral leaflet complex; (3) intertrigonal distance "size" of the anterior leaflet; (4) choice of remodeling mitral annular ring or incomplete annular band; (5) relative flexibility of the mitral annuloplasty device; and (6) degree of downsizing necessary to produce functional valve orifice.

For a given mitral anterior leaflet with a specific intertrigonal distance "size," there is a range of optimal posterior mitral annular circumferences that will comprise a functional valvular complex. The choice of size for a remodeling mitral annular device is made through the left atrium, usually with the heart in a flaccid, diastolic arrested state. In this state, the anterior leaflet measurements are made using proprietary annuloplasty ring sizers, comparing the posterior circumference with the anticipated valve closure line on the sizer. Selection of the proper posterior mitral annular circumference is a function of surgeon experience and is very accurate in most (80-90%) cases. In the remainder, however, both visual inspection of the valve function via the open atrium or dynamic intraoperative evaluation of mitral valve function with transesophageal echocardiography discloses mild to moderate valve leakage. One of the readily treatable causes of repair leakage is incomplete coaptation of the anterior leaflet with the posterior leaflet complex. One simple method of improving anterior leaflet coaptation is to further downsize the posterior circumference of the remodeled annulus so as to improve the anterior-posterior "fit" of the leaflets within the device. Certain methods of *in situ* reshaping and downsizing the posterior mitral ring dimensions is outlined below. The timing of this valve annuloplasty adjustment can be made at any point during implant time or in post-implant time, from immediately in the operating room to up to six months or more.

Certain embodiments of these methods use one or more of the adjustment devices disclosed herein, which are useful in the intraoperative adjustment of a size and/or dimension of certain adjustable devices, for example, embodiments of an externally adjustable annuloplasty ring described above.

Certain embodiments of the adjustment devices disclosed herein may be guided to an adjustable device using magnets. For example, in certain embodiments, the adjustment device may include a magnet at or near a distal end of the adjustment device for directing or otherwise facilitating the guidance of the adjustment device to an adjustable device which includes a material magnetic to a magnetic field.

Also provided are methods and systems for adjusting a size and/or dimension of certain embodiments of adjustable devices described above. The following disclosure is made with reference to an adjustable annuloplasty ring as the adjustable device; however, those skilled in the art will understand that the disclosed device is also useful in adjusting other types of adjustable devices known in the art. Embodiments of the device are configured to deliver an amount of activation energy, such as radio frequency (RF) energy, effective for adjusting a size and/or dimension of at least a portion of the adjustable device. Those skilled in the art will understand that certain embodiments are configured to deliver other forms of activation energy, for example, microwave energy, magnetic energy, electric energy, thermal energy, ultrasonic energy, light energy, and the like. Certain embodiments deliver a combination of energy types. In certain embodiments, the adjustment device can be configured to reduce the temperature of an adjustable device by producing coldness at its distal end (i.e., the adjustment device can be configured to conduct energy away from its distal end). For example, in certain embodiments, the adjustment device may produce a cold fluid at the distal end.

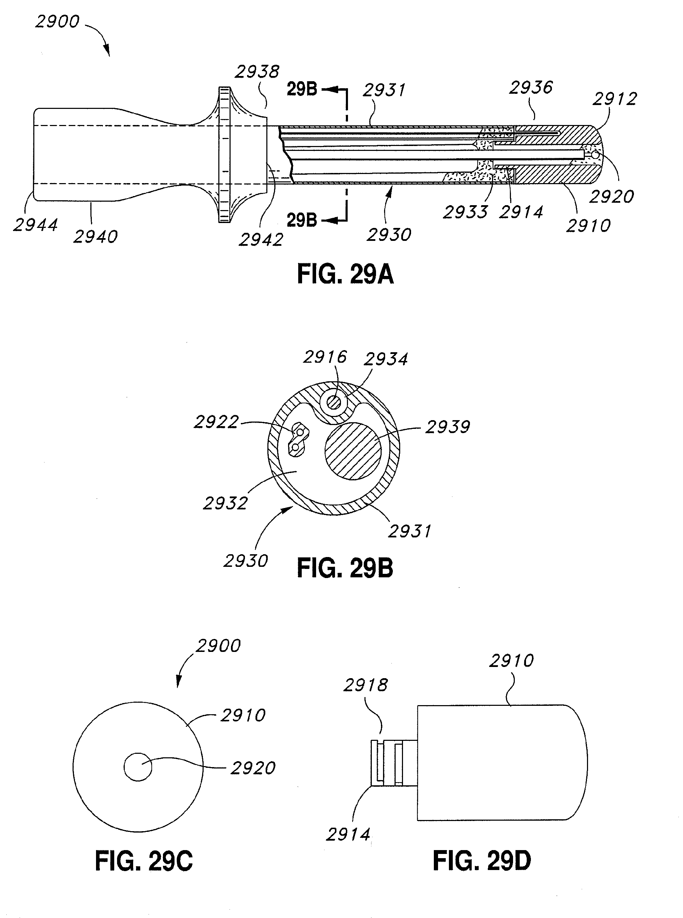

FIG. 29A illustrates a longitudinal cross section of an embodiment of an intraoperative adjustment device 2900 comprising a tip electrode 2910, a thermocouple 2920 mounted therein, a body 2930 to which the tip electrode 2910 is mounted, and a handle 2940 to which the body 2930 is mounted.

The tip electrode 2910 is fabricated from any suitable material known in the art, for example, platinum, platinum-iridium, stainless steel, nitinol, combinations, alloys, and/or mixtures thereof, and the like. In certain embodiments, the tip electrode 2910 comprises a biocompatible material. In certain embodiments, a coating or layer is disposed on at least a portion of tip electrode. In certain embodiments, the coating comprises a polymer, for example, polytetrafluoroethylene (Teflon®, Dupont). In the illustrated embodiment, the tip electrode 2910 is generally cylindrical, comprising a rounded first end 2912, and a second end terminating in a shank 2914. In certain embodiments, the tip electrode 2910 is from about 1 mm (0.04") to about 5 mm (0.2"). In certain embodiments, the tip electrode 2910 is from about 2 mm to about 4 mm. In certain embodiments, the tip electrode 2910 can have other shapes.

The thermocouple 2920 is mounted proximal to the first end 2912 of the tip electrode 2910 using any means known in the art, for example, adhesively. Suitable adhesives are known in the art, for example, epoxy, polyurethane, silicone, and the like. In an end view of the intraoperative adjustment device 2900 illustrated in FIG. 29C, the thermocouple 2920 is substantially concentric with the tip electrode 2910. In certain embodiments, the thermocouple 2920 is not concentric. The thermocouple 2920 is of any type known in the art that is useful for determining a temperature of the first end 2912 of the tip electrode. The use of the thermocouple is discussed in greater detail below. Certain embodiments of the intraoperative adjustment device 2900 do not comprise a thermocouple.

Some features of the construction of the body 2930 are best viewed in section A-A, illustrated in FIG. 29B. In the illustrated embodiment, the body 2930 is an elongate structure comprising a dual lumen tube 2931, which comprises a first lumen 2932 and a second lumen 2934. In the illustrated embodiment, the first and second lumens 2932 and 2934 have different dimensions, which in the illustrated embodiment include different diameters. In certain embodiments, the first and second lumens 2932 and 2934 have substantially similar dimensions. In the illustrated embodiment, the first and second lumens 2932 and 2934 serve to electrically, fluidly, and/or mechanically isolate components disposed in the lumens, as discussed in greater detail below. In certain embodiments, the body 2930 comprises a single lumen. In certain embodiments, the body 2930 comprises more than two lumens.

Electrical leads 2922 for the thermocouple 2920 extend through the first lumen 2932 from the first end 2936 to the second end 2938 of the body. In certain embodiments, the lead 2922 can be a RB ethibond lead. Also disposed in the first lumen 2932 is a malleable element 2939, which permits the body 2930 to be manually malleable, deformable, and/or bendable, thereby permitting a user to vary the direction of the tip electrode 2910 as desired. The malleable element 2939 comprises any suitable material known in the art, for example a metal wire and/or rod. Suitable metals for the malleable wire are known in the art, and include pure metals, as well as alloys, composites, and the like, for example, malleable steel, copper, and the like. In embodiments with a malleable body 2930, components extending within the body 2930, for example, the two-lumen tube 2931, the thermocouple leads 2922, and an electrode lead wire 2916 are independently flexible, bendable, and/or malleable. In certain embodiments, the tube 2931 comprises a malleable material and does not comprise a separate malleable element 2939. In certain embodiments, the body 2930 is not substantially malleable.

An electrode lead wire 2916 in electrical connection with the tip electrode 2910 extends through the second lumen 2934 from the first end 2936 to the second end 2938 of the body. The electrode lead wire 2916 comprises any suitable material known in the art, for example, a metal wire. In certain embodiments, the electrode lead wire 2916 is a copper wire. In certain embodiments, an electrically insulating layer, such as a polyimide layer, is disposed on at least a portion of the electrode lead wire 2916.

In certain embodiments, the body is from about 5 cm (2") to about 20 cm (8") long. In the illustrated embodiment, the diameters of the body 2930 and the tip electrode 2910 are substantially similar. In certain embodiments, the diameters of the body 2930 and the tip electrode 2910 are different. The body 2930 comprises a first end 2936 proximal to the tip electrode 2910 and a second end 2938 proximal to the handle 2940. In the illustrated embodiment, the tip electrode 2910 is substantially permanently mounted to the body 2930 through the shank 2914. In certain embodiments, the tip electrode 2910 is detachably mounted to the body 2930. In the illustrated embodiment, the tip electrode 2910 is secured to the first end of the body 2936 using securing means 2933 known in the art, for example, adhesively. Suitable adhesives are known in the art, for example, epoxy, polyurethane, silicone, and the like. Certain embodiments use other securing means known in the art, for example, threading, clamping, swaging, pinning, lock rings, clips, and the like. Combinations of securing means are also used in certain embodiments.

As best illustrated in FIG. 29D, which is a side view of the tip electrode 2910, in certain embodiments, the shank 2914 of the tip electrode also comprises a means 2918 for securing the tip electrode 2910 to the body 2930. In the illustrated embodiment, the means 2918 comprises a pair of grooves provided on the shank 2914, which work in concert with the securing means 2918. Those skilled in the art will understand that other means known in the art are also useful for the means 2918, for example, ridges, barbs, hooks, threads, combinations thereof, and the like. In certain embodiments in which the tip electrode 2910 is secured the body 2930 mechanically, the shank 2914 comprises means 2918 known in the art that cooperate with the securing means 2933.

The handle 2940 and/or means for manipulation comprises a first end 2942 and a second end 2944, and is dimensioned and configured to permit a user to grasp the device 2900 and to position the tip electrode 2910 as desired. In certain embodiments, the length of the handle 2940 is from about 8 cm (3") to about 16 cm (6"), and the diameter is from about 13 mm (0.5") to about 25 mm (1"). The second end 2938 of the body is mounted to the first end 2942 of the handle. In certain embodiments, the body 2930 is detachably mounted to the handle 2940 using any means known in the art, for example, a bayonet mount, a screw mount, pins, clips, or the like. In certain embodiments, the body 2930 is substantially permanently mounted to the handle 2940. Electrical connections (not illustrated) are provided, for example, at the second end 2944 of the handle for the thermocouple leads 2922 and the electrode lead wire 2916. The handle 2940 comprises any suitable material known in the art, for example, polymers, metals, inorganic materials, and alloys, blends, or composites thereof. In certain embodiments, the handle 2940 comprises a suitable polymer, for example, acrylonitrile-butadiene-styrene (ABS), polyvinylchloride (PVC), polyamide (Nylon®, Delrin®), polypropylene, polyethylene, styrene-butadiene, polyether block amide (PEBAX®), blends thereof, and the like. In certain embodiments, a non-slip coating is provided. In certain embodiments, the handle 2940 is equipped with a immobilization means, for example, a clamping system, a threaded portion, a mounting system, or the like, which permits a user to maintain the intraoperative adjustment device 2900 in a desired position. In certain embodiments, the handle 2940 comprises means (not illustrated) for controlling the intraoperative adjustment device 2900, for example, a switch controlling power to the tip electrode 2910. In certain embodiments, the handle 2940 comprises one or more indicators of the status or state of the intraoperative adjustment device 2900, for example, a power, power level, and/or warning(s) (not illustrated). In certain embodiments, one or more of the indicators is provided on a different component, for example, on the body 2030 and/or the tip electrode 2010.

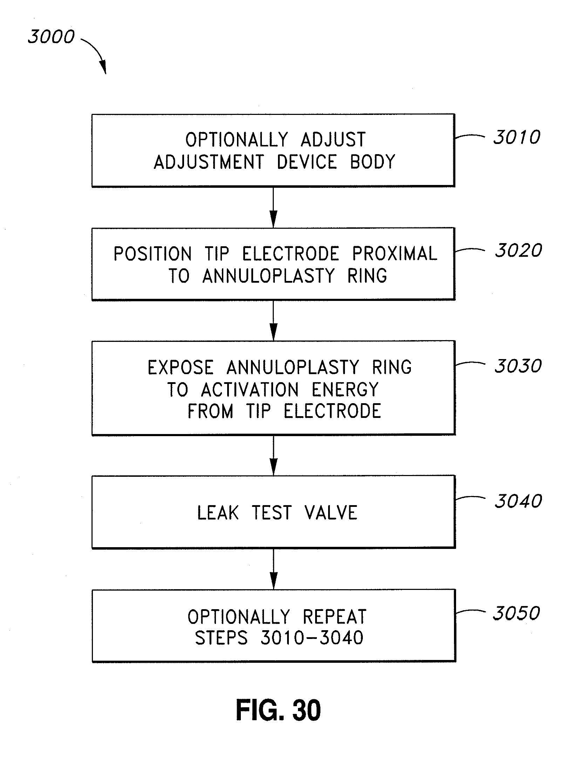

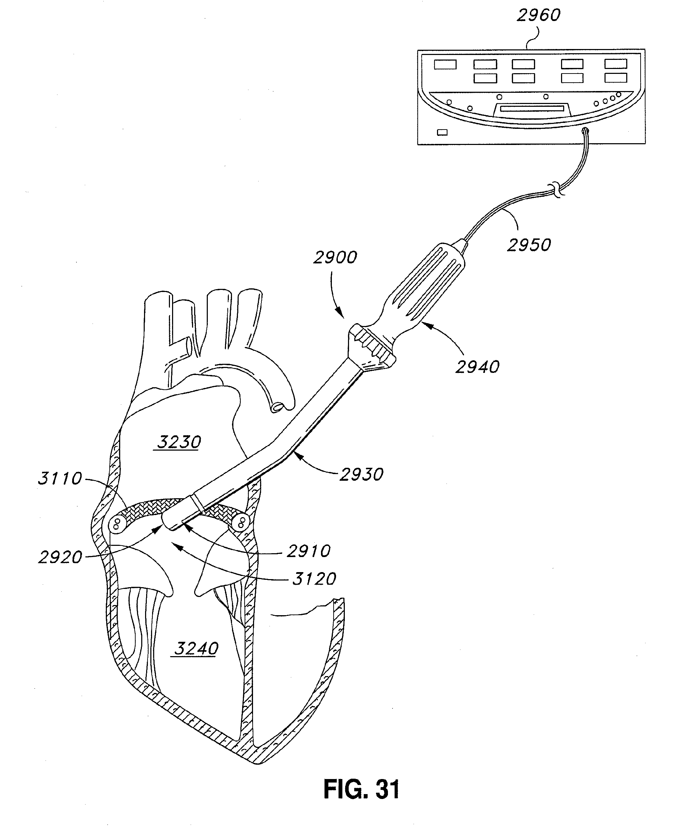

FIG. 30 is flowchart illustrating a method for intraoperatively adjusting an annuloplasty ring with reference to FIG. 31, which illustrates the intraoperative adjustment device 2900 illustrated in FIGS. 29A-29D in use. In certain embodiments, the adjustment procedure is performed substantially contemporaneously with the annuloplasty procedure. Accordingly, the heart is bypassed and the patient is maintained using a heart-lung machine (on pump) during the adjustment procedure.

Briefly, the composition and characteristics of the valve annuloplasty device, discussed above, permits adjustment of both its shape and dimensions after implant by exploiting its physiochemical nature. Once sutured *in situ* to valve annular tissue, the device is downsized by applying an activation energy source to effect a net change in the device's dimensions. A variety of changes in length and shape of the annuloplasty device can be made, depending on the location of the built-in adjustability along the perimeter of the device. The two most commonly sites of adjustment of the mitral annular device is the region of the ring/band that is sutured in the region of the P2 segment of the posterior leaflet complex with equivalent overlap with the medial halves of the P1 and P3 regions. This region is the location of major posterior mitral annular remodeling that is necessary for most mitral valve repairs and is also the region of greatest expansive force that is applied to the mitral annulus during systole. Shortening of the ring/band length in this region reduces the anterior-posterior dimension of the mitral annulus by 5-10% in certain embodiments. A net reduction of this dimension without removing the annuloplasty device is highly desirable for the reasons described above, for example, uncertainty of optimal mitral anterior-posterior (AP) dimension depending on the "size" of the intertrigonal distance as a function of the AP length of the anterior leaflet. Therefore, under direct vision or with transesophageal echocardiography, the anterior-posterior dimension can be downsized to improve mitral valve function. The accuracy of the downsizing maneuver can be immediately assessed and further changes made depending on the result. Thus, the application of energy to the mitral annular remodeling device to effect a "shrinkage" of the posterior dimensions can be titrated to achieve a gradual decrease in the dimensions until the mitral closing performance is suitable (zero detectable leakage).

Further modifications of the posterior mitral annular shape can be made in a similar manner. This is particularly applicable to mitral regurgitation of ischemic etiology in which there is asymmetric dilation of the posterior annulus adjacent to the P2 and half of P3 leaflet segment. Selective regional downsizing of the remodeling annuloplasty device in this area can improve the dynamic function of the mitral valve after the muscle is revascularized and an operating load is placed on the left ventricle to create the working stress that will be placed on the annulus and annuloplasty device.

The use of the disclosed adjustment system for an implanted mitral repair ring/band permits precise changes to be made by open suture of the remodeling ring with subsequent "titration" of the fit by external means. The ability to downsize an implanted ring increases the safety factor for mitral valve repair by allowing a surgeon to implant a ring or band that would not cause initial systolic anterior motion (SAM), but could be ratcheted down in size if the effective orifice were too large for adequate anterior leaflet function.

The above-described devices and techniques are also useful for tricuspid valve remodeling rings/bands. The appeal and applicability of downsizing after implanting is especially suitable for a tricuspid device because the tricuspid valve function is more load-dependent than the mitral valve and therefore is dependent on adequate left ventricle/mitral valve function as well as pulmonary mechanics for its good performance. The tricuspid valve ring is also more amenable to external energy application because of its "exposed" anterior location in the chest. Accordingly, certain embodiments of magnetic energy application are easier for this device.

**FIG.** 31 illustrates an adjustable annuloplasty ring 3110 sutured above the tricuspid valve 3120 to reduce the effective size of the valve annulus. The following description is made with reference to the tricuspid valve 3120, which separates the right atrium 3230 from the right ventricle 3240. Those skilled in the art will understand that the devices, methods, and systems described herein are also applicable in the treatment of other valves, for example, the mitral valve.

As discussed above, the illustrated embodiment of the adjustable annuloplasty ring 3110 comprises a suturable material disposed over at least a portion of an adjustable ring. At least one dimension and/or size of the adjustable ring is adjustable using RF energy, thereby adjusting the annuloplasty ring. In certain embodiments, at least one dimension and/or size of the annuloplasty ring is adjustable using a different type of activation energy, as discussed above. In certain embodiments, the annuloplasty ring 3110 is adjustable to reduce a diameter, thereby further reducing the effective size of the cardiac valve annulus. After the annuloplasty ring 3110 is implanted, for example, by suturing, the valve 3120 is leak-tested. The valve 3120 may be leak tested according to many techniques, including ultrasound, such as transesophageal echocardiography (TEE) (with or without Doppler), or by irrigation with saline. If leakage is detected, the annuloplasty ring is adjusted as described below. Without the use of the device as discussed herein, if leakage were detected, the annuloplasty ring 3110 would be removed and replaced with a new one.

As discussed above, the intraoperative adjustment device 2900 comprises a tip electrode 2910, a thermocouple 2920, a body 2930, and a handle 2940. Wires 2950 electrically connect the thermocouple lead wires 2922 and the electrode lead wire 2916 to a control unit 2960. The control unit 2960 is configured to determine a temperature from the output of the thermocouple 2920. In the illustrated embodiment, the control unit also comprises an RF generator configured to provide RF power to the tip electrode 2910 through one or more cables and/or conduits 2950 and the electrode lead wire 2916, thereby generating an RF output at the tip electrode. In certain embodiments, the control unit 2960 can provide other types of activation energy. In certain embodiments, the control unit 2960 comprises a microprocessor and/or microcomputer, which permits automated and/or partially automated operation, as discussed in greater detail below.

Returning to FIG. 30, in optional step 3010, the body 2930 of the intraoperative adjustment device 2900 is adjusted to facilitate the adjustment of the annuloplasty ring 3110. As illustrated in FIG. 31, the body 2930 of the intraoperative adjustment device is manually deformed and/or bent to permit the desired positioning of the tip electrode 2910 relative to the annuloplasty ring 3110.

In step 3020, the tip electrode 2910 is positioned proximal to a portion of the annuloplasty ring 3110 at which adjustment is desired. In certain embodiments, the tip electrode 2910 is substantially stationary relative to the annuloplasty ring 3110. In certain embodiments, the tip electrode 2910 is in motion relative to the annuloplasty ring 3110, for example, to adjust an extended portion of the annuloplasty ring 3110. In certain embodiments, the annuloplasty ring 3110 comprises markings and/or indicia indicating predetermined positions for the tip electrode 2910, and consequently, for adjustment.

In step 3030, the annuloplasty ring 3110 is exposed to an amount of activation energy, in this case RF energy. from the tip electrode 2910 effective for adjusting at least a portion of the annuloplasty ring 3110. As discussed above, activating an RF generator in the control unit 2960 causes an RF output from the tip electrode 2910. The RF energy is absorbed by at least a portion of the annuloplasty ring, thereby causing a shape, size, and/or dimensional change therein, as discussed in greater detail below. In certain embodiments, markings and/or indicia are provided on the annuloplasty ring, which permit the user to visualize the degree of adjustment, for example, parallel lines, an array of dots, combinations, and the like.

Optionally, the temperature of the thermocouple 2920 is also determined. High temperatures will cause undesired blood coagulation. Accordingly, in certain embodiments, the control unit 2960 modifies the RF output, either reducing and/or terminating the output, when the thermocouple detects a temperature at or below which coagulation occurs. In certain embodiments, the intraoperative adjustment device 2900 is run in a constant temperature mode in which the RF power is adjusted to provide a substantially constant temperature at the thermocouple 2920. In certain embodiments, the intraoperative adjustment device 2900 is run in a constant power mode, in which the RF power is substantially constant. Certain embodiments of these modes are automated. In certain embodiments, the intraoperative adjustment device 2900 is run in another mode, for example, a user-controlled mode.

In step 3040, the valve 3120 is again leak-tested, for example, using ultrasound, such as transesophageal echocardiography (TEE), to determine if the adjustment in step 3030 was effective. If the valve 3120 still leaks, one or more of steps 3010-3040 are repeated in optional step 3050 to further adjust the annuloplasty ring 3110. Further adjustments are performed at the substantially the same location of the annuloplasty ring 3110 or at one or more different locations. In certain embodiments, the adjustment is repeated until the leakage is substantially eliminated. Accordingly, in certain embodiments, the adjustment of the annuloplasty ring 3110 comprises a plurality of adjustment steps. In certain embodiments, the adjustment is performed using a single adjustment step.

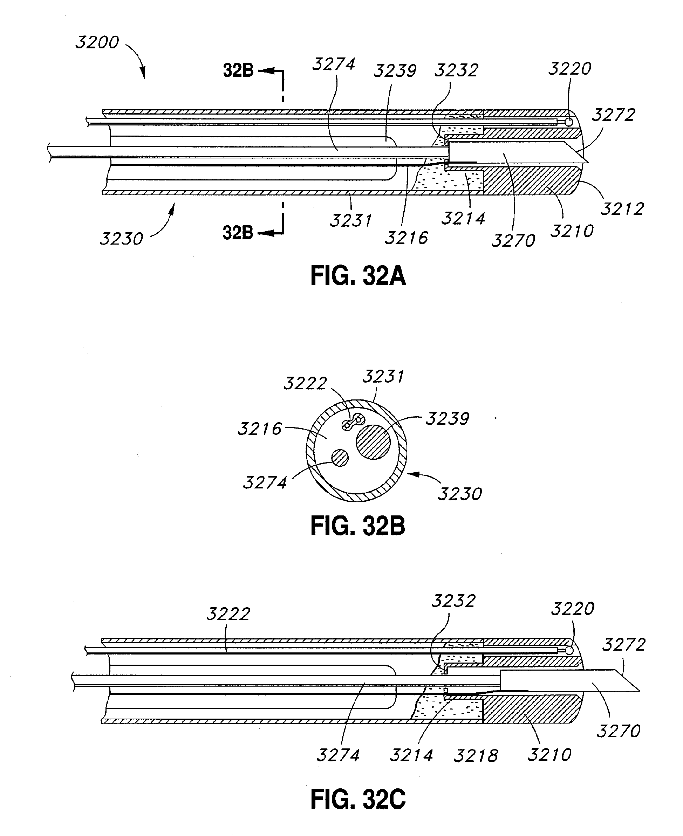

FIG. 32A illustrates a cross-section of an intraoperative adjustment device 3200 which is similar to the device illustrated in FIG. 29A. Because of the similarities to the device 2900, only the electrode 3210, thermocouple 3220, and body 3230 are illustrated in **FIG.** 32A. The tip electrode 3210 comprises a first end 3212 and a second end comprising a shank 3214. The tip electrode 3210 is generally cylindrical with a generally convex first end 3212. Extending from the first end 3212 to the second end 3214 is a passageway 3218. In the illustrated embodiment, the passageway 3218 is substantially straight. The thermocouple 3220 is disposed in the tip electrode 3210 with leads 3222 extending longitudinally through the tip electrode 3210. In certain embodiments, the adjustment device 3200 comprises a stop mechanism 3232 which inhibits the extendable probe 3270 from retracting beyond a certain point within the adjustment device 3200. For example, in the illustrated embodiment 3200, the stop mechanism is a rear wall 3232.

The construction at the body 3230 is best viewed in section 32B and illustrated in FIG. 32B. In the illustrated embodiment, the body 3230 comprises a single lumen tube 3231, enclosing a lead wire 3216, thermocouple leads 3222, and a malleable element 3239.

Returning to FIG. 32A the intraoperative adjustment device 3200 further comprises an extendable probe 3270 comprising a puncture tip 3272 at a first end and a push rod 3274 at the second end. The extendable probe 3270 has at least a first, retracted position, and a second, extended position. In the illustrated embodiment, the push rod 3274 extends through the lumen of the body 3230, and is operable by the user to extend and/or retract the extendable probe 3270 between at least the first and second positions. In FIG. 32A, the extendable probe 3270 is retractable into the passageway 3218 of the tip electrode 3210 in the first position. The lead wire 3216 is in an electrical connection with the extendable probe 3270, thereby providing power to the extendable probe 3270.

FIG. 32C illustrates in cross-section the electrode tip 3210 with the extendable probe 3270 in the second, extended position in which the push rod 3274 is used to extend the probe 3270 from the opening 3218.

The extendable probe 3270 is dimensioned and configured to puncture or otherwise penetrate a suture material on an annuloplasty ring as described herein, thereby permitting the focused application of RF energy on a desired portion of the annuloplasty ring. In the illustrated embodiment, the lead wire 3216 transmits RF power from an RF generator (not illustrated) to the extendable probe.

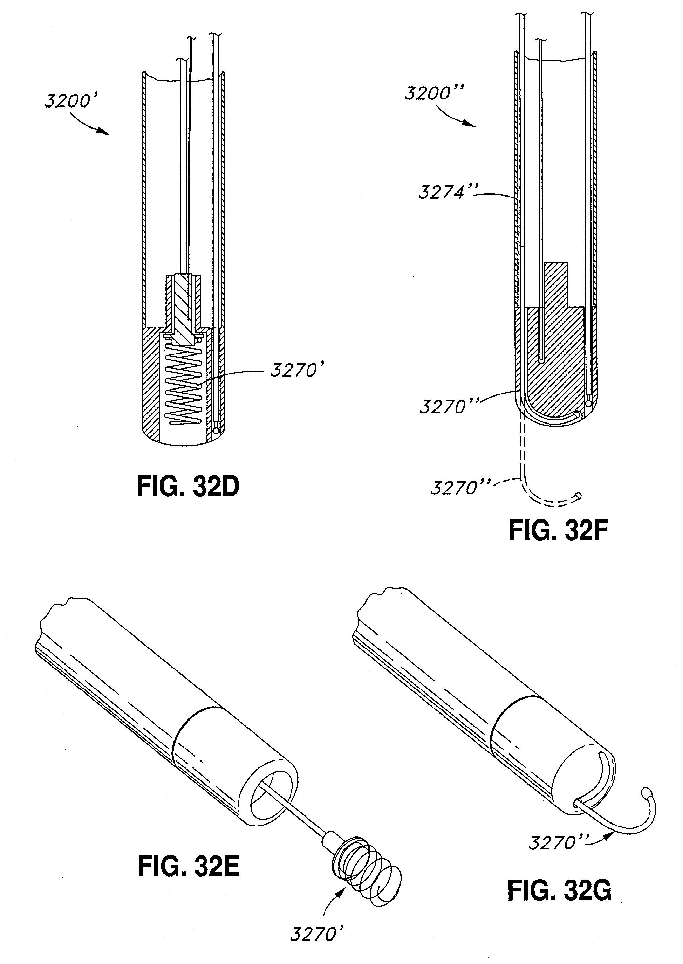

FIG. 32D illustrates in cross-section another embodiment of the intraoperative adjustment device 3200' comprising a generally helical and/or spiral extendable probe 3270'. In FIG. 32D, the extendable probe 3270' is in the retracted position. FIG. 32E illustrates in perspective the helical extendable probe 3270' in the extended position. In certain embodiments, the helical probe 3270' can penetrate an outer surface of an implantable annuloplasty ring via a corkscrew pattern.

FIG. 32F illustrates in cross-section another embodiment of the intraoperative adjustment device 3200" comprising a generally hook-shaped extendable probe 3270" in the retracted position in solid and in the extended position in phantom. In the illustrated embodiment, the extendable probe 3270" is integral with the push rod 3274", and are offset from the longitudinal axis of the device 3200", that is, not concentric. FIG. 32G illustrates in perspective the hook-shaped extendable probe 3270" in the extended position. Certain embodiments of the hook-shaped extendable probe 3270" permit the activation of a small, user selectable portion of the annuloplasty ring.

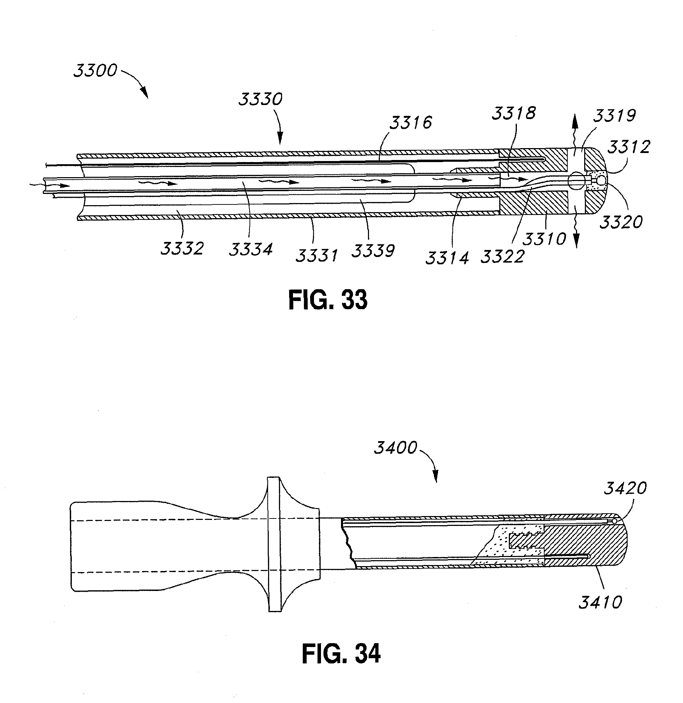

FIG. 33 illustrates another embodiment of an intraoperative adjustment device 3300. Because this embodiment is substantially similar to the embodiment illustrated in FIG. 29A, FIG. 33 illustrates only the tip electrode 3310, thermocouple 3320, and body 3330. In the illustrated embodiment, the tip electrode comprises a first end 3312 and a second end comprising a shank 3314, and a passageway 3318 extending from the second end of the tip electrode into the body of the tip electrode 3310. In the illustrated embodiment, the passageway 3318 is generally concentric with the tip electrode 3310. A plurality of conduits 3319 extend radially from the opening 3318 to the outer surface of the tip electrode 3310.

In the illustrated embodiment, the thermocouple 3320 is mounted substantially concentrically with the tip electrode 3310, and the thermocouple leads 3322 extend through the passageway 3318.

The body 3330 of the intraoperative adjustment device 3300 comprises a dual lumen tube 3331, comprising an outer lumen 3332 and an inner lumen 3334, which in the illustrated embodiment, is substantially concentric, and is fluidly connected with the passageway 3318 in the tip electrode 3210. Extending through the outer lumen 3332 is a lead wire 3316 and a malleable element 3339.

The embodiment of the intraoperative device 3300 illustrated in FIG. 33 permits the application of a fluid through the inner lumen 3334 through the passageway 3318 and at the conduits 3319. For example, in certain embodiments the fluid is a saline solution which is useful in detecting leakage in the valve for example in step 3040 of the adjustment method 3000. In certain embodiments, applying an aqueous composition to the annuloplasty ring during the activation in step 3030 of adjustment method 3000 improves the energy transfer to the annuloplasty ring.

FIG. 34 illustrates an embodiment of an intraoperative adjustment device 3400 which is similar to the embodiment illustrated in FIG. 29A except that the thermocouple 3420 is offset from the longitudinal axis of the tip electrode 3410 rather than concentric.

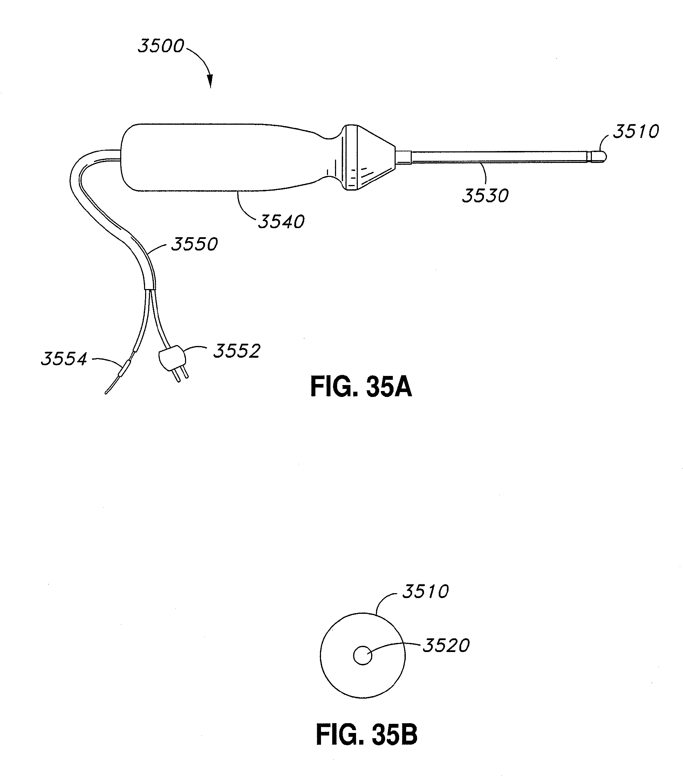

FIG. 35A illustrates a side view of another embodiment of an intraoperative adjustment device 3500 comprising a tip electrode 3510, a thermocouple 3520 (also illustrated from a front view in FIG. 35B), a body 3530, a handle 3540, and a cable 3550. In the illustrated embodiment, the tip electrode 3510 is secured to the body 3530 adhesively, for example, using an epoxy. The body 3530 in the illustrated embodiment is stainless steel tubing and is substantially not malleable. The cable composes a bundle of wires and/or conductors, terminating, for example, in a plug 3552 in electrical contact with the thermocouple 3520 and a single wire plug 3554, which is in electrical contact with the tip electrode 3510.

As discussed above, in certain embodiments, the intraoperative adjustment device generates another type of energy suitable for adjusting the annuloplasty ring, for example, microwave energy, magnetic energy, electric energy, thermal energy, ultrasonic energy, light energy, and the like. Combinations, either with or without RF energy are also suitable. Suitable means for generating the desired type of energy are known in the art. For example, in certain embodiments, microwave energy is generated in one or more of the intraoperative adjustment devices described herein by applying microwave power to the electrode tip. In certain embodiments, thermal energy is generated using a resistive heater in the tip of the device. An infrared (IR) laser is another method for providing thermal energy. For example, certain embodiments comprise a solid state IR laser on the intraoperative adjustment device itself, for example, on or near the tip. In certain embodiments, the intraoperative adjustment device comprises a waveguide and/or optical fiber suitable for carrying IR laser energy, and an IR laser source separate from the device itself. Certain embodiments also comprise a visible light source, for example, a solid state laser, that indicates the focus and/or power status of the IR laser. In certain embodiments, ultrasonic energy is provided using an ultrasonic transducer known in the art, disposed, for example, at the device tip. Magnetic energy is generated in certain embodiments using one or more coils, for example, in the tip of the device.

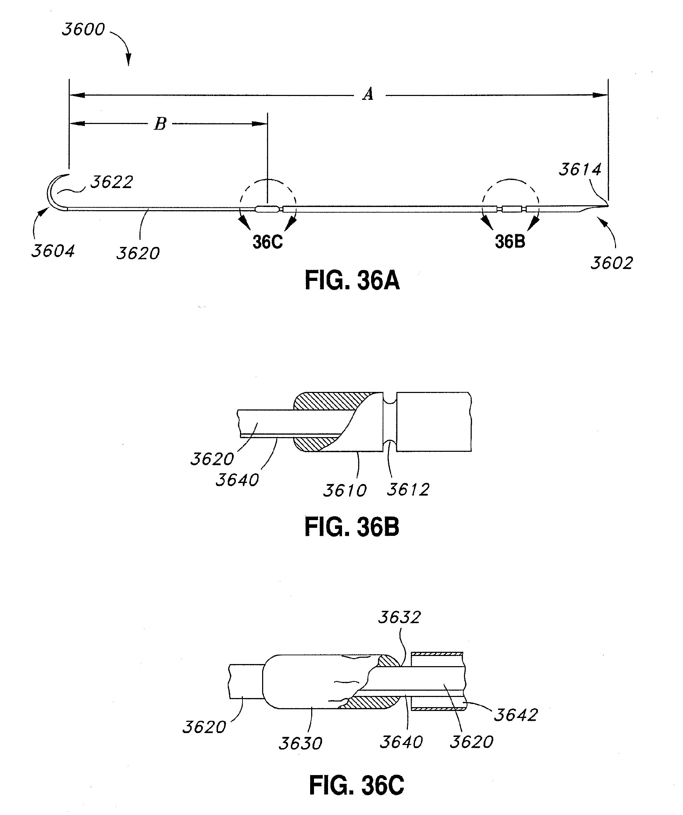

FIG. 36A illustrates a side view of an embodiment of an implantable adjustment device 3600 useful for adjusting an adjustable device, for example, an adjustable annuloplasty ring, as disclosed herein. The illustrated device activates an adjustable device using RF energy. Those skilled in the art will understand that certain embodiments use other types of energy and/or combinations of energy types, for example, resistive heating and the like.

The electrode 3600 comprises a proximal end 3602 and a distal end 3604. The proximal end 3602 terminates in a proximal electrode 3610, which in the illustrated embodiment, comprises a puncture tip 3614. In certain embodiments, the proximal electrode 3610 does not comprise a puncture tip, as will become apparent below. The puncture tip in the illustrated embodiment is formed on a needle of any type known in the art. In the illustrated embodiment, the needle is about 0.025" (about 0.6 mm) O.D., although those skilled in the art will understand that other sizes are useful in certain embodiments. The proximal electrode 3610 comprises any suitable material known in the art, for example, stainless steel.

In certain embodiments, both the proximal end 3602 and the distal end 3604 each comprise a plurality of electrodes. In certain embodiments, each of the plurality of electrodes is connected to a corresponding suture and suture needle. For example, in certain embodiments, the proximal end 3602 and the distal end 3604 each comprise two electrodes, a positive electrode and a negative electrode, along with two sutures connected to two suture needles. In certain embodiments, the proximal end 3602 and the distal end 3604 each comprise a positive electrode, a negative electrode, and a ground electrode.

A suture 3620 extends from the distal end 3604 to the proximal electrode 3610, and is secured thereto. The suture 3604 is of any suitable type known in the art, for example, polyester (Dacron®), silk, polyamide (Nylon®), or polypropylene (Prolene®), monofilament or braided. In the illustrated embodiment, the suture 3620 is 2-0 braided polyester, although those skilled in the art will understand that other materials and sizes are useful in certain embodiments. The illustrated embodiment comprises an integrated suture needle 3622 secured at the distal end of the suture 3620.

In the illustrated embodiment, the suture 3620 is secured to the proximal electrode 3610 by crimping in an opening formed in the distal end of the proximal electrode 3610, as best seen in FIG. 36B, which is a partial cross section of the connection between the proximal electrode 3610 and suture 3620. Those skilled in the art will understand that other securing means are used in certain embodiments. Also illustrated in FIG. 36B is a groove 3612 formed in the needle 3610, which is used for detaching the puncture tip 3614 of the needle as discussed in greater detail below.

Returning to FIG. 36A, the overall length of the adjustment electrode 3600 is indicated by dimension A, which in the illustrate embodiment, is about 30" (about 76 cm). Disposed on the suture 3620 is a distal electrode 3630 at a distance *B* from the distal end 3604. In the illustrated embodiment, B is about 4" (about 10 cm), although those skilled in the art will understand that other sizes are useful in certain embodiments. The electrode 3630 can be manufactured using at least any of the materials described above with reference to FIG. 29.

As best seen in the embodiment illustrated in FIG. 36C, which is an enlarged, partial cut-away view of the apparatus 3600 around the distal electrode 3630, the distal electrode 3630 is generally cylindrical with an opening 3632 coaxial with the longitudinal axis and passing through the distal electrode 3630. The opening 3632 is dimensioned and configured to accept the suture 3620 therethrough. The distal electrode 3630 is secured to the suture 3620 by any suitable means known in the art, for example, by crimping. The distal electrode 3630 comprises any suitable material, for example, stainless steel, platinum, nitinol, combinations, and the like. In the illustrated embodiment, the diameter of the distal electrode 3630 is about 0.025" (about 0.6 mm) O.D., although those skilled in the art will understand that other sizes are useful in certain embodiments.

In certain embodiments, the distal electrode 3630 comprises a shape memory material, and is dimensioned and configured to permit positioning as described below, but to prevent removal prior to activation with an RF power source.

As illustrated in FIGS. 36B and 36C, an electrically conductive power wire 3640 extends between and operatively couples the proximal electrode 3610 and the distal electrode 3630. The power wire 3640 comprises any suitable material, for example, copper. In the illustrated embodiment, the power wire 3640 comprises a 38 gauge copper wire. In the illustrated embodiment, the power wire 3640 is secured to each of the proximal 3610 and distal 3630 electrodes by crimping, for example, concomitantly with the securing of the suture 3620, as illustrated in FIGS. 36B and 36C.

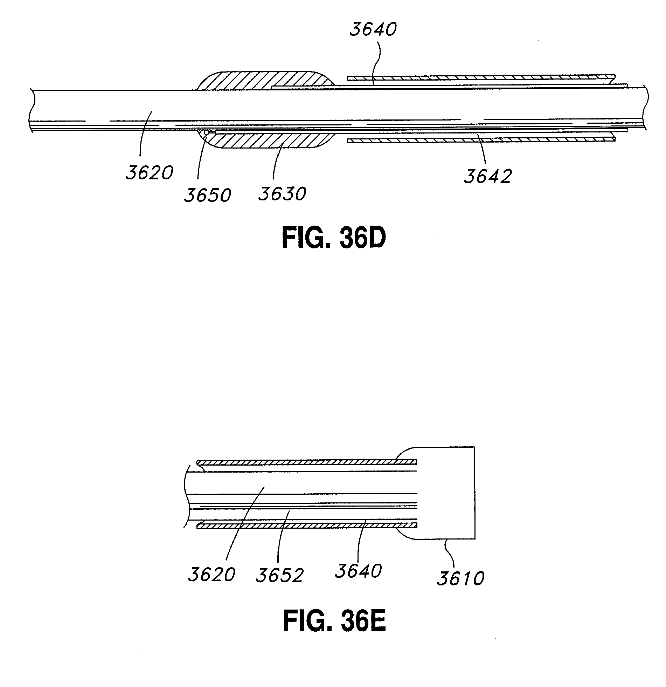

As illustrated in FIG. 36D, which is cross-sectional detail of the distal electrode 3630, and FIG. 36E, which is a cross-sectional view of a portion of the proximal electrode 3610, the illustrated embodiment also comprises a thermocouple 3650 disposed at the distal electrode 3630 and associated thermocouple wires 3652. In the illustrated embodiment, the thermocouple 3650 and thermocouple wires 3652 are secured by crimping, for example, concomitantly with the securing of the suture 3620 and/or power wire 3640.

Disposed over the power wire 3640, thermocouple wires 3652, and suture 3620 is a flexible sheath 3642 extending generally between the proximal electrode 3610 and the distal electrode 3630. In the illustrated embodiment, the sheath 3642 electrically insulates the power wire 3640, as well as protecting the power wire 3640 and thermocouple wires 3652 from physical damage. In certain embodiments, the sheath comprises a single lumen tube. In certain embodiments, the lumen is sized and dimensioned to prevent the sheath from passing over at least one of the proximal electrode 3610 or distal electrode 3630. The sheath 3642 is of any suitable biocompatible material, for example, one or more polymers. In the illustrated embodiment, the sheath comprises polyether polyamide (PEBAX®) tubing about 0.020" × 0.026" (about 0.5 mm× 0.66 mm).

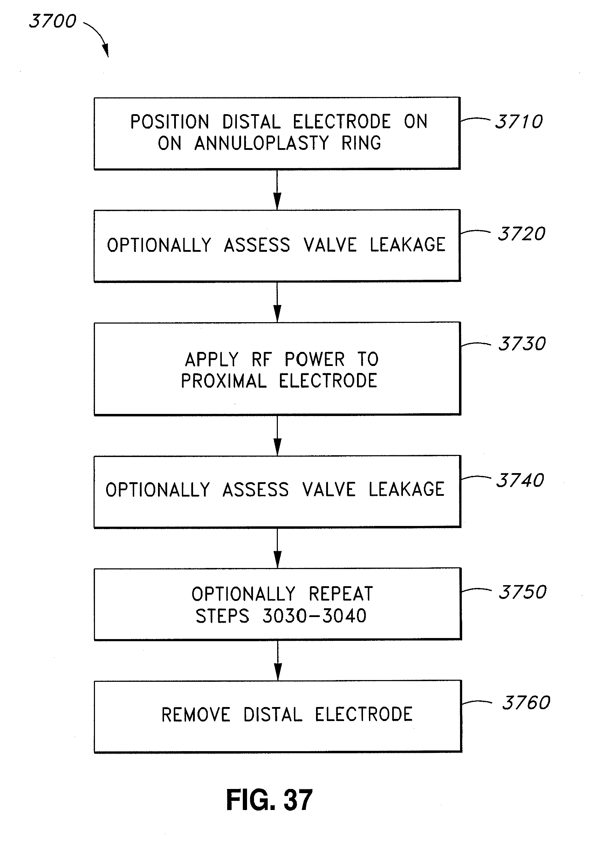

A method 3700 for intraoperatively (on pump) and/or post-operatively (off pump) adjusting an adjustable implant is illustrated as a flowchart in FIG. 37, with reference to FIGS. 36A-36C. The following describes the adjustment of an annuloplasty ring, for example, as illustrated in FIG. 1A; however, those skilled in the art will understand that the method is also applicable to other types of adjustable annuloplasty rings, as well as other types of adjustable devices.

In step 3710, the distal electrode 3630 is positioned between the adjustable body 112 of the annuloplasty ring and the suturable material 128. For example, for the device illustrated in FIGS. 36A-36C, the suturable material 128 is punctured using the suture needle 3622 and the distal electrode 3630 drawn into position using the suture 3620. In certain embodiments, the suture needle 3622 can puncture a layer of silicone in order to be drawn into position. Certain embodiments use a plurality of devices 3600 positioned at different portions of the annuloplasty ring. Certain embodiments of the annuloplasty ring comprise markings or other indicia indicating one or more predetermined locations for positioning the distal electrode(s) 3630. In certain embodiments, the distal electrode 3630 is positioned after the annuloplasty ring is implanted. In certain embodiments, the distal electrode 3630 is positioned before the annuloplasty ring is implanted.

In some post-operative applications, the puncture tip 3614 is used to thread the proximal portion of the device 3600, for example, through a patient's chest wall, such that the proximal electrode 3610 is accessible outside the patient's body. In some of these embodiments, the puncture tip 3614 is then removed, for example, at the groove 3612. In certain embodiments, the incision in the patient's chest is closed around the suture 3620 such that the proximal end 3602 of the device extends out of the closed incision. In some of these embodiments, the proximal electrode 3610 does not comprise a puncture tip.

In optional step 3720, leakage at the repaired valve is assessed. In intraoperative embodiments, a leak test is performed as described above, for example, using ultrasound, such as transesophageal echocardiography (TEE). In post-operative embodiments, leakage is assessed by means known in the art, for example, by echocardiogram, ultrasound, MRI, or the like.

In step 3730, RF power is applied to the proximal electrode 3610 using an RF generator of any suitable type, for example, as illustrated in FIG. 31 and described above. The RF power is transmitted to the distal electrode 3630 through the power wire 3640, causing the temperature of the distal electrode 3630 to increase. In certain embodiments, the temperature is monitored using the thermocouple 3650 and the RF power is modulated accordingly. In certain embodiments using a plurality of adjustment electrodes 3600, RF power is applied to one or more of the adjustment electrodes contemporaneously. In certain embodiments, power is not applied contemporaneously.

In optional step 3740, leakage at the repaired valve is assessed, as described above. In step 3750, steps 3730 and 3740 are optionally repeated until leakage at the valve is reduced to an acceptable level. In step 3760, the distal electrode 3730 is removed, for example, by pulling on the proximal end of the suture.

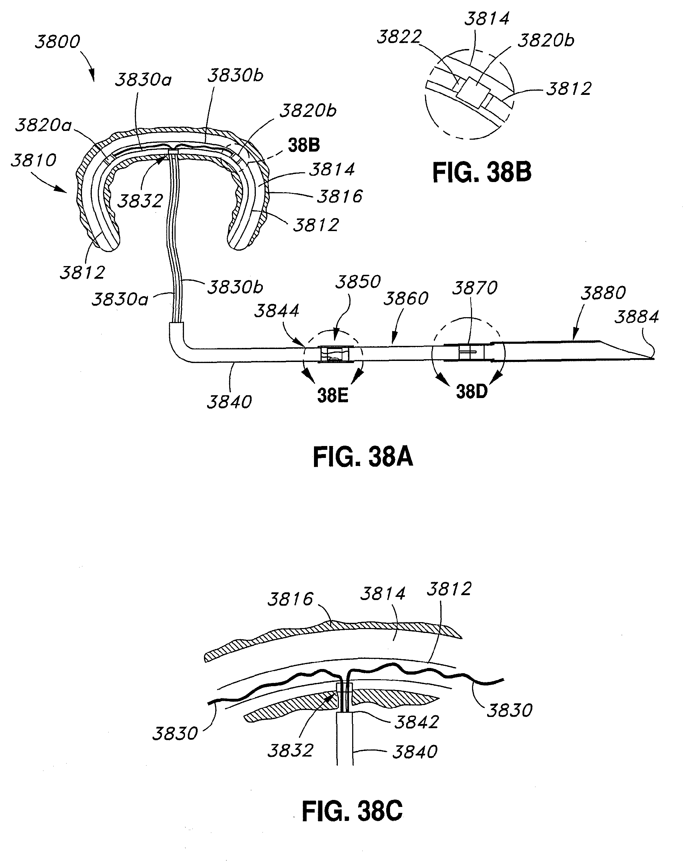

FIG. 38A illustrates an embodiment of an integrated implantable adjustment electrode and adjustable annuloplasty ring system 3800. The system comprises an annuloplasty ring 3810 comprising a shape memory ring 3812, which is discussed above. In certain embodiments, the shape memory ring 3812 comprises nitinol. A flexible material 3814 covers the shape memory ring 3812, and a suturable material 3816 covers the flexible material 3814. Suitable flexible materials and suturable materials are described in greater detail below. In certain embodiments, the flexible material 3814 comprises silicone rubber, and the suturable material comprises polyester (Dacron®).

Disposed between the shape memory ring 3812 and flexible material 3814 are one of more ring electrodes 3820, which are best seen in FIG . 38B. Two ring electrodes 3820*a* and 3820*b* are illustrated; however, those skilled in the art will understand that certain embodiments use a different number of ring electrodes 3820 and/or different locations. In the illustrated embodiment, the ring electrodes 3820 are platinum band electrodes secured to the shape memory ring 3812 using heat shrink tubing 3822. Those skilled in the art will understand that certain embodiments use different materials, configurations, and/or securing means for the ring electrodes 3820.

In electrical connection with each ring electrode 3820 is a power wire 3830. In the illustrated embodiment, power wires 3820*a* and 3830*b* are disposed between the shape memory ring 3812 and flexible material 3814 towards a common exit point 3832, where they enter and are captured by a distal end 3842 of a tubular conduit 3840. In FIG. 38A, the distal end 3842 of the conduit is illustrated pulled away from the from the exit point 3832 for clarity. FIG. 38C illustrates the distal end 3842 of the conduit penetrating the suturable material 3816 and flexible material 3814, and terminating proximal to the common exit point 3832, where the power wires 3820 enter the open distal end 3842. In the illustrated embodiment, the conduit 3840 is about 0.020" × 0.026" (about 0.5 mm × 0.66 mm) PEBAX® tubing that is about 35" (about 89 cm) long. Those skilled in the art will understand that other types of biocompatible tubing in other sizes are also useful in certain embodiments.

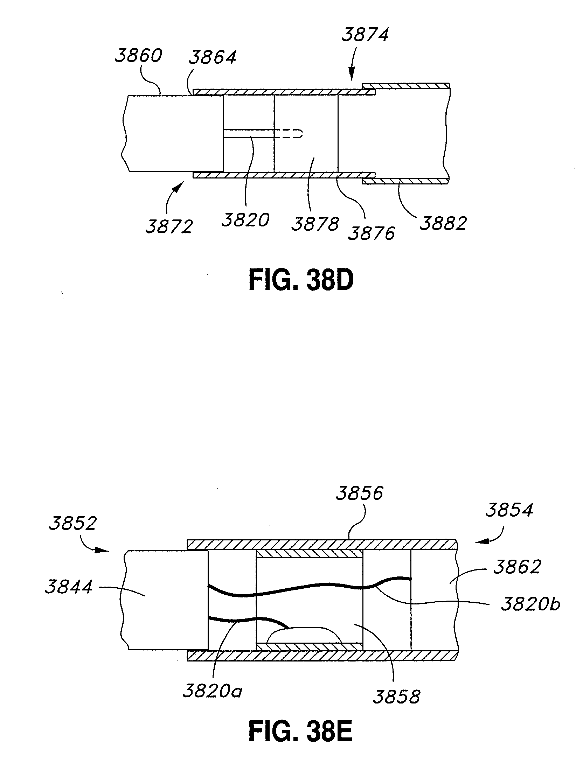

A proximal end 3844 conduit 3840 terminates in a first proximal electrode 3850, best seen in a cross section illustrated in FIG. 38E. The first proximal electrode 3850 comprises a distal end 3852 and a proximal end 3854, an outer tube 3856, and an inner tube 3858. In the illustrated embodiment, the outer tube 3856 and inner tube 3858 comprise electrically conductive materials, for example, stainless steel. In the illustrated embodiment, the outer tube 3856 comprises an about 0.025" (about 0.6 mm) O.D. piece of hypodermic tubing. The inner tube 3858 is disposed within the outer tube 3856, and is secured in the illustrated embodiment by crimping the outer tube 3856. The first power wire 3820*a* is placed in electrical connection with the inner tube 3858 in the illustrated embodiment, for example, by soldering. Because both the inner tube 3858 and outer tube 3856 are electrically conductive, the first power wire 3820*a* is also in electrical connection with the outer tube 3856. Those skilled in the art will understand that the electrical connection between the first power wire 3820*a* and the first proximal electrode 3850 is made in other ways in certain embodiments, for example, by crimping the first power wire 3820*a* between the inner tube 3858 and outer tube 3856. In the illustrated embodiment, the conduit 3840 is secured by crimping to the outer tube 3856.

The second power wire 3820*b* traverses the first proximal electrode 3850 from the distal end 3852 to the proximal end 3854, where it enters a tubular connector 3860 comprising a distal end 3862 and a proximal end 3864. In the illustrated embodiment, the connector comprises a short piece of about 0.020" × 0.026" (about 0.5 mm × 0.66 mm) PEBAX® tubing, the distal end of which is crimped to the proximal end 3854 of the outer tube 3856 of the first proximal electrode 3850.

The second power wire 3820*b* exits proximal end 3864 of the connector into a second proximal electrode 3870, which is illustrated in cross section in FIG. 38D. The illustrated embodiment is substantially similar to the first proximal electrode 3850, comprising a distal end 3872 and a proximal end 3874, an outer tube 3876, and an inner tube 3878. The second power wire 3820*b* is in electrical connection with the second proximal electrode 3870, for example, as described above for the first power wire 3820*a* and the first proximal electrode 3850. In the illustrated embodiment, the proximal end 3864 of the connector is secured to the proximal end 3872 of the second proximal electrode by crimping the outer tube 3876 to proximal end 3864 of the connector.

The illustrated embodiment of the device 3800 also comprises a hollow needle 3880 comprising a distal end 3882 and a proximal puncture tip 3884. The distal end 3883 secured to the proximal end 3874 of the second proximal electrode. In the illustrated embodiment, the proximal end 3874 of the second proximal electrode comprises a break-off section, used for removing the needle 3880, as discussed in greater detail below.

Those skilled in the art will understand that certain embodiments use a different number of ring electrodes and/or proximal electrodes. For example, in certain embodiments, a plurality of ring electrodes is in electrical connection to a single proximal electrode. Furthermore, in certain embodiments, the proximal electrodes have a different configuration, for example, in a plug, socket, or connector of any type known in the art.

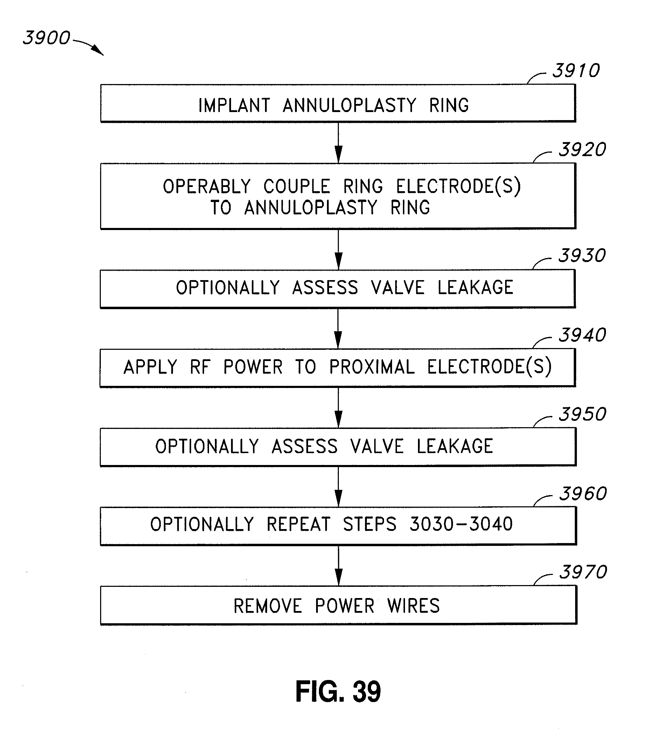

An embodiment of a method 3900 for adjusting an adjustable annuloplasty ring using the integrated implantable adjustment electrode and adjustable annuloplasty ring system 3800 is illustrated as a flowchart in FIG. 39. The following description of the method 3900 references the embodiment of the device 3800 illustrated in FIGS. 38A-38D. The method 3900 is useful for both intraoperative (on pump) and/or post-operative (off pump) adjustment.

In step 3910, the annuloplasty ring 3810 is implanted. In some post-operative applications, the puncture tip 3884 of the needle 3880 is used to thread the conduit 3840, for example, through a patient's chest wall, such that the proximal electrodes 3850 and 3870 are accessible outside the patient's body. In some of these embodiments, the puncture tip 3884 is then removed, broken off. In certain embodiments, the incision in the patient's chest is closed around the conduit 3840 such that the proximal electrodes 3850 and 3870 extend out of the closed incision. In some of these embodiments, the device 3800 does not comprise a puncture tip 3884.

In step 3920, each ring electrode 3820 is operably coupled to the annuloplasty ring such that the ring electrode can conduct activation energy to the annuloplasty ring. In optional step 3930, leakage at the repaired valve is assessed. In intraoperative embodiments, a leak test is performed as described above, for example, using ultrasound, such as transesophageal echocardiography (TEE). In post-operative embodiments, leakage is assessed by means known in the art, for example, by echocardiogram, ultrasound, MRI, or the like.

In step 3940, RF power is applied to at least one of the proximal electrodes 3850 and/or 3870 using an RF generator of any suitable type, for example, as illustrated in FIG. 31 and described above. The RF power is transmitted to the ring electrode(s) 3820*a* and/or 3820*b* through the power wire(s) 3830*a* and/or 3830*b*, causing the temperature of the ring electrode(s) 3820*a* and/or 3820*b* to increase. In certain embodiments, the temperature is monitored using a thermocouple (not illustrated) and the RF power is modulated accordingly. In certain embodiments using a plurality of ring electrodes, RF power is applied to one or more of the ring electrodes contemporaneously. In certain embodiments, power is not applied contemporaneously.

In optional step 3950, leakage at the repaired valve is assessed, as described above. In step 3960, steps 3930 and 3940 are optionally repeated until leakage at the valve is reduced to an acceptable level. In step 3970, the power wires 3830*a* and 3830*b* are removed along with the conduit 3840.

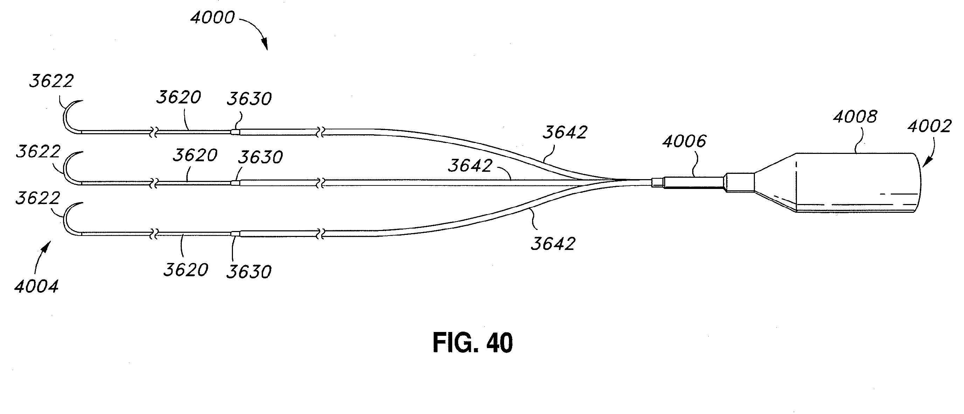

FIG. 40 is a side view illustrating one embodiment of an adjustment device for intraoperative adjustment of an adjustable device. The illustrated device activates an adjustable device using activation energy from an energy source, as discussed above.

The adjustment device 4000 comprises a proximal end 4002 and a distal end 4004. The proximal end 4002 includes a plug 4008 configured to be connected to an energy source. The plug 4008 may be formed using any energy source connector known in the art, for example, such as a plug and socket connector or a crimp-on terminal. For example, in certain embodiments, the plug is a 8-pin connector cable. In certain embodiments, the proximal end 4002 further includes a flexible stress/strained relief member 4006. The flexible stress/strained relief member 4006 provides flexible relief with respect to the activation energy connection maintained by the adjustment device. The flexible stress/strained relief member 4006 may be formed using any energy source connector known in the art.

In certain embodiments, the proximal end 4002 of the adjustment device 4000 includes a plurality of power wires 3640 (see FIGS. 36B-36C) as described above, each power wire 3640 configured to operatively couple its associated distal electrode 3630 to an energy source via the plug 4008. In certain embodiments, the proximal end 4002 of the adjustment device 4000 includes one power wire 3640. Disposed over the power wire 3640 and sutures 3620 is a flexible sheath 3642, as discussed above.

In certain embodiments, each power wire 3640 comprises a distal electrode 3630. For example, in the illustrated embodiment, the distal end 4004 comprises three distal electrodes 3620. The electrodes may emit any combination of positive charges or negative charges. For example, in the illustrated embodiment, there can be two positive electrodes 3620 and one negative electrode. In certain embodiments, the three electrodes can be a positive electrode, negative electrode, and ground electrode. In certain embodiments, the adjustment device 4000 may comprise up to six power wires 3640, each comprising an electrode 3620.

For each distal electrode 3620, a suture 3620 extends from the distal end 3604 to the proximal electrode 3610, and is secured thereto, as described above with reference to FIGS. 36A-36E. In certain embodiments, the features, lengths, distances, dimensions, and materials of the adjustment device 4000 are the same as or similar to the features, lengths, distances, dimensions, and materials of the adjustment device 3600 discussed above.

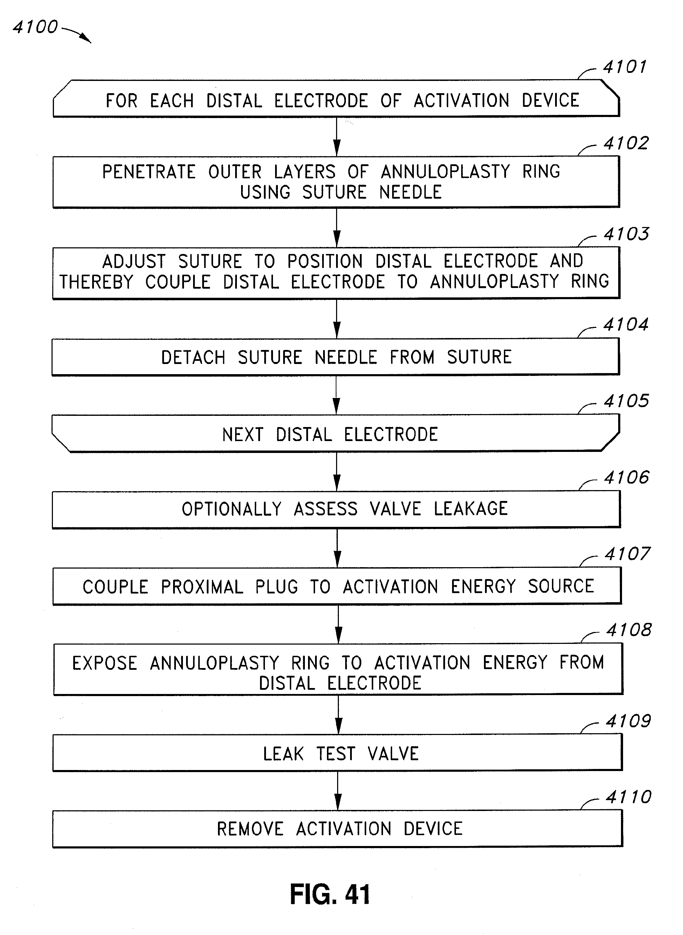

FIG. 41 is a flowchart illustrating an embodiment of a method for adjusting an adjustable device using the device illustrated in FIG. 40. In certain embodiments, the method may be practiced intraoperatively. The following describes the adjustment of an annuloplasty ring, for example, as illustrated in FIG. 1A; however, those skilled in the art will understand that the method is also applicable to other types of adjustable annuloplasty rings, as well as other types of adjustable devices.

According to step 4101, each distal electrode 3630 is to be positioned in order to be operatively coupled with a corresponding portion of an adjustable device. Thus, for each distal electrode 3630, its corresponding suture needle 3622 is used to penetrate outer layers of the adjustable device. For example, using the device illustrated in FIG. 40, a suturable material 128 (as illustrated above) is punctured using the suture needle 3622. In certain embodiments, the suture needle 3622 can puncture other outer layers, such as a layer of silicone. Then, in step 4103, each distal electrode 3630 is adjustably positioned between the adjustable body 112 of the adjustable device and the suturable material 128. For example, the distal electrode 3630 may be adjusted by pulling on either end of the suture 3620. Certain embodiments of the annuloplasty ring comprise markings or other indicia indicating one or more predetermined locations for positioning each of the distal electrode(s) 3630. In certain embodiments, each distal electrode 3630 is positioned after the annuloplasty ring is implanted, such as through open heart surgery. In certain embodiments, each distal electrode 3630 is positioned before the annuloplasty ring is implanted. After the position of each distal electrode 3630 has been appropriately adjusted, the corresponding suture needle 3622 may be detached from the suture wire 3620 and removed. For example, the suture needle 3622 may be detached by using scissors to cut the suture needle 3622 from the suture 3620 at a location on the suture 3620 proximal to the suture needle 3622. In step 3605, the process of steps 4102 through 4104 is completed for each distal electrode 3630. If the adjustment device 4000 and the ring have not been implanted, then they may then be implanted into a heart valve annulus by open heart surgery.

In optional step 4106, leakage at the repaired valve is assessed, as described above. In certain embodiments, at this point the heart may be restarted and the heart of the patient may then be closed. In certain embodiments, a proximal end of each suture 3620 remains protruding from the heart of the patient. Then, in step 4107, the plug 4008 may be connected to an activation energy source. In certain embodiments, step 4107 may occur before the heart of the patient is restarted and the heart is closed.

In step 4108, the activation energy source is activated, thereby providing activation energy to each distal electrode 3630. The activation energy is transmitted to each distal electrode 3630 through the power wire 3640, in certain embodiments causing the temperature of the distal electrode 3630 to increase. In certain embodiments, the temperature of the distal electrode 3630 can be monitored using a thermocouple 3650, as described above, and the activation energy is modulated accordingly. In certain embodiments, activation energy is applied to one or more of the adjustment electrodes 3630 contemporaneously. In certain embodiments, activation energy is not applied contemporaneously.

In optional step 4109, leakage at the repaired valve is assessed, as described above. The adjustment device 4000 can then be removed in step 4010. For example, the adjustment device 4000 can be removed by pulling on the proximal end of the suture until the remaining portion of the adjustment device 4000 is removed. The exit point from the heart may then be closed, such as by suturing. The sternum of the patient can then be closed.

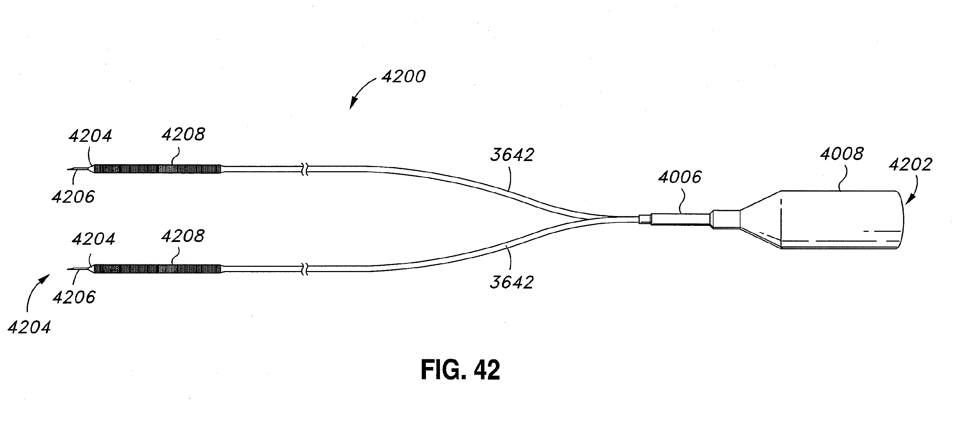

FIG. 42 is a side view illustrating one embodiment of an adjustment device for intraoperative adjustment of an adjustable device. The illustrated device activates an adjustable device using activation energy from an energy source, as discussed above.

The adjustment device 4200 comprises a proximal end 4202 and a distal end 4204. The proximal end 4202 includes a plug 4008, and optionally a flexible stress/strained relief member 4006, as described above with reference to FIG. 40.

In certain embodiments, the proximal end 4202 of the adjustment device 4200 includes a plurality of power wires 3640 as described above with reference to FIG. 40. In certain embodiments, the proximal end 4202 of the adjustment device 4200 includes one power wire 3640. Disposed over the power wire 3640 is a flexible sheath 3642, as discussed above. In certain embodiments, each power wire 3640 can connect to the proximal end of each distal electrode by soldering. In certain embodiments, the power wire 3640 is covered with an insulating layer, such as a polyimide layer, as discussed above.

In certain embodiments, each power wire 3640 comprises a distal electrode 4206 configured to pierce an outer layer of an adjustable device. For example, in certain embodiments, the distal electrode 4206 can take the shape of a needle, as illustrated. In other embodiments, the distal electrode 4206 can be configured to pierce an outer layer of an adjustable device using other shapes. The electrode 4206 can be manufactured using at least any of the materials described above with reference to FIG. 29. In the illustrated embodiment, the distal end 4204 of the adjustment device 4200 comprises two distal electrodes 4206. The electrodes may emit any combination of positive charges or negative charges. For example, in the illustrated embodiment, there can be one positive electrodes 4206 and one negative electrode 4206. In certain embodiments, one electrode 4206 can be a positive electrode and one electrode 4206 can be a ground electrode. In certain embodiments, the adjustment device 4200 may comprise up to six power wires 3640, each comprising an electrode 4206. In certain embodiments, the length of the distal electrode 4206 is sized to be substantially equal to the depth of the outer layers of an adjustable device. Consequently, after the distal electrode 4206 penetrates the outer layers and is operably coupled to an activatable portion of the adjustable device, substantially all of the distal electrode 4206 is encompassed within the outer layers of the adjustable device. In certain embodiments, the distal electrode 4206 is two millimeters in length. In certain embodiments, the distal electrode 4206 is four millimeters in length. In certain embodiments, the distal electrode 4206 is between two millimeters and four millimeters in length. In certain embodiments, the distal electrode 4206 is less than two millimeters in length. In certain embodiments, the distal electrode 4206 is greater than four millimeters in length.

In certain embodiments, the distal end 4000 of the adjustment device further comprises a thermocouple 4204, proximal to the distal electrode 4206. The type, mounting, connection, and reading of the thermocouple is discussed above, with reference to FIG. 29.

In certain embodiments, a portion of each power wire 3640 is housed by a coiled housing 4208 which houses the power wire 3640 and thermocouple lead wires 2922. In certain embodiments, the coiled housing 4208 houses the flexible sheath 3642. In certain embodiments, the coiled housing 4208 is not thermally or electrically conductive. In certain embodiments, the housing 4208 can take shapes other than a coil. The coil shape of the housing 4208 facilitates penetration by the distal electrode 4206 because when force is applied from the proximal end toward the distal end 4204 in a direction substantially parallel to the coiled housing 4208 and distal electrode 4206, the coiled housing 4208 remains substantially firm. For example, the coiled housing 4208 facilitates the ability of the distal electrode 4206 to penetrate an outer surface of an annuloplasty ring or other adjustable device. On the other hand, the coil shape facilitates maneuverability of the distal end 4204 of the adjustment device 4200 because the coiled housing 4208 provides flexibility without creating a kink when force is applied in a direction substantially perpendicular to the coiled housing 4208 and distal electrode 4206. Thus, the flexibility of the coiled housing 4208 assists in keeping the distal end 4204 of the adjustment device 4200 coupled to an adjustable device if, for example, such perpendicular force is applied. In certain embodiments, the coiled housing 4208 is six inches in length. In certain embodiments, the coiled housing 4208 is between two inches and six inches in length. In certain embodiments, the coiled housing 4208 is less than two inches in length. In certain embodiments, the coiled housing 4208 is greater than six inches in length. In certain embodiments, each power wire 3640 does not include a coiled housing, such that the flexible sheath 3642 extends to the thermocouple 4204.

For each distal electrode 3620, a suture 3620 extends from the distal end 3604 to the proximal electrode 3610, and is secured thereto, as described above with reference to FIGS. 36A-36E. In certain embodiments, certain remaining features, lengths, distances, dimensions, and materials of the adjustment device 4000 are the same as or similar to the features, lengths, distances, dimensions, and materials of the adjustment device 4000 discussed above.

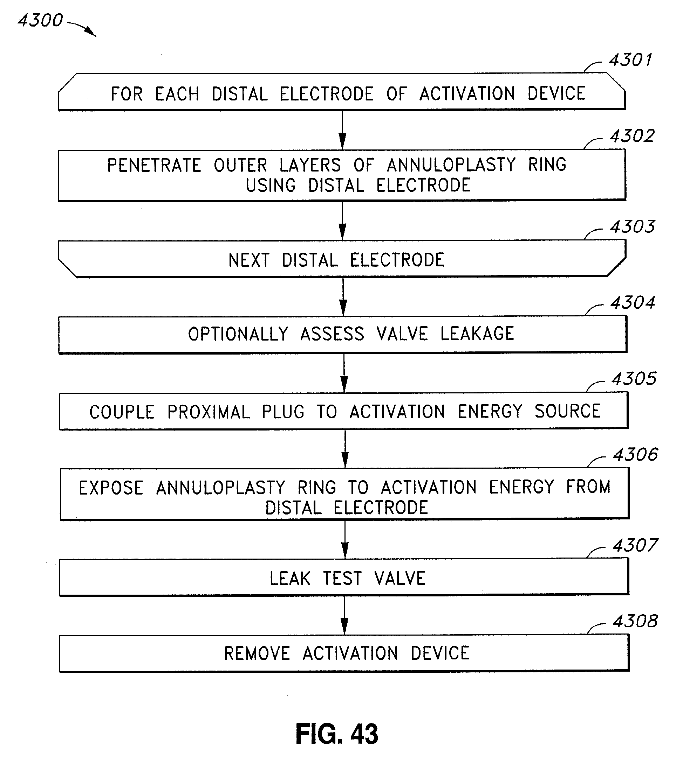

FIG. 43 is a flowchart illustrating an embodiment of a method for adjusting an adjustable device using the device illustrated in FIG. 42. In certain embodiments, the method may be practiced intraoperatively, such as during an open heart surgery. The following describes the adjustment of an annuloplasty ring, for example, as illustrated in FIG. 1A; however, those skilled in the art will understand that the method is also applicable to other types of adjustable annuloplasty rings, as well as other types of adjustable devices.

According to step 4301, each distal electrode 4206 is to be appropriately positioned in order to be operatively coupled with a corresponding portion of an adjustable device. Thus, each distal electrode 4206 penetrates the outer layers of the adjustable device. For example, using the device illustrated in FIG. 40, a suturable material 128 (as illustrated above) is punctured using the needle-shaped distal electrode 4206. The distal electrode 4206 is then positioned to deliver an activation energy to the adjustable device. Certain embodiments of the adjustable device comprise markings or other indicia indicating one or more predetermined locations for positioning each of the distal electrode(s) 4206. In certain embodiments, each distal electrode 4206 is positioned after the annuloplasty ring is implanted. In certain embodiments, each distal electrode 4206 is positioned before the annuloplasty ring is implanted. In step 4303, the process of step 4302 is completed for each distal electrode 4206.

In optional step 4304, leakage at the repaired valve is assessed, as described above. Then, in step 4305, the plug 4008 may be connected to an activation energy source.

In step 4306, the activation energy source is activated, thereby providing activation energy to each distal electrode 4206. The activation energy is transmitted to each distal electrode 4206 through the power wire 3640, in certain embodiments causing the temperature of the distal electrode 4206 to increase. The temperature of the distal electrode 4206 can be monitored using thermocouple 4204, as described above, and the activation energy modulated accordingly. In certain embodiments, activation energy is applied to one or more of the adjustment electrodes 4206 contemporaneously. In certain embodiments, activation energy is not applied contemporaneously.

In optional step 4307, leakage at the repaired valve is again assessed, as described above. The adjustment device 4200 can then be removed in step 4308. For example, the adjustment device 4200 can be removed by pulling on the proximal end of the suture until the remaining portion of the adjustment device 4200 is removed. The exit point in the heart can then be closed, such as by suturing.

Those skilled in the art will understand that various features illustrated and described for the above embodiments are independently combinable in certain embodiments.

While certain embodiments of the inventions have been described, these embodiments have been presented by way of example only, and are not intended to limit the scope of the inventions. Indeed, the novel methods and systems described herein may be embodied in a variety of other forms; furthermore, various omissions, substitutions and changes in the form of the methods and systems described herein may be made without departing from the spirit of the inventions. The accompanying claims and their equivalents are intended to cover such forms or modifications as would fall within the scope and spirit of the inventions.

## Claims

1. An intraoperative adjustment device comprising:
an elongate body comprising a proximal end and a distal end, the distal end configured to penetrate an outer surface of an adjustable cardiac implant implanted in a patient's heart, and the proximal end and the distal end connected by at least one energy-transfer member;
wherein the distal end comprises at least one electrode coupled to the energy-transfer member and configured to deliver an activation energy to the adjustable cardiac implant; and
wherein the proximal end is configured to attach to an external energy source that provides the activation energy, and the proximal end is configured to be located outside the patient's body while the distal end is coupled to the adjustable cardiac implant that is implanted in the patient's heart.

2. The intraoperative adjustment device of Claim 1, wherein the distal end further comprises at least one thermocouple.

3. The intraoperative adjustment device of Claim 2, wherein the at least one thermocouple is proximal to the at least one electrode.

4. The intraoperative adjustment device of Claim 1, the distal end further comprising:
a flexible member extending distally from the at least one electrode; and
a needle coupled to a distal end of the flexible member.

5. The intraoperative adjustment device of Claim 4, wherein the needle is curved.

6. The intraoperative adjustment device of Claim 4, wherein the flexible member comprises a suture.

7. The intraoperative adjustment device of Claim 1, wherein the at least one electrode is configured to penetrate the outer surface of the adjustable cardiac implant.

8. The intraoperative adjustment device of Claim 1, wherein a portion of the distal end that penetrates the adjustable cardiac implant is sharp.

9. The intraoperative adjustment device of Claim 7, wherein a length of the electrode is substantially equal to or less than a cross-sectional thickness of the adjustable cardiac implant.

10. The intraoperative adjustment device of Claim 7, wherein the distal end further comprises a coiled housing proximal to the at least one electrode.

11. The intraoperative adjustment device of Claim 1, wherein the distal end further comprises a plurality of electrodes.

12. The intraoperative adjustment device of Claim 10, wherein the at least one energy-transfer member comprises a plurality of energy-transfer members, each of which is coupled to at least one of the plurality of electrodes.

13. The intraoperative adjustment device of Claim 1, wherein the electrode comprises a biocompatible, thermally conductive material.

14. The intraoperative adjustment device of Claim 1, wherein the electrode comprises a biocompatible, electrically conductive material.

15. The intraoperative adjustment device of Claim 1, wherein the external energy source comprises a radio frequency generator; and
further comprising the radio frequency generator.

16. The intraoperative adjustment device of Claim 1, further comprising a malleable element that permits the elongate body to flex from a first shape to a second shape and substantially retain the second shape.

17. An intraoperative adjustment device comprising:
an elongate body comprising a proximal end and a distal end, the distal end configured to enter a chamber of a heart;
an extendable probe comprising a biocompatible, thermally and/or electrically conductive material disposed on the first end of the body;
a handle coupled to the proximal end of the body;
wherein a distal end of the extendable probe is configured to move from a retracted position that is substantially within the distal end, to an extended position that is substantially protruding distally from the distal end;
wherein the extendable probe, when in the extended position, is configured to transfer energy from the proximal end to an adjustable cardiac implant that is implanted in the heart; and
a stop mechanism configured to prevent a distal end of the extendable probe from proximally retracting out of the elongate body.

18. The intraoperative adjustment device of Claim 17, wherein the extendable probe is configured to penetrate an outer surface of an adjustable device.

19. The intraoperative adjustment device of Claim 17, wherein the distal end of the elongate body further comprises a thermocouple.

20. The intraoperative adjustment device of Claim 19, wherein the extendable probe comprises a sharp end.

21. The intraoperative adjustment device of Claim 19, wherein the extendable probe comprises a substantially helical shape.

22. The intraoperative adjustment device of Claim 21, wherein the extendable probe is configured to screw into the adjustable cardiac implant.

23. The intraoperative adjustment device of Claim 19, wherein the extendable probe comprises a substantially hook shape.

24. An intraoperative adjustment device, comprising:
an elongate body having a distal portion, a proximal portion, and an electrode assembly disposed between the proximal portion and the distal portion;
wherein the electrode assembly comprises an electrode configured to deliver energy to an adjustable cardiac implant;
wherein the distal portion of the elongate body comprises a suture coupled to a distal end of the electrode assembly;
wherein the proximal portion of the elongate body comprises a conducting element that transfers energy from an energy source to the electrode; and
a needle coupled to a distal end of the suture, the needle configured to penetrate a surface of the adjustable cardiac implant.

25. The intraoperative adjustment device of Claim 24, further comprising a sheath disposed over at least a portion of the conducting element.

26. The intraoperative adjustment device of Claim 24, further comprising a thermocouple positioned to detect a temperature at the distal electrode.

27. An adjustable cardiac implant system, comprising:
an adjustable cardiac implant configured to be implanted at or near a base of a patient's heart valve, the adjustable cardiac implant comprising:
a shape-memory element configured to undergo a transformation in shape and/or size in response to an application of energy, thereby resulting in a change in a dimension of the annulus of the patient's heart valve; and
a first conducting member coupled to the adjustable cardiac implant, the first conducting member configured to transfer energy from an energy source to the adjustable cardiac implant resulting in the transformation in shape and/or size of the shape-memory element, wherein the first conducting member extends through a point located at least one centimeter away from an outer surface of the adjustable cardiac implant.

28. The system of Claim 27, further comprising a second conducting member coupled to the adjustable cardiac implant, the second conducting member configured to transfer energy from the energy source to the adjustable cardiac implant.

29. The system of Claim 27, wherein the first conducting member comprises a wire.

30. The system of Claim 27, wherein the first conducting member is reversibly coupled to the adjustable cardiac implant.

31. The system of Claim 27, wherein the first conducting member extends outside the patient's heart when the adjustable cardiac implant is implanted at or near the base of the patient's heart valve.

32. The system of Claim 27, wherein the first conducting member conducts at least one of heat and electromagnetic energy to the adjustable cardiac implant.

33. The system of Claim 27, wherein the adjustable cardiac implant further comprises a flexible material disposed over the shape-memory element and the first conducting member.
